# EUROPEAN PATENT SPECIFICATION

(11) **EP 0 369 733 B1**
(45) Date of publication and mention of the grant of the patent: **21.02.1996**
(21) Application number: 89311760.6
(22) Date of filing: 14.11.1989
(51) Int. Cl.: C07D 273/00, C07D 413/04, C07D 413/14, C07D 491/00, C07D 519/00, G01N 33/84

(54) **Fluorescent indicator dyes for alkali metal cations, their preparation and use**
Fluoreszierende Indikatorfarbstoffe für Alkalimetall-Kationen, ihre Herstellung und Verwendung
Indicateurs colorants fluorescents pour cations de métaux alcalins, leur préparation et leur utilisation

(30) Priority: 14.11.1988 US 271502; 09.08.1989 US 391879
(43) Date of publication of application: 23.05.1990
(73) Proprietor: THE REGENTS OF THE UNIVERSITY OF CALIFORNIA, Oakland, California 94612-3550 (US)
(72) Inventor: Tsien, Roger Yonchien, La Jolla California 92037 (US); Minta, Akwasi, (Nmn), Eugene Oregon 97405 (US)
(74) Representative: Bizley, Richard Edward

(56) References cited:
- EP-A- 0 010 615
- US-A- 4 659 815
- JOURNAL OF THE CHEMICAL SOCIETY, CHEMICAL COMMUNICATIONS, 1988, 1st March 1988, pages 384-385; B. VALEUR et al.: "First crown ether derivative of benzoxazinone; a new fluoroionophore for alkaline earth metals recognition"
- CHEMISCHE BERICHTE, vol. 113, 1st February 1980, pages 457-470; F. VÖGTLE et al.: "Ionenselektive Farbstoffkronenether"
- JOURNAL OF BIOLOGICAL CHEMISTRY, vol. 264, 15th November 1989, pages 19449- 19456; A. MINTA et al.: "Fluorescent indicators for cytosolic sodium"

## Description

The present invention relates generally to new macrocyclic fluorescent chelating compounds for alkali metal cations. More specifically, the present invention relates to novel chemical compositions comprised of: the combination of (1) an aza-crown ether and (2) ligand(s), at least one of which will be a heteroaromatic fluorophore that bears additional cation chelating centers, wherein the ligand(s) is attached to the aza-crown ether via the sp³-hybridized core nitrogen(s). The new fluorescent indicator compounds of the present invention are used to nondestructively observe concentrations of free alkali metal cations such as Na⁺, K⁺, and Li⁺, particularly inside living cells and tissues.

Nearly all animal cells maintain a large difference in sodium concentrations between their interiors (typically 10-40 mM) and the extracellular milieu (120-450 mM). This gradient is used to power nutrient uptake, epithelial transport, regulation of other intracellular ions, and transmission of electrical impulses. These functions are so important that organisms devote a major part of their metabolic energy to maintaining the sodium gradient (1,2).

Measurements of intracellular Na⁺ are essential to understanding the many biological roles of this ion. Current techniques fall into three categories: (1) Assays that measure total cell Na⁺ but destroy the tissue; (2) Non-destructive assays that rely on nuclear magnetic resonance; and (3) Non-destructive assays that rely on well-defined physiochemical equilibria to measure free [Na⁺] or Na⁺ activity.

Examples of assays that measure total cell Na⁺ but destroy the tissue include flame photometry, atomic absorption, neutron activation, counting of Na at isotopic equilibrium, and electron microprobe analysis (30). The destructive nature of these techniques is obviously a drawback when time courses are desired. Except for the electron-microscopic methods, these techniques lack spatial resolution and demand careful removal of extracellular fluid, which usually has a much higher concentration of Na⁺ than the cells. The most general problem (18,31,32) is that the total intracellular [Na⁺] usually considerably exceeds free [Na⁺]ᵢ, and it is the latter that affects binding equilibria, transmembrane electrochemical gradients, and cell function. Free and total [Na⁺] are known to be able to vary independently (32).

It is well known that NMR techniques using dysprosium shift reagents can quantify the amount of intracellular Na⁺ that is readily exchangeable on the NMR time scale (33,34). This probably includes weakly bound Na⁺ as well as free. Though non-destructive, this technique requires relatively large amounts of tissue packed at high density in a magnet cavity, an environment awkward for other manipulations.

Techniques that rely on well-defined physiochemical equilibria to nondestructively measure free [Na⁺] (or Na⁺ activity) include ¹⁹F NMR of Na⁺-sensitive chelators (6), Na⁺-selective microelectrodes (32), and the new fluorescent indicators of the present invention. Advantages of fluorescent indicators include excellent spatial and unsurpassed temporal resolution, compatibility with cell types too small or fragile to impale with ion-selective and voltage reference barrels, and applicability to single cells as well as to populations (35). Perhaps the chief disadvantage is the demand for optical clarity of the tissue.

Prior art techniques for measuring and manipulating intracellular free sodium concentrations ([Na⁺]ᵢ) have been severely limited by the lack of synthetic ligands that can bind sodium with the requisite affinity and specificity in aqueous solution at pH 7. A particularly desirable ligand would have the following properties:
(1) Na⁺ should bind with a dissociation constant (K_{d}) of 5-50 mM at pH 7, in aqueous solution with no organic co-solvents permitted. Such a K_{d} would approximately match the expected range for [Na⁺]ᵢ and maximize sensitivity to small changes in [Na⁺]ᵢ. Excessive Na⁺ affinity would be undesirable, since the indicator would then either be Na⁺-saturated and unresponsive, or if applied in excess would depress [Na⁺]ᵢ.
(2) The indicator should have enough discrimination against K⁺ (at least twenty-fold, or a K_{d} > 150 mM), H⁺ (highest pKₐ <6.5), Mg⁺ (K_{d} > 10 mM), and Ca⁺ (K_{d} > 10 µM) so that physiological variations in those ions have little effect.
(3) It would show reasonably strong fluorescence, characterizable by a product of extinction coefficient and fluorescence quantum yield exceeding 10³ M₋₁ cm₋₁.
(4) Its excitation wavelengths should exceed 340 nm, because shorter wavelengths demand expensive quartz rather than glass microscope optics and are strongly absorbed by nucleic acids and aromatic amino acids.
(5) Emission wavelengths should exceed 500 nm to reduce overlap with tissue autofluorescence from reduced pyridine nucleotides peaking near 460 nm.
(6) Either the excitation or emission spectrum or both should undergo a large wavelength shift upon binding Na⁺, so that ratioing of signals at two excitation or two emission wavelengths can cancel out the local pathlength, dye concentration, and wavelength-independent variations in illumination intensity and detection efficiency.
(7) The indicator should have enough polar groups such as carboxylates to render it highly water-soluble and impermeant through membranes, so that it does not rapidly leak out of cells.
(8) The polar groups just mentioned should be maskable by nonpolar protecting groups hydrolyzable by cytoplasm, so that large populations of cells can be loaded with the indicator by incubating them with the membrane-permeant nonpolar derivative rather than requiring microinjection or other techniques of membrane disruption. The most obvious protecting groups are acetoxymethyl esters, which have proven to be useful with a wide variety of cation indicators (51,52).

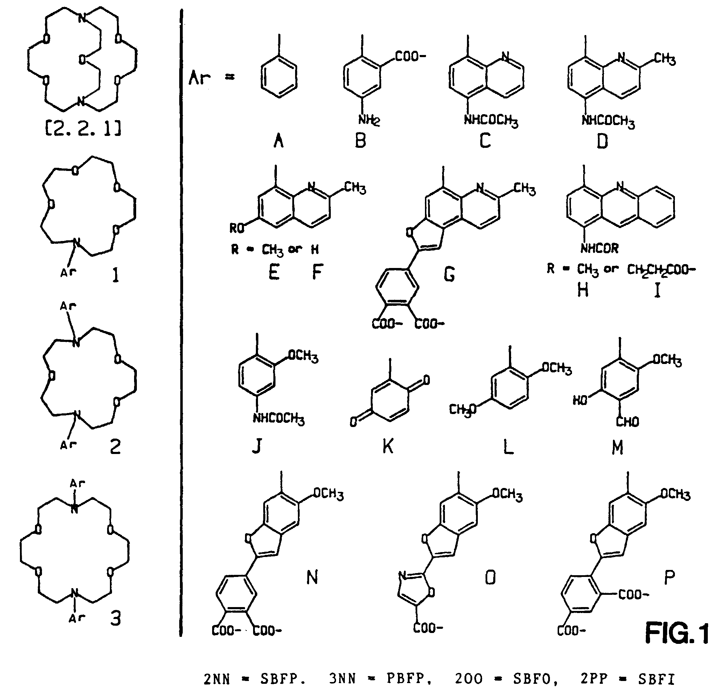

No such compound has yet been demonstrated to work in living cells despite nearly two decades worth of research on crown ethers and related ligands. Indicator dyes with visible absorbance and moderate preference for Na⁺ over K⁺ have been reported (3,4) but their operation is limited to nonaqueous solvents like acetonitrile, and no quantitative data is available on their cation binding constants. Higher affinity and selectivity for Na⁺ over K⁺ in water can be obtained with macrobicyclic chelators, for example the cryptand "[2.2.1]" (*see* ref. 5 and Fig. 1). Recently, fluorine-substituted cryptands have been introduced for measurement of [Na⁺]ᵢ by ¹⁹F-NMR (6). A promising fluorescent version was also described by Smith, *et al.,* (1988) (*see* ref. 7), but its excitation and emission spectra peaked at rather short wavelengths, 320 and 395 nm respectively, and no demonstration of intracellular use was given. The highest selectivities for Na⁺ over K⁺ are obtained in very large rigid chelators called "spherands"; so far these require organic solvents for solubility and are so rigid that hours to days are required for equilibration with Na⁺ (8,9). The main mechanism by which they give optical shifts upon metal binding has been the displacement of a proton from the binding cavity, but this equilibrium must inherently be pH-sensitive, which is an unwanted feature. We chose to explore crown ethers rather than the more elaborate cryptands and spherands both for ease of synthesis and because of a concern that the conformational rigidity and preorganization of cryptands and spherands would tend to reduce the spectroscopic shift upon metal binding.

The present invention discloses a new series of macrocyclic compounds that can chelate alkali metal cations and that attain the basic goals described above for a desirable fluorescent sodium indicator. Tests in lymphocytes, hepatocytes, fibroblasts (10), smooth muscle cells (11), and gastric glands (12) demonstrate the biological utility of the macrocyclic compounds of the present invention for nondestructive observation of [Na⁺]ᵢ in individual cells viewed by fluorescence microscopy.

In the accompanying drawings:-

The drawings comprise 6 Figures, of which:

FIGURE 1 is a drawing that shows the structures of some of compounds of the present invention. For comparison, Lehn's cryptand [2.2.1] (*see* ref. 5) is shown in the upper left hand corner. Below cryptand [2.2.1] are three generic types of crown ethers that have been prepared for use in the making some of the compounds of the present invention. To the right of the vertical dividing line are examples of some of the aromatic and heterocyclic substituents that can be attached to the nitrogens of the crown ethers. Compound "**SBFI**" is **2PP**; "**SBFO**" is **2OO**; "**PBFP**" is **3NN**.

Dissociation constants, cation affinities, plus the absorbance and emission maxima and quantum efficiency for sodium are given in TABLE 1.

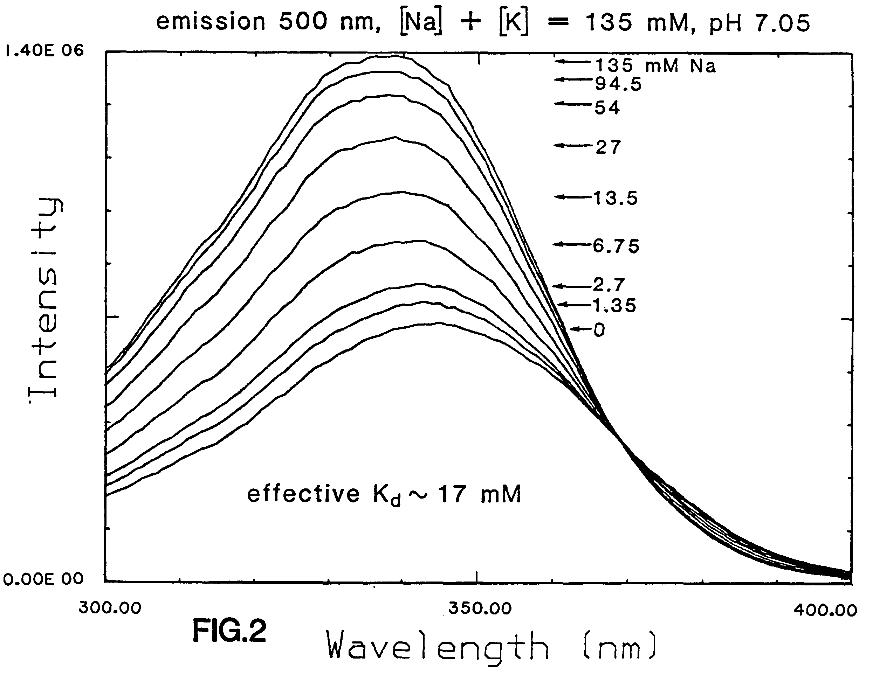

FIGURE 2 is a graph that shows the fluorescence excitation spectra of **SBFI** as a function of increasing [Na⁺] and decreasing [K⁺]. The lowest curve ([Na⁺] = 0) was obtained with 5 µM **SBFI** in 130 mM KCl, 10 mM MOPS, KOH to pH 7.05, approximately 135 mM total K⁺. The highest curve ([Na⁺] = 135 mM) was analogously recorded from 5 µM **SBFI** in 130 mM NaCl, 10 mM MOPS, NaOH to pH 7.05. The intermediate curves from 1.35 to 94.5 mM Na⁺ were obtained by successively 1/100, 1/99, 3/98, 1/19, 1/9, 1/4, and 1/2 of the K⁺-rich SBFI solution by the **SBFI** in Na medium. The excitation bandwidth was 1.85 nm; emission was collected at 500 nm with 9.3 nm bandwidth. The temperature was 22°, ±2°.

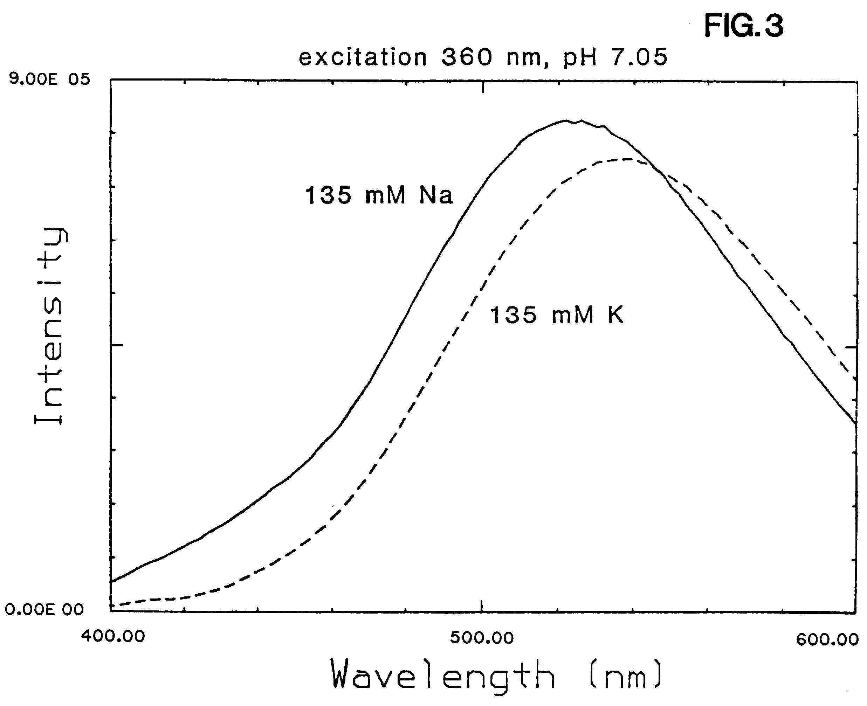

FIGURE 3 is a graph that shows the fluorescence emission spectra of **SBFI** at 135 mM K⁺ and 135 mM Na⁺, recorded from the same solutions as used in Fig. 2 for 0 and 135 mM Na⁺. Excitation was at 360 nm; bandwidths were the same as in Fig. 2.

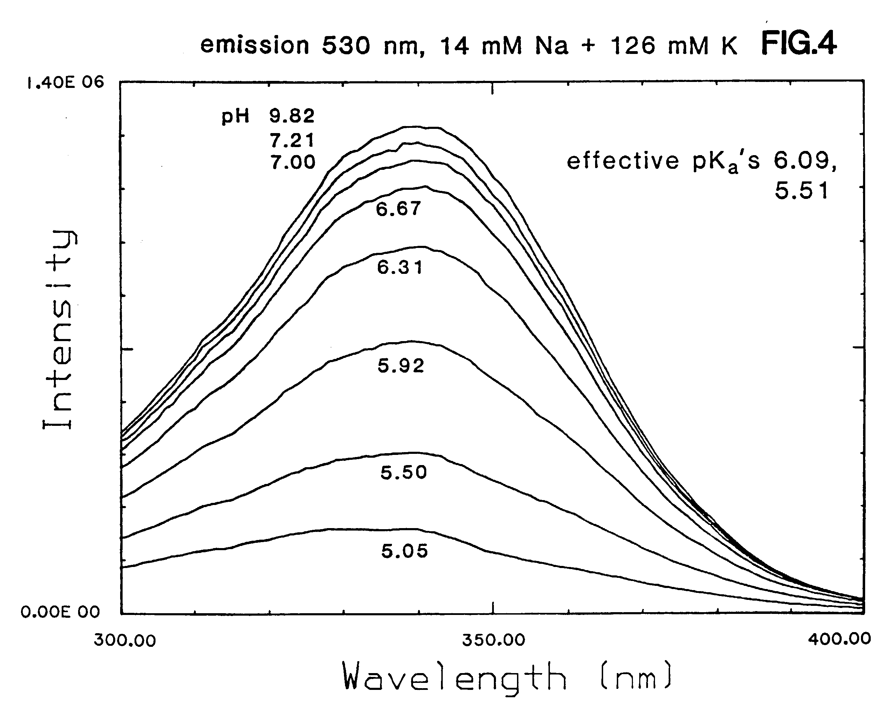

FIGURE 4 is a graph that shows the fluorescence emission spectra of 5 µM **SBFI** in 14 mM NaCl, 126 mM KCl, 1 mM MgCl₂, 4 mM Tris, titrated to the indicated pH values by small additions of 5M H₃PO₄. Emission was collected at 530 nm. Bandwidths and temperature were as in Fig. 2.

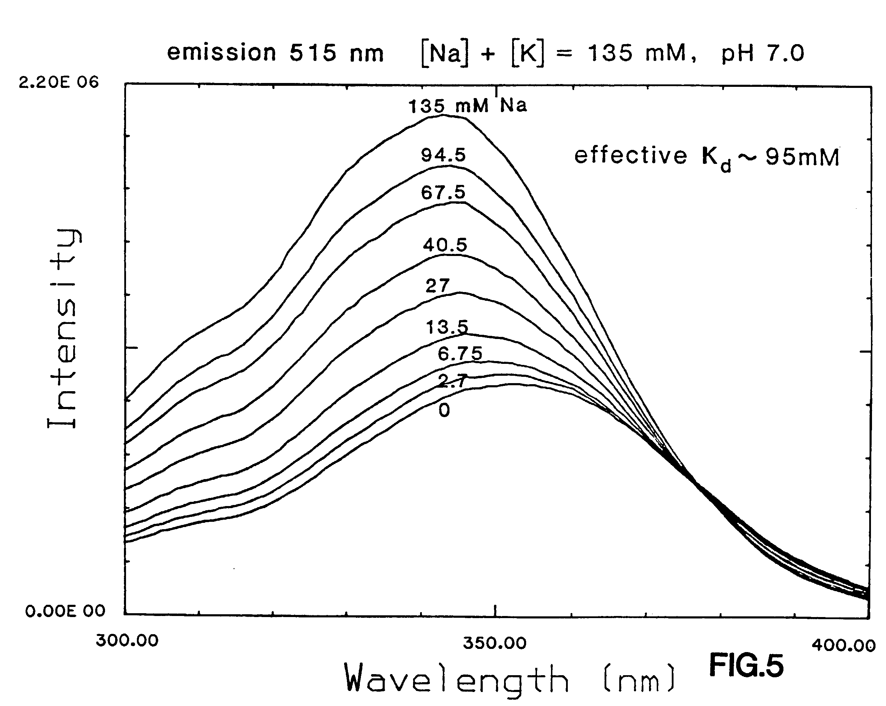

FIGURE 5 is a graph that shows the fluorescence excitation spectra of 6 µM **SBFO** as a function of increasing [Na⁺] and decreasing [K⁺] in solutions similar to those in Fig. 2. The intermediate Na⁺ concentrations from 2.7 to 94.5 mM were obtained by the iterative replacement of 1/50, 3/98, 1/19, 1/9, 1/8, 2/7, and 2/5 of the high K⁺ medium by the 135 mM Na⁺ mixture. Excitation bandwidth was 1.85 nm; emission was collected at 515 nm and 4.7 nm bandwidth.

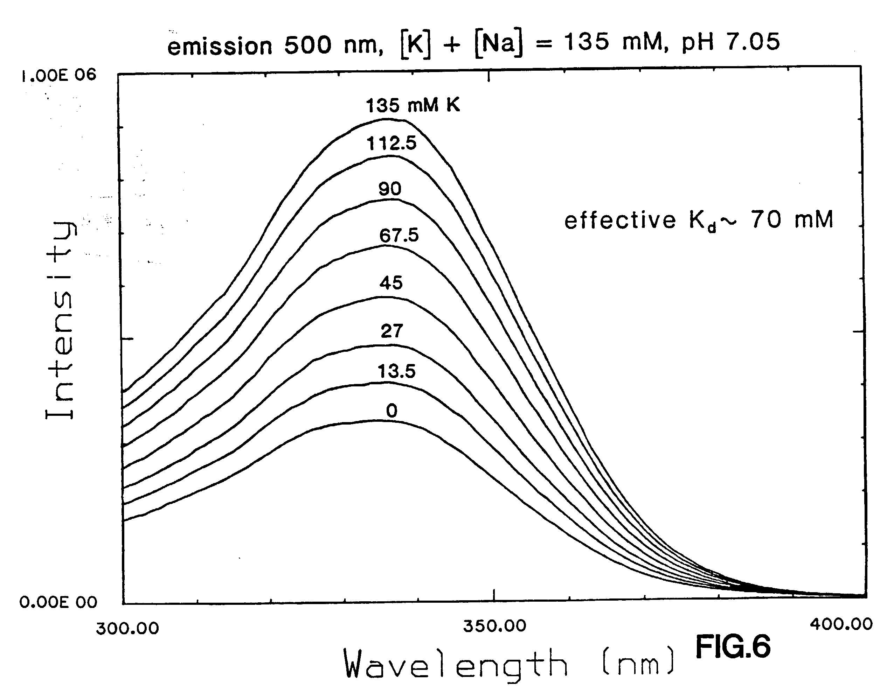

FIGURE 6 is a graph that shows the fluorescence excitation spectra of 10 µM **PBFP** as a function of increasing [K⁺] and decreasing [Na⁺] in solutions similar to those of Fig. 2. The lowest curve was obtained in 130 mM NaCl, 10 mM MOPS, NaOH to pH 7.05. The highest curve was in 130 mM KCl, 10 mM MOPS, KOH to pH 7.05. The intermediate curves with 13.5 to 112.5 mM K⁺ were obtained by iteratively replacing 1/10, 1/9, 1/6, 1/4, 1/3, and 1/2 of the low K⁺ medium by the 135 mM KCl solution of **PBFP**.

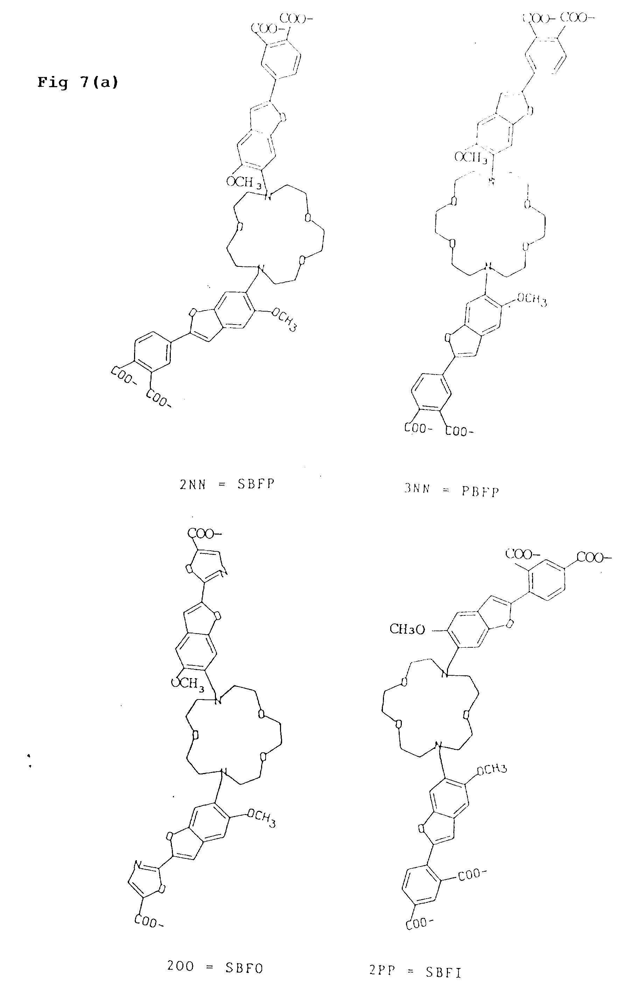

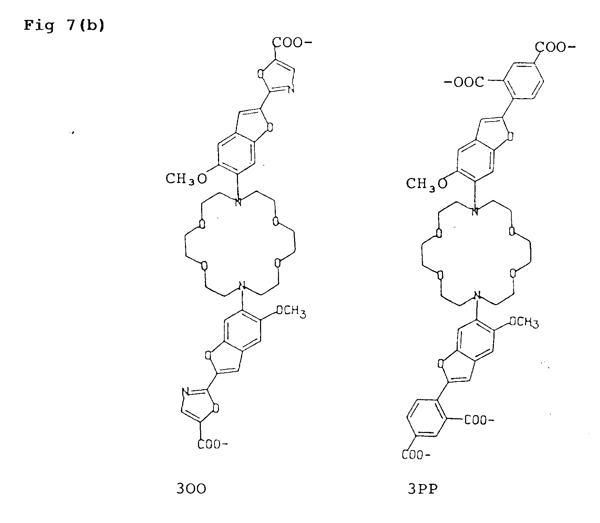

Figure 7 shows explicitly the structures of six compounds of the invention.

In the present specification and claims, reference will be made to phrases and terms of art which are expressly defined for use herein as follows:

As used herein, [Na⁺] means free sodium concentration, typically in a test solution.

As used herein, [Na⁺]ᵢ means intracellular free sodium concentration.

As used herein, crown ethers mean macrocylic polyethers with the repeating unit, (-CH₂-CH₂-Y-)ₙ, where Y is a heteroatom (*e.g.*, O, S, N, P), and n is greater than 2. In the crown ethers that are useful in making the macrocylic fluorescent compounds of the present invention, the total number of atoms in the crown ring will be at least 12, but not greater than 18; n will be at least 4, but not greater than 6; Y will be N or O, not S or P; and at least one Y will always be N. For examples that illustrate preparation of crown ethers, *see* C.J. Pedersen, *J. Am. Chem. Soc.*, **89**, 2495, 7017 (1967); *also see* United States Patent 3,687,978, which issued to C.J. Pedersen in 1972. For other examples and reviews of the crown ethers, *see* D.J. Cram, J.M. Cram, *Science*, **183**, 803-809 (1974); J.J. Christensen *et al., Chem Rev.*, **74**, 351-384 (1974); G.W. Gokel, H.D. Durst, *Synthesis*, 168-184 (1976); A.C. Knipe, *J. Chem. Ed.*, **53**, 618-622 (1976); S. Kulstad and L. A. Malmsen, *Acta Chemica Scandinavica*, **B 33**, 469-474 (1979); Macrocyclic Polyether Synthesis, Gokel, G.W., and Korzeniowski, S.H., (Eds.), Springer Verlag, Berlin (1982); D.A. Laidler and J.F. Stoddart, in The Chemistry of Ethers, Crown Ethers, Hydroxyl Groups and Their Sulphur Analogues, Supplement E, part 1, (S. Patai, ed.), pp. 1-58, John Wiley, New York (1980); F. Vögtle, F., and E. Weber, *ibid*., at pages 59-156; Progress in Macrocyclic Chemistry, Izatt, F.M. and J.J. Christensen, J.J, (Eds.), a continuing series from J. Wiley, New York.

As used herein, "aza-" is used generically to mean containing nitrogen, "monoaza-" is used more specifically to mean a crown ether that contains one nitrogen, "diaza-" means containing two nitrogens, *etc*.

As used herein, when referring to the crown ethers, nomenclature lists non-ring substituents, ring substituents, number of atoms in the ring, the class (crown), and the number of heteroatoms in the ring. For example, diaza-15-crown-5-pyridinophane would be represented by the following formula (Note: In the structures, L means ligand):

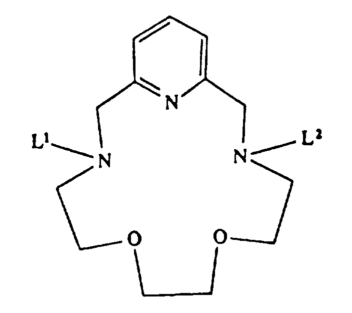

As used herein, when it is said that a diaza crown ether is "symmetrical" it means that the two nitrogens are located in positions as remote as possible from each other in the crowns. Examples of symmetrical diaza-18-crown-6 ethers would be 1,10-diaza-18-crown-6 or 1,7-diaza-15-crown-5. The formula for 1,7-diaza-15-crown-5 is given below.

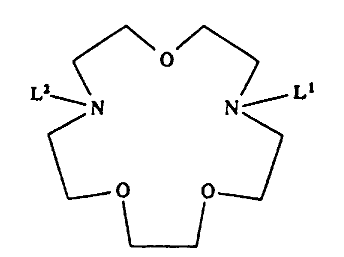

As used herein, when it is said that a diaza crown ether is "asymmetrical" it means that the two nitrogens are located in positions as close as possible to each other in the crown. Examples of asymmetrical crown ethers include: 1,4-diaza-15-crown-5, 1,4-diaza-18-crown-6, and 1,7-diaza-18-crown-6. The formula for 1,7-diaza-18-crown-6 is given below:

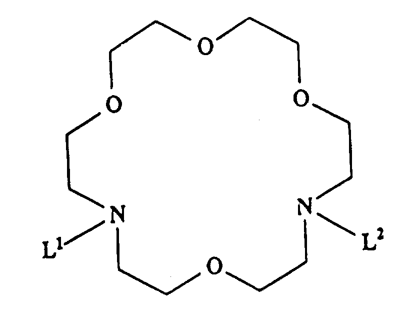

As used herein, "diaza[15]-crown-5" and "Kryptofix 21" mean the commercially available crown ether 1,7-diaza-4,10,13-trioxacyclopentadecane.

As used herein, "Kryptofix 22" means the commercially available crown ether 1,10-diaza-4,7,13,16-tetraoxacyclooctadecane.

As used herein, compounds are not always referred to by their chemical names. In some instances the compounds are referred to by their structures, *i.e.*, the structures shown in Figure 1. Such compounds are printed in bold. The compounds are named with a number and a letter, *e.g.*, **1B** where the number refers to the crown ether shown as structure 1, 2 or 3, and the letter refers to the substituents shown as structures **A-P**.

As used herein, compound "**SBFP**" means the compound whose structure is shown in Figure 1 as **2NN**, *i.e.*, the combination of crown ether **2** and two **N** substituents. (SBFP has one **N** substituent attached to each of the two core nitrogens in crown ether 2.) "**SBFP**" is short for sodium-binding benzofuran phthalate. Both **SBFP** and **2NN** are used herein to refer to this compound.

As used herein, compound "**SBFO**" means the compound whose structure is shown in Figure **1** as **2OO**, *i.e.*, the combination of crown ether **2** and two **O** substituents. (SBFO has one **O** substituent attached to each of the two core nitrogens in crown ether 2.) "**SBFO**" is short for sodium-binding benzofuran oxazole. Both **SBFO** and **200** are used herein to refer to this compound.

As used herein, compound "**SBFI**" means the compound whose structure is shown in Figure 1 as **2PP**, *i.e.,* the combination of crown ether **2** and two **P** substituents. (SBFI has one **P** substituent attached to each of the two core nitrogens in crown ether 2.) "**SBFI**" is short for sodium-binding benzofuran isophthalate. Both **SBFI** and **2PP** are used herein to refer to this compound.

As used herein, compound "**PBFP**" means the compound whose structure is shown in Figure 1 as **3NN**, *i.e.,* the combination of crown ether **3** and two **N** substituents. (PBFP has one **N** substituent attached to each of the two core nitrogens in crown ether 3.) "**PBFP**" is short for potassium-binding benzofuran phthalate. Both **PBFP** and **3NN** are used herein to refer to this compound.

As used herein, compound "**PBFO**" means the compound whose structure is shown in Figure 1 as **3OO**, i.e., the combination of crown ether **3** and two **O** substituents. (PBFO has one **O** substituent attached to each of the two core nitrogens in crown ether 3.) "**PBFO**" is short for potassium-binding benzofuran **o**xazole. Both **PBFO** and **3OO** are used herein to refer to this compound.

As used herein, compound "**PBFI**" means the compound whose structure is shown in Figure 1 as **3PP**, *i.e.*, the combination of crown ether **3** and two **P** substituents. (PBFI has one **P** substituent attached to each of the two core nitrogens in crown ether 3.) "**PBFI**" is short for potassium-binding benzofuran isophthalate. Both **PBFI** and **3PP** are used herein to refer to this compound. Structures 2NN, 3NN, 2OO, 2PP, 3OO and 3PP are shown in their entirety in Figure 7(a) and (b).

As used herein, when it is said that the "ligands" are attached to the aza-crown ether via the sp³-hybridized core nitrogen(s), it means that the ligands are attached via the nitrogen atomic orbitals that arise from the mixing of one *s* orbital and 3 *p* orbitals. *See* Organic Chemistry, Second Edition, R.T. Morrison and R.N. Boyd, Allyn and Bacon, Inc., Boston, (1970), at pages 10-18. An example of this kind of bonding is shown in the following formula for PBFI:

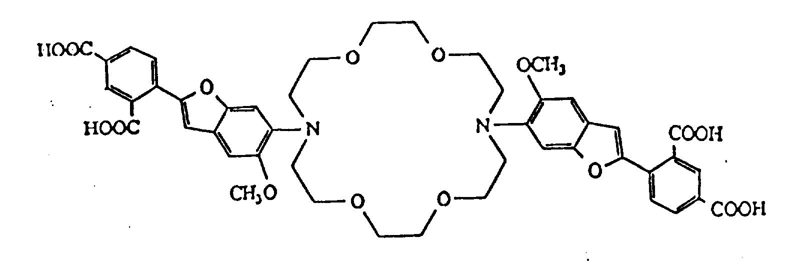

As used herein, when referring to the compounds of the present invention, when it is said that the "ligands" that are attached to the aza-crown ether (via the sp³-hybridized core nitrogen(s) of the aza-crown ether) "are identical", it means that the ligand attached to any one core nitrogen is the same as the ligand attached to any other core nitrogen. The formula shown above for **PBFI** is an example of a symmetric diaza-18-crown-6 having identical ligands attached to the two core nitrogens.

As used herein, when referring to the compounds of the present invention, when it is said that the "ligands" that are attached to the aza-crown ether (via the sp³-hybridized core nitrogen(s) of the aza-crown ether) "are different", it means that the ligand attached to any one core nitrogen is not the same as a ligand(s) attached to another core nitrogen(s). The following formula is an example of a diaza-18-crown-6 having different ligands attached to the two core nitrogens.

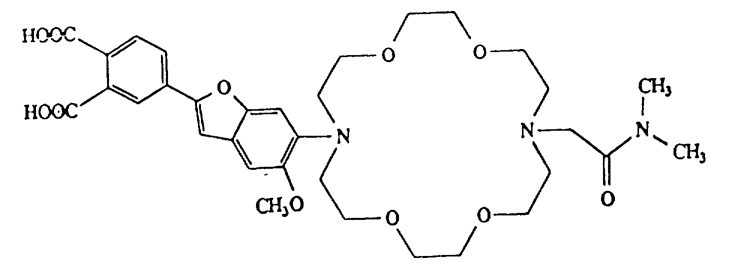

The present invention discloses new macrocyclic fluorescent chelating compounds for alkali metal cations. The new macrocyclic fluorescent chelating compounds are comprised of the combination of (1) an aza-crown ether and (2) ligand(s), at least one of which will be an aromatic or heteroaromatic fluorophore that bears an additional cation chelating center, wherein the ligand(s) is attached to the aza-crown ether via the sp³-hybridized core nitrogen(s).

In the compounds of the present invention, the crown ether is normally selected from the group consisting of: aza-12-crown-4, aza-15-crown-5 and aza-18-crown-6. According to the invention, the aza-crown ethers may be symmetric or asymmetric monoaza-, diaza-, triaza-, tetraaza-, pentaaza-, or hexaaza-crown ethers. Also according to the invention, at least one of the hydrogens on the core carbons of the crown ether may be substituted by -COOH, -CH₂OH, -C(O)N(CH₃)₂, or C₁-C₄ alkyl. Alternately, hydrogens on adjacent core carbons of the aza-crown ether may be substituted with R¹ and R (*see* Structure A below) wherein R¹ and R together are either -(CH₂)₃- or -(CH₂)₄- (*see* Structure B below, where R¹ and R are part of an aliphatic system); or R¹ and R together are -(CH)₄- *(see* Structure C below, where R¹ and R are part of an aromatic system). Finally, hydrogens on core carbons on either side of core nitrogen or oxygen atom may be substituted with R¹ and R wherein R¹ and R together are -(CH)₃- such that a pyridinium or oxonium ring is incorporated into the aza-crown ether *(see* Structure D below). In the structures, L means ligand.

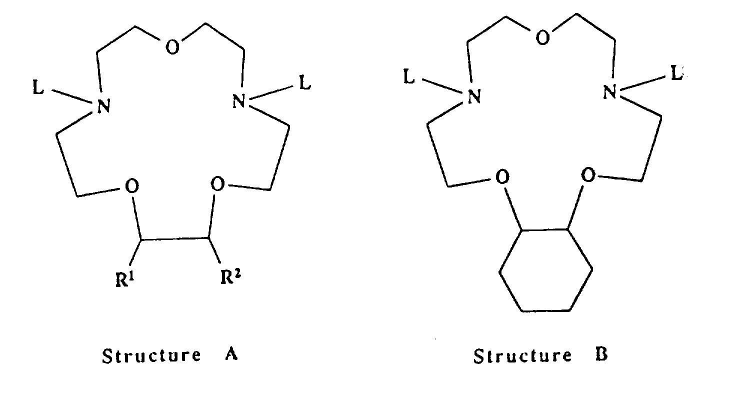

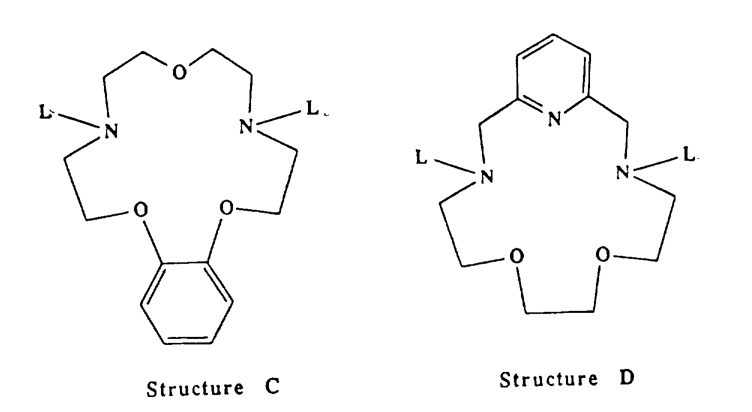

In the compounds of the present invention, the crown ethers are preferably selected from the group consisting of: monoaza-12-crown-4, diaza-12-crown-4, monoaza-15-crown-5, diaza-15-crown-5, monoaza-18-crown-6 and diaza-18-crown-6. Especially preferred crown ethers are 1,7-diaza-4,10-dioxacyclododecane, 1,7-diaza-4,10,13-trioxacyclopentadecane and 1,10-diaza-4,7,13,16-tetraoxacyclooctadecane.

According to the invention, at least one of the ligand(s) that is attached to the aza-crown ether via the sp³-hybridized core nitrogen(s) will be a heteroaromatic fluorophore that bears an additional cation chelating center. Heteroaromatic fluorophores bearing additional cation chelating centers that are used in the compounds of the present invention are shown below as formula (i) or (ii) ligands or as formula (iii) or (iv) ligands. In addition, there may be one or more non-fluorophore moieties attached to the aza-crown ether. These non-fluorophore moieties are collectively referred to as "Structure (v) ligands."

The invention accordingly provides aza-crown ethers of the formula: 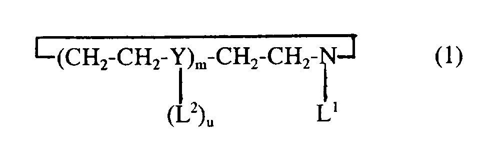 wherein:
m is an integer from 3 to 5,
each Y is independently N or O,
u is 1 if Y is N and 0 if Y is O
L¹ is a ligand of formula (i) or formula (ii) below 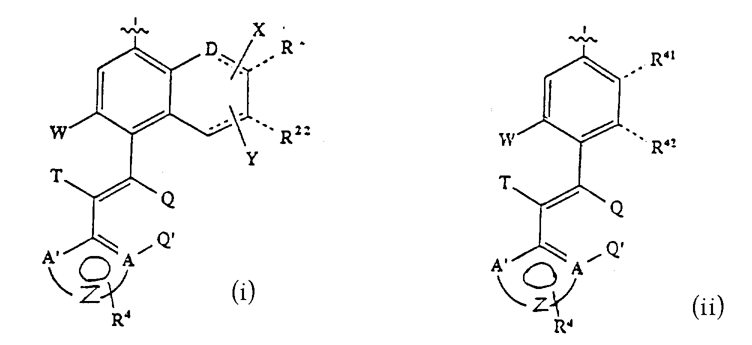
wherein:
A and A¹ are independently C, N, O or S;
D is N or O;
Q is H or NR¹R where R¹ and R are independently -H, C₁-C₄ alkyl, -CH₂COOH, -CH₂CH₂OH, or phenyl or R¹ and R together are -(CH₂)₄-, -(CH₂)₅-, or -CH₂CH₂OCH₂CH₂-;
Q¹ is H unless A is NR³, O or S; or
Q and Q¹ together are NR³, and A is C;
R³ is H, -CH₃, -C₂H₅ or CH₂COOH;
R⁴ is (E)ₙ where n = 0-3, and E is a polar electron-withdrawing function selected from -CO₂H, -CO₂R¹, -CONR¹R, -SO₃H, -SO₂NR¹R, -SO₂CF₃, -COCH₃, and -CN where R¹ and R are independently -H, C₁-C₄ alkyl, -CH₂COOH, -CH₂CH₂OH, or phenyl or R¹ and R together are -(CH₂)₄-, -(CH₂)₅-_{,} or -CH₂CH₂OCH₂CH₂-;
R¹ is -H, -CH₃, -C₂H₅, other lower alkyl up to C₄, -COOH, C₁-C₄ alkoxy, or -OC(O)CH₃; or
R¹ and R together are -CH=CH-CH=CH-, both X and Y are double bonds and D is N so that D, R¹, and R form an acridine ring system;
R⁴¹ is -OH, -OCH₂COOH, -OCH₂CH₂OH, methoxy or C₂-C₄ alkoxy,-NR¹⁴R¹⁵, -COOH, -C(O)NR¹⁴R¹⁵, or -OC(O)CH₃ where R¹⁴ and R¹⁵ are independently -H, C₁-C₄ alkyl, -CH₂COOH, -CH₂CH₂OH, or phenyl;
R⁴ is -H, -CH₃ or -COOH;
W and T are each H or, together are O or NR³;
X is a double bond when D is N, and a single bond when D is O;
Y may be either a double or a single bond;
   and
Z is a moiety forming a (hetero) aromatic ring;
and L is H, a ligand of formula (i) or formula (ii) or a structure (v) ligand.

Also provided are aza-crown ethers of the formula: 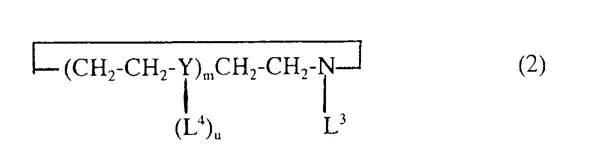 wherein:
m, Y and u are as defined above;
L³ is quinone or a ligand of formula (iii) or formula (iv) below 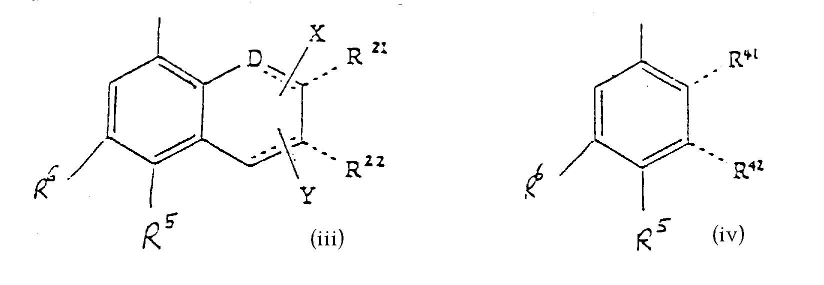
wherein:
D, R¹, R, R⁴¹, R⁴, X and Y are as defined above;
R⁵ is H,-NH₂, -CHO or -NHCOCH₃, or -NHCOCH₂CH₂COO⁻ or other acylated amino group;
R⁶ is H,-OH, or -OCH₃ or other alkoxy, provided that one of R⁵ and R⁶ is not H;
and L⁴ is H, quinone, a ligand of formula (iii) or formula (iv) or a structure (v) ligand.
R⁵ is preferably H, -NH₂, -CHO, or -NHCOCH₂CH₂COO⁻ or other amido group and more preferably H, -NH₂, -CHO or -NHCOCH₂CH₂COO⁻. It is further preferred that R⁵ is not -NH₂.
R⁶ is preferably H, -OH or -OCH₃.

Included in the invention are aza-crown ethers comprising the crown ether core of formulae (1) and (2) substituted with a combination of ligands of formula (1) compounds and ligands of formula (2) compounds.

In the compounds of the present invention, compounds that contain "Structure (v) ligands" will contain non-fluorophore ligands selected from the group of consisting of:
-CH₂C(O)NR¹R where R¹ and R are independently -H, lower alkyl (C₁₋C₄), -CH₂COOH, -CH₂CH₂OH, or phenyl (-C₆H₅), or R¹ and R together are -(CH₂)₄-, -(CH₂)₅-, -CH₂CH₂OCH₂CH₂- (to form a pyrrolidine, piperidine, or morpholine ring, respectively);
-CH₂COOH; -2-pyridylmethyl (shown below as structure 4);
-2-tetrahydrofuranylmethyl (shown below as structure 5);
-CH₂CH₂OR^{a} where R^{a} is -H, (C₁-C₄) alkyl, -CH₂COOH, -CH₂CH₂OH, or phenyl (-C₆H₅);
or -2,5-dialkoxyphenyl where the alkoxy substituent is C₁-C₄ alkoxy (shown below as structure 6).

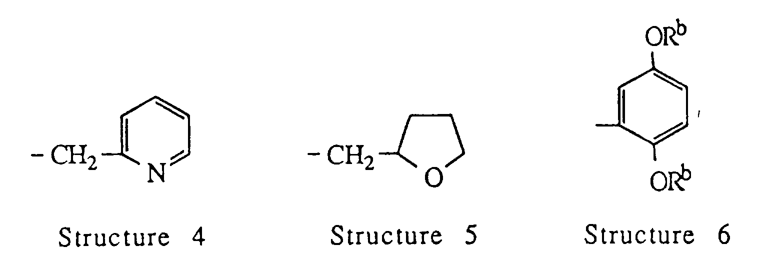

According to the invention, if a compound of the invention contains an aza-crown ether having at least two core nitrogens, the ligands (that are attached to the crown ether via sp³-hybridized core nitrogen(s)) can all be identical, or at least one ligand can be different from the other(s).

Also, according to the invention, in the compounds of the invention, any of the carboxylates can be esterified with physiologically hydrolyzable esters, preferably acetoxymethyl esters.

The invention also includes a process for preparing a macrocyclic fluorescent alkali metal chelating agent comprising forming a combination of an aza-crown ether and one or more ligands attached to the crown ether via the sp³-hybridized core nitrogen(s), wherein the crown ether is as defined above and at least one of the ligand(s) is a Structure 1 or Structure 2 ligand as defined above.

Other aspects of the invention are:-
(A) a process for preparing a compound as defined above comprising either:
   (a) effecting a method chemically equivalent to a method comprising that described hereinafter under any one of the headings "N,N-bis- (2-(3,4-dicarboxyphenyl)-5-methoxy-benzofuran-6-yl)- diaza[15]-crown-5 (**2NN** Me ester) (= **SBFP/Me**)", "N,N-bis-(2-(5-carboxy-oxazol-2-yl)-5-methoxybenzofuran-6-yl)-diaza[15]- crown-5 (**200** ethyl ester) (= **SBFO/Et**)" or "N,N-bis-(2-(2,4-dicarboxyphenyl)-5-methoxybenzofuran-6-yl)-diaza[15]-crown-5 (**2PP** methyl ester) (= **SBFI/Me**)", optionally preceded by one or more of the method steps in sequence leading up to compound 2MM as set out hereinbefore under "COMPOUND SYNTHESIS"; or
   (b) effecting a process analogous to (a) but employing as the crown ether 1,7-diaza-4, 10-dioxacyclododecane or 1,10-diaza-4,7,13,16-tetra-oxacyclooctadecane;
(B) The use of a compound as defined above or a salt or ester as defined above in the preparation of an alkali metal chelating formulation for non-destructive observation of an alkali metal ion concentrate in tissue; or
(C) A method of non-destructive observation of an alkali metal ion concentration in tissue comprising employing in said tissue, as an alkali metal chelating agent, a compound as defined above or a salt or ester as defined above and observing or measuring fluorescence.

The synthetic routes to chelators of the present invention are shown schematically in Fig. 1 and described in detail in the section entitled COMPOUND SYNTHESIS. Additional details regarding the test equipment and procedures are given in the section entitled EXPERIMENTAL PROCEDURES.

### Compound Design and Organic Synthesis

The new chelators of the present invention (which are comprised of (1) aza-crown ethers, whose core carbons may or may not be substituted with lower (C₁-C₄) alkyl, hydroxymethyl carboxy, carboxamido (including N-substituted) or saturated or unsaturated rings that are fused to the crown ring), wherein the crown ethers are linked via the core nitrogen(s) to (2) heteroaromatic fluorophores that bear additional cation liganding centers, begin with structure **1A**. Structure **1A** allows the initial combination of bare minimum of molecular parts, a macrocyclic ring of ligand groups with at least theoretically (17) the right size to favor Na⁺ over K⁺ binding, connected via an sp³-hybridized nitrogen to a rudimentary chromophore. The use of such aniline-type nitrogens to link chelating groups to chromophores has proved highly successful in the rational design of calcium indicators (16,18) and gives far larger spectral shifts than reliance on aryl ether oxygens (19).

Structure **1A** was synthesized by Dix and Vögtle (3) but not characterized for cation binding properties. In the course of the present work, the affinities of **1A** for NA⁺ and K⁺ were found to be on the order of 0.5 and 0.2 M⁻¹ in water, too weak to be characterized accurately. It was concluded that more donor groups were needed, especially out of the plane approximately defined by the macrocyclic ring.

Synthetic convenience suggested **1B**, which was made by reacting 1-aza-4,7,10,13-tetraoxacyclopentadecane with 2-chloro-5-nitrobenzoic acid, followed by catalytic reduction of the nitro group to counteract its extreme electron-withdrawing power. Compound **1B** had considerably higher Na⁺ affinity, 14M⁻¹, and Na⁺:K⁺ selectivity (13:1) than **1A**, but its pKₐ was too high, 9.21.

Comparably high pKₐ's in other N,N-dialkylanthranilates are attributed to internal hydrogen bonding between zwitterionic amino and carboxylate groups (20). To eliminate such chelation of protons, the carboxylate was abandoned in favor of an sp³-hybridized nitrogen in **1C**, in which the two nitrogens are too far apart to engage the same proton at once. Chelator **1C** was prepared by reaction of 1-aza-4,7,10,13-tetraoxacyclopentadecane with 8-tosyloxy-5-nitroquinoline followed by reduction of the nitro group. The resulting primary amino group was acetylated to block a tendency to auto-oxidize. Despite the replacement of the carboxylate of **1B** by an uncharged ligand group in **1C**, the Na⁺ affinity, 15 M⁻¹, was not diminished. As desired, the pKₐ was lowered below 7.

Since addition of one additional donor group out of the main ring plane increased the Na⁺ affinity by more than an order of magnitude, a second donor group was added, as shown in structure **2CC**. Molecular models suggested that this complex could neatly fold up around a sodium cation with the macrocyclic ring, forming an equatorial belt with the two additional donor groups capping the north and south poles. Chelator **2CC** was prepared from the commercially available 1,7-diaza-4,10,13-trioxacyclopentadecane with 8-tosyloxy-5-nitroquinoline, followed by reduction and acetylation. Compound **2CC** indeed had a greatly increased Na⁺ affinity, 190-415 M⁻¹ depending on ionic strength. Fortunately, the K⁺ affinity did not increase to the same extent. Since the spectral change associated with K⁺ binding was only half that caused by Na⁺ binding, K⁺ probably could interact with only one of the two quinoline rings, presumably because K⁺ was too big to fit fully inside the macrocyclic ring but rather had to stay on one side of it. Compound **2CC** was found, however, to have a major drawback in its excessive affinity for Mg⁺; nearly 1.3 x 10⁴ M⁻¹, which would give overwhelming interference from the typical value of 1 mM free intracellular Mg⁺. This Mg⁺-binding is unusual in its kinetic sluggishness, with association and dissociation rate constants of only 1.48 M⁻¹s⁻¹ and 1.28 x 10⁻⁴ s⁻¹ at 25 degrees, easily observable in a spectrophotometer without rapid mixing equipment. A reasonable explanation for the high affinity of Mg⁺ for **2CC** is that the binding site can readily collapse compactly to fit the small Mg⁺ ion. As confirmation of this hypothesis, **2CC** proved to have an affinity for Li⁺ comparable to that for Na⁺.

To prevent such compaction of the binding site, methyl groups were added to the quinoline 2-positions to act as buttresses to prevent the quinoline nitrogens from too closely approaching the plane of the macrocyclic ring. The resulting chelator, **2DD**, had <10⁻⁴ the Mg⁺ affinity, and <10⁻ the Li⁺ affinity of **2CC**, yet retained more than half the Na⁺ affinity. Since the two methyl substituents did not affect the Na⁺:K⁺ selectivity, they clearly made a major improvement overall. Nevertheless, though **2DD** had highly satisfactory ionic selectivities for Na⁺ over the other alkali and alkaline earth metals, it proved to lack the further properties necessary to make it biologically useful as a fluorescent indicator. Its fluorescence quantum yield was only 0.01, probably inadequate for use as a fluorescent intracellular indicator, considering its need for UV excitation and its modest extinction coefficients. Its proton affinity was undesirably high, the pKₐ for the 1st proton being 7.55. Of course, it had no carboxylates to enforce water solubility and retention inside cells.

In the hope of increasing the fluorescence quantum efficiency, several analogues of **2DD** were synthesized with 6-alkoxy substituents on the quinaldines instead of the 5-acetamido groups. The rationale was that 6-methoxyquinolinium fluorophores are responsible for the strong fluorescence of quinine. Also, addition of a 6-methoxy substituent did turn a weakly-fluorescing Ca⁺ indicator, **quin-1**, into a considerably stronger fluorescent dye, **quin-2**. (*See* United States Patent 4,689,432.) The vacancy of the 5-position would permit attachment of more extensive conjugation to extend the wavelengths of excitation and emission. However, an entirely different synthetic strategy was needed since the 5-position could not readily bear the nitro group needed to activate aromatic nucleophilic substitution. For this reason the crown macrocycle was synthesized from scratch by reaction of 6-methoxy-8-aminoquinaldine (21) with 3,6-dioxaoctanedioyl chloride (22), reduction of the diamide with diborane, re-acylation of the diamine with diglycolic acid chloride, then aluminum hydride (23) reduction of the amide. Aluminum hydride was found to give better results than diborane, since the latter gave products from which it was difficult to remove boron fully. The re-acylation with diglycolic acid chloride was run under high dilution conditions to favor macrocycle closure over polymer formation. Conventionally (22), triethylamine is added to neutralize the HCl generated during acylation, but this base itself slowly destroyed the acid chloride, presumably through ketene intermediates. When the amine to be acylated is reactive, this side reaction is not serious, but with a bulky and less nucleophilic aromatic diamine such as 1,8-bis(2-methyl-6-methoxy-8-quinolinylamino)-3,6-dioxaoctane, a weaker tertiary base such as N,N-dimethylaniline is preferable to triethylamine.

First trials of 6-substituted quinolines were conducted on the 6-hydroxyquinaldine **2FF** obtained by demethylation of the precursor **2EE** bearing methoxyls. Compound **2FF**, however, proved to have yet another disappointment in its quantum efficiency; about 0.01 and 0.005, with and without Na⁺, respectively. Therefore, **2EE** was formylated at the 5-position, demethylated, and coupled with dimethyl 4-bromomethyl phthalate (24) to form the quinolinofuran **2GG**. The Na⁺ affinity of **2GG** was even higher than that of **2DD**, perhaps aided by some long-range electrostatic attraction of the cation to the four negative charges. Unfortunately, the proton affinity likewise rose to a pKₐ of 7.9. Since the quantum efficiencies were the same low values as those of **2FF**, this approach had to be abandoned.

In order to bring about the hoped-for increase in both the wavelength and quantum efficiency of fluorescence, a shift was made from quinoline nuclei to acridines as in **2HH** and **2II**. These compounds were prepared from 4-methoxy-9-acridone by reduction to the acridine (25), nitration at the 1-position, replacement of the methoxy group by hydroxy, tosylation, reaction with 1,7-diaza-4,10,13- trioxacyclopentadecane, finishing with reductive acylation. The acetamido derivative **2HH** analogous to **2CC** and **2DD** proved insufficiently soluble in water for spectrophotometry, so the more hydrophilic hemisuccinamide **2II** was prepared. This had the highest Na⁺ affinity (3125 M⁻¹) and Na⁺:K⁺ selectivity (>500) yet obtained. Unfortunately, the pKₐ, 8.19, was also nearly a record. Though the absorbance band was indeed shifted into the visible, the fluorescence quantum yield remained very poor, so that the acridine nucleus was only accentuating the undesirable features of **2DD**.

The excessively high pKₐ's of the quinaldines and acridines seemed attributable to protonation on the heterocyclic rather than the amino nitrogens, because protonation gave rise to bathochromic shifts whereas metal cations were hypsochromic. One way to eliminate the high pKₐ is to replace the heterocyclic nitrogens by less basic donor atoms such as ether oxygens. The first attempt at such a molecule was **2JJ**, prepared by reaction of the diaza crown with 2-fluoro-5-nitroanisole (26), followed by reduction and acetylation as usual. **2JJ** indeed had the highest pKₐ at 6.31, much lower than the pKₐ's for the quinaldines and acridines. Also, protonation of **2JJ** gave a hypsochromic shift very similar to metal cation binding, confirming that the site of protonation had shifted to an amino nitrogen. Despite the decreased donor strength of oxygens compared to nitrogens, Na⁺ affinity was still respectable, 83 M⁻¹ at I = 0.15. However, **2JJ** absorbed only in the deep UV, as expected from the small size of its chromophore.

A useful fluorescent indicator would require a chromophore with a much longer conjugation path. The normal site of attachment of such conjugation would be *para* to the dialkylamino group, but that position is occupied by a nitrogen. All common substituents that extend conjugation through a -N= are significantly electron-withdrawing, so they would depress the Na⁺ affinity strongly.

To escape this quandary, an electrophile other than a nitrohalobenzene was needed that would react with amines and then be reducible to an electron-donor-substituted aromatic ring. An attractive electrophile was p-benzoquinone. By reaction (27,28) of a large excess of this cheap reagent with the diaza crown, it was possible to produce **2KK** in which each quinone bears only one amino substituent. Reduction and alkylation gave aminoquinol ether **2LL**. Vilsmeier formylation of **2LL** followed by regioselective demethylation (29) of the phenol *ortho* to the formyl gave salicylaldehyde **2MM**. From this intermediate, various benzofurans can be prepared, all representing styryl fluorophores with *cis-trans* isomerism prevented by heterocyclic ring formation. Thus **2MM** reacted with 2 moles of dimethyl 4-bromomethylphthalate to form the tetramethyl ester of the benzofuran phthalate **2NN**. Alternatively, **2MM** with ethyl 2-chloromethyloxazole-5-carboxylate gives the ester of benzofuran oxazole **2OO**, a relative of the successful fluorescent Ca⁺ indicator **fura-2**. (*See* United States Patent 4,603,209.) Finally, when **2NN** and **2OO** proved difficult to load as acetoxymethyl esters, **2PP** was synthesized from **2MM** plus 2 moles of dimethyl 4-bromomethylisophthalate. For convenience we refer to **2NN**, **2OO**, and **2PP** as "**SBFP**", "**SBFO**", and "**SBFI**" respectively, short for sodium-binding benzofuran phthalate, oxazole, or isophthalate.

The most successful compounds of the present invention synthesized and tested so far are N-substituted derivatives of commercially available 1,7-diaza-4,10,13-trioxacyclopentadecane. This crown ether (also trivially known as "diaza[15]-crown-5", "diaza-15-crown-5" or "Kryptofix 21") was chosen initially because it forms a "belt" of about the right size to fit equatorially around a Na⁺ cation (17). To make indicators for Li⁺ or K⁺ one would choose the next smaller or next larger crown ethers, 1,7-diaza-4,10-dioxacyclododecane or 1,10-diaza-4,7,13,16-tetraoxacyclooctadecane, respectively. The latter did indeed confer K⁺ selectivity, as shown by compounds **3JJ** and **3NN**.

Methods for synthesizing crowns with substituents on the carbons of the macrocyclic ring, or rings fused to the macrocycle, have been reviewed by Weber & Vögtle (1976), Laidler & Stoddart (1980) and Nakatsuji, *et al.* (1983). (*See* refs. 48-50, respectively.) Such methods can be used by those skilled in the art, without undue experimentation, to make crowns with substituents on the carbons of the macrocyclic ring, and rings fused to the macrocycle. By way of example, reaction of the bis(dimethylamide) of tartaric acid or of a dialkyl ester of tartaric acid with ethyleneimine or a N-protected derivative thereof would give an analogue of 1,8-diamino-3,6-dioxaoctane bearing ester or amide substituents on the 4 and 5 positions. (Instead of an ethyleneimine derivative, N-(2-bromoethyl)phthalimide could also be used, followed by removal of the phthalimide protecting groups using hydrazine.) This diamine would be condensed with diethyleneglycol ditosylate or a bis(2-haloethyl)ether to form a 1,7-diaza-15-crown-5 with carboxamide or ester substituents at the 11- and 12-positions of the macrocycle. Alternatively, condensation of the diamine with triethyleneglycol ditosylate or bis(2-haloethoxy)ethane would give a 1,10-diaza-18-crown-6 with carboxamide or ester substituents at the 5- and 6-positions of the macrocycle. If hydroxymethyl substituents were desired, they could be obtained by metal hydride reduction of the ester substituents. These crown building blocks would replace Kryptofix 21 (1,7-diaza-4,10,13-cyclopentadecane) and Kryptofix 22 (1,10-diaza-4,7,13,16-tetraoxacyclooctadecane) in the syntheses described above for fluorescent indicators. Upon the final mild basic hydrolysis of ester functions, any carboxamide or hydroxymethyl groups would be expected to survive unchanged, whereas any remaining ester groups derived from tartaric acid would be hydrolyzed to carboxylate groups.

Similarly, if 2,3-butanediol were used at the outset instead of a tartaric acid derivative, the result would be the addition of a pair of vicinal methyl substituents to the 11- and 12- positions of the 1,7-diaza-4,10,13-cyclopentadecane ring or the 5- and 6-positions of the 1,10-diaza-4,7,13,16-tetraoxacyclooctadecane ring. Use of catechol, 1,2-cyclohexanediol or 1,2-cyclopentanediol instead of 2,3-butanediol would eventually generate crown ethers with benzene, cyclohexane or cyclopentane rings fused to the 11- and 12-positions of the 1,7-diaza-4,10,13-cyclopentadecane ring or to the 5- and 6- positions of the 1,10-diaza-4,7,10,13-tetraoxacyclooctadecane ring.

Yet another standard permutation of the crown ether system consists of replacing one or more of the oxygen donor atoms by the nitrogen of a pyridine ring. For example, an obvious analog of 1,7-diaza-4,10,13-trioxacyclopentadecane is 5,8-dioxa-2,11-diaza[12]-(2,6)-pyridinophane, prepared either by diborane or lithium aluminum hydride reduction of 5,8-dioxa-2,11-diaza[12](2,6)-pyridinophane-1,12-dione, or by removal of tosyl groups from 2,11-ditosyl-5,8-dioxa-2,11-diaza[12](2,6)pyridinophane (*see* Reaction Scheme 1, below; *also see* ref. 48). The resulting pyridinophane could be used in place of 1,7-diaza-4,10,13-trioxacyclopentadecane in all the syntheses of fluorescent indicators described above.

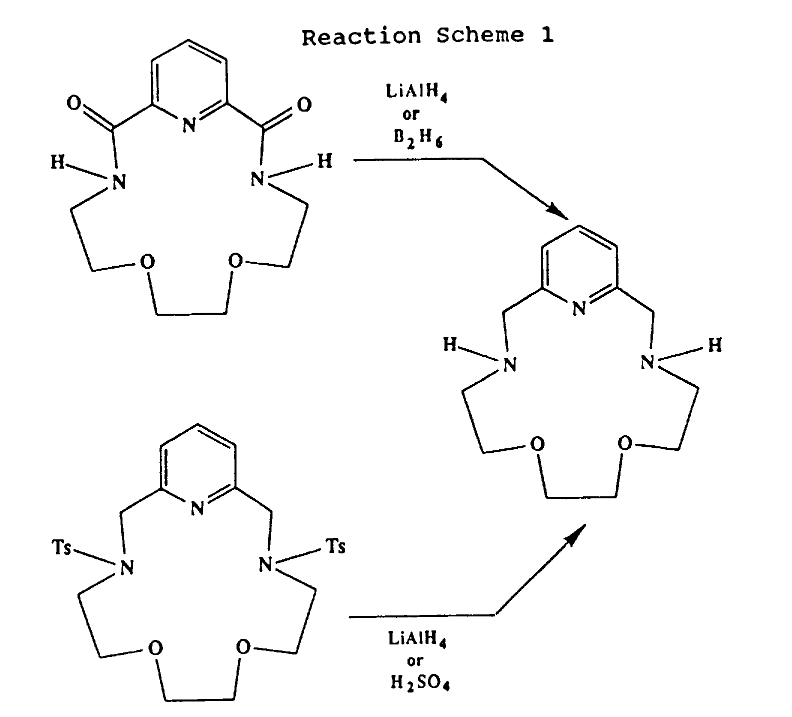

In order to prepare indicators with two different substituents attached to the nitrogens of the diaza crown ring, the most general strategy is to acylate the parent diaza crown with one equivalent of a standard protecting reagent such as acetic anhydride, trifluoroacetic anhydride, ethyl chloroformate, or benzyl chloroformate. A mixture of unreacted crown, monoamide, and diamide will form, from which the monoamide should be separable by chromatography. Both the unreacted crown and the diamide can be recycled, the latter by complete or partial deprotection. Once the monoprotected diaza crown is available, the first liganding side arm or fluorophore can be attached to the single free nitrogen in precise analogy to the reactions of 1-aza-4,7,10,13-tetraoxacyclopentadecane described above. The protecting group can then be removed by standard means to allow the second liganding side arm or fluorophore to be attached to the newly freed remaining nitrogen of the diaza crown. Of course, the use of the amine protecting groups might be bypassed by directly reacting the parent diaza crown with a limited quantity of an electrophile such as a nitrohalobenzene or benzoquinone, then separating the desired mono-substittion product from unreacted crown and di-substitution products. However, the di-substitution product would not be recyclable. In the particular case that one ligand group is a -CH₂COO⁻ or an ester or amide derived therefrom, a useful approach is to react the parent diaza crown with one equivalent of bromoacetyl bromide, BrCH₂COBr, under high-dilution conditions. This reaction is shown below as Reaction Scheme 2:

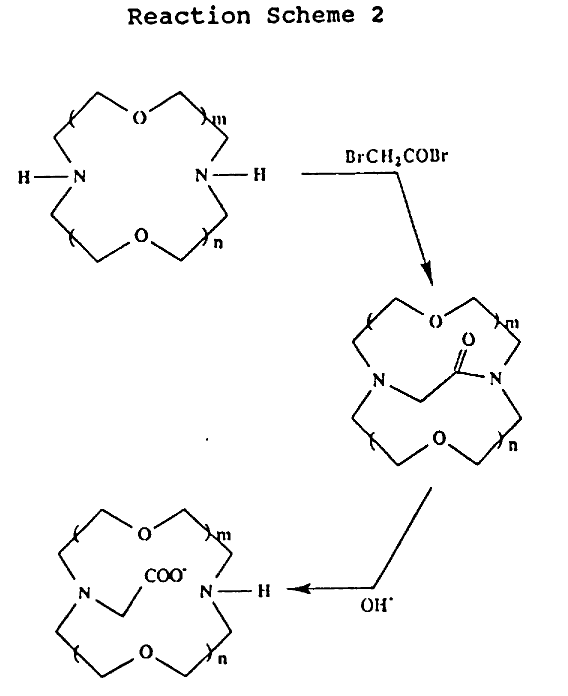

Upon hydrolysis of the amide bond, one nitrogen would be freed to allow attachment of a fluorophore by the usual reactions already described above.

The diaza crowns that do not have liganding substituents attached to the main crown ring actually have very poor cation affinities and selectivities (36) and of course lack any optical properties. Several workers have previously tried adding one liganding substituent above the plane formed by the main crown ring (37-39). Such structures, dubbed "lariats" for their shape, can have modestly improved Na⁺ affinities. Some "lariats" can extract alkali and alkaline-earth cations into 1,2-dichloroethane from aqueous medium at high pH (38,39). However, selectivities for Na⁺ over K⁺ are poor, <1 log unit, and none has been shown to respond optically to Na⁺ in a purely aqueous medium. Our initial trials of crowns with one axial substituent (**1A-1C**) confirmed their inadequate Na⁺ affinity and selectivity. However, when we increased the number of axial substitutents to two aromatic ether or sp nitrogen ligand groups, adequate to very good selectivity and affinity for Na⁺ resulted. The highest Na⁺ affinities and selectivities over K⁺ were obtained with quinoline or acridine ring nitrogens as the axial donors, but despite many permutations, all such chelators suffered from inadequate proton rejection and low fluorescence quantum efficiencies. The latter properties were greatly improved by changing the axial donors to aromatic ether oxygens. Upon extension of the aromatic rings to form suitable fluorophores, Na⁺ indicators **SBFP**, **SBFI** and **SBFO** were obtained with properties suitable for biological application. **SBFP** and **SBFI** have somewhat better Na⁺ affinity and selectivity than **SBFO**, but **SBFO** has somewhat longer wavelengths of excitation and higher quantum efficiency of fluorescence. **SBFI** is our currently preferred indicator because (1) it is the most easily loaded into cells by hydrolysis of its acetoxymethyl ester, (2) its excitation and emission wavelengths are slightly longer than **SBFP**, (3) its ionic selectivities are slightly better than **SBFP**, and (4) the brightness of its fluorescence seems adequate.

In retrospect, the Na⁺ to K⁺ selectivities (20 to 500) and Na⁺ to divalent cation selectivities (>1) of **2CC-2NN** are unexpectedly good compared to recently reported results with other derivatives of diaza[15]-crown-5 (40-43). Addition of carboxymethyl groups to both nitrogens gives a chelator dianion (40,41) with a strong preference for divalent and trivalent metals over Na⁺. Gokel and coworkers (42,43) have synthesized several "bibracchial lariat ether" derivatives of diaza[15]-crown-5. These "BiBLEs" have liganding sidearms on each nitrogen, linked through flexible aliphatic linkages rather than rigid aromatic rings. They show Na⁺/K⁺ selectivities of only 1.0 to 4.9 even though they were measured (43) in anhydrous methanol, a solvent known to foster much higher absolute affinities and selectivities (17) than the aqueous salt solutions in which biologists must work. The improved selectivities of the present compounds may at least partly arise from the rigidity of our pendant aromatic groups, which force the crown nitrogen and the pendant ligand -OMe or -N= into a *cis* conformation ready to make the desired five-membered chelate ring with the Na⁺ ion. Indeed, the bulkier and more rigid the heteroaromatic ligand, the higher the observed Na⁺/K⁺ selectivity (compare **2JJ**, **2DD**, and **2II**).

Though **SBFI** is at the low end of the selectivity range, it remains useful inside cells for two reasons. Firstly, Na⁺ binding affects its spectra more strongly than does K⁺ binding, so that replacement of a bound K⁺ by a Na⁺ is spectroscopically visible. Secondly, under most circumstances cellular Na⁺ and K⁺ are not free to vary independently but are constrained by osmotic balance and electroneutrality to have a constant sum which is approximately known (31,44). Under these conditions, [Na⁺]ᵢ can be calibrated even when it is <10% of [K⁺]ᵢ. The main effect of the imperfect selectivity is to compress the dynamic range of the fluorescence signal. Calibration and interpretation would become more problematic if the cells were subjected to drastic changes of osmolarity or ionic substitutions.

Higher Na⁺ affinities and selectivities are known to be attainable in cryptands (5,22) and spherands (8,9), chelators whose metal binding sites are more rigidly defined in three dimensions than those of the modified crown ethers exploited in this work. The choice was made, originally, to avoid these more elaborate structural types mainly because the conformational rigidity and preorganization of their cavity would tend to reduce the spectroscopic shift upon metal binding. The large spectral shifts obtained with our crown ethers are analogous to protonation of the aromatic amino groups or to Ca⁺ binding to tetracarboxylate indicators like **fura-2**. Such shifts are consistent with a mechanism in which cation binding causes a major loss of conjugation between the amino groups and the rest of the chromophore, at least partly by twisting the >N-Ar bond (13,16,18).

Molecular models of analogous cryptands suggested to us that the >N-Ar bond would start mostly twisted even before metal binding. Indeed, in one cryptand indicator of Na⁺, the spectral shifts were modest, at most 30% change in intensity at any wavelength (6). However, a more recent cryptand has been shown (7) to give a large emission shift upon Na⁺ binding, a finding that suggests that in the excited state there is more >N-Ar conjugation for Na⁺ to disrupt. Spherands are even larger, more hydrophobic, and more rigid. The main mechanism by which they can give optical shifts upon metal binding is by ejection of a proton from the cavity (9), but this mechanism inherently must be pH-sensitive, an unwanted feature. The three-dimensional rigidity of both cryptands and spherands also tends to slow the kinetics of association and dissociation of cations; dissociation time constants of seconds to days often result (45). Finally, it should be noted that excessive Na⁺ affinity (9) would actually be undesirable in an indicator intended to signal free [Na⁺] concentrations without perturbing them. Instead, the dissociation constant should ideally be at the middle of the range of physiological concentrations of interest, therefore in the 5-20 mM range as actually achieved in **SBFP** and **SBFI**.

### Spectral and Cation-Binding Properties

SBFI has extinction coefficients of 42,000-47,000, as expected for molecules containing two styryl chromophores. Its fluorescence quantum efficiency is respectable, 0.08 and 0.045 with and without Na⁺. Both the Na⁺ affinity and K⁺ affinities, 166 M⁻¹ and 7 M⁻¹, are a little stronger than those of the model compound **2JJ**. Some or all of this increment may just be the electrostatic attraction of the four remote carboxylates for cations. However, the highest pKₐ, 6.1, is still low enough so that most physiological pH variations will have little effect on the dye spectra or effective Na⁺ affinity. To enable physiochemical comparison with earlier chelators, the above affinities were measured against truly inert background cations such as tetramethylammonium or Cs⁺, at a pH high enough for protonation to be completely negligible. Biologically more relevant values are obtained in Na⁺-K⁺ mixtures. When the sum of the two cations is held constant at 135 mM, with 1 mM Mg⁺ present at pH 7.05 as would be reasonable for vertebrate cytoplasm, the apparent dissociation constant for Na⁺ is 17-19 mM, as may be seen in Fig. 2A. Na⁺ binding shifts both the excitation and emission spectra to shorter wavelengths. Though Na⁺ shifts the excitation peak wavelength only 8 nm from 344 to 336 nm, it also makes the long-wavelength side of the excitation spectra roll off much more steeply. The ratios of excitation efficiencies at 335-340 to that at 375-380 nm therefore undergoes a large increase with Na⁺ binding. This sort of spectral shift is roughly similar to the way **fura-2** responds to Ca⁺, except that **SBFP** and **SBFI** are excited at slightly shorter wavelengths and do not change their ratio quite as much as fura-2 does (13). The emission spectra of **SBFP** and **SBFI** (Fig. 3) shift very little as Na⁺ replaces K⁺, so that these dyes are like **fura-2** in being most sensitive in excitation ratioing rather than emission ratioing (13).

Selectivities of **SBFP** and **SBFI** against other cations are also adequate (Table I). In the presence of 13.5 mM Na⁺ and 121.5 mM K⁺, the highest apparent pKₐ of SBFI is 6.09. Moreover, acidification depresses the 335-340 nm and the 375-380 nm excitation amplitudes about equally (Fig. 4), so that the ratio does not change significantly. Ratioing therefore not only normalizes for amount of dye in the optical path but also improves the discrimination against pH changes. Li⁺ binding causes a greater shift of the **SBFI** excitation peak to shorter wavelengths but a lesser increase in amplitude than Na⁺ binding does; the Li⁺ affinity, 15 M⁻¹ (K_{d} 67 mM), is also weaker than the Na⁺ affinity. Dissociation constants for Mg⁺ and Ca⁺ are high enough (about 60 mM and 38 mM respectively) for cytosolic levels of those ions to have insignificant effect. Curiously, Ca⁺ is unique in causing a large hypsochromic shift of the emission peak, to 432 nm. All the above-mentioned salts except tetramethylammonium seem to cause a slight non-specific quenching of **SBFI** at very high concentrations. For example, CsCl causes no spectral shift at all, but 100, 200, and 500 mM CsCl depress the fluorescence by 9, 14, and 21% from the metal-free level. This effect is not a heavy-atom effect of Cs, since large excesses of Li⁺, Na⁺, and K⁺ also slightly quench their **SBFI** complexes. It may represent weak quenching by Cl⁻, since acetate and fluoride gave much less of the effect.

Compared with **SBFP** and **SBFI**, **SBFO** has even higher quantum efficiencies of fluorescence, 0.44 and 0.14 with and without Na⁺. Because the oxazole group in **SBFO** is more electron withdrawing than the phthalate in **SBFP**, **SBFO** has somewhat longer wavelengths of excitation and emission as well as a reduced affinity for Na⁺, 20 M⁻¹. This 50 mM dissociation constant with tetramethylammonium as background ion rises to 95 mM when measured against a K⁺ background with [Na⁺] + [K⁺] = 135 mM (Fig. 5). Again, Na⁺ binding causes a large change in the ratio of excitation efficiencies at 340-350 nm to 380-390 nm, rather like the effect of Ca⁺ on the related **fura-2**. Competition from protons is also reduced, with a highest pKₐ of only 5.34.

To check the basis for the Na⁺ selectivity of the above indicators, we synthesized **3JJ-3NN**, analogs of **2JJ-2NN** but with six heteroatoms in an 18-membered ring instead of the usual five heteroatoms in a 15-membered ring. As expected, the increased cavity size made K⁺ the preferred cation, though by only a small margin over Na⁺ (Table I). **3NN** is named "**PBFP**" for **p**otassium-binding **b**enzo**f**uran **p**hthlate. Its excitation spectra are shown in Fig. 6 under the usual conditions of [Na⁺] + [K⁺] = 135 mM. Now it is increasing K⁺ or decreasing Na⁺ that enhances the fluorescence at 340 nm excitation. The apparent K_{d} for K⁺ is 70 mM. Curiously, K⁺ increases the intensity without much change in wavelength, so that **PBFP** works best at a single wavelength rather than in dual-wavelength ratio mode. Though its K⁺:Na⁺ selectivity is small, it may find some use for intracellular (though not extracellular) measurements because [K⁺]ᵢ usually far exceeds [Na⁺]ᵢ.

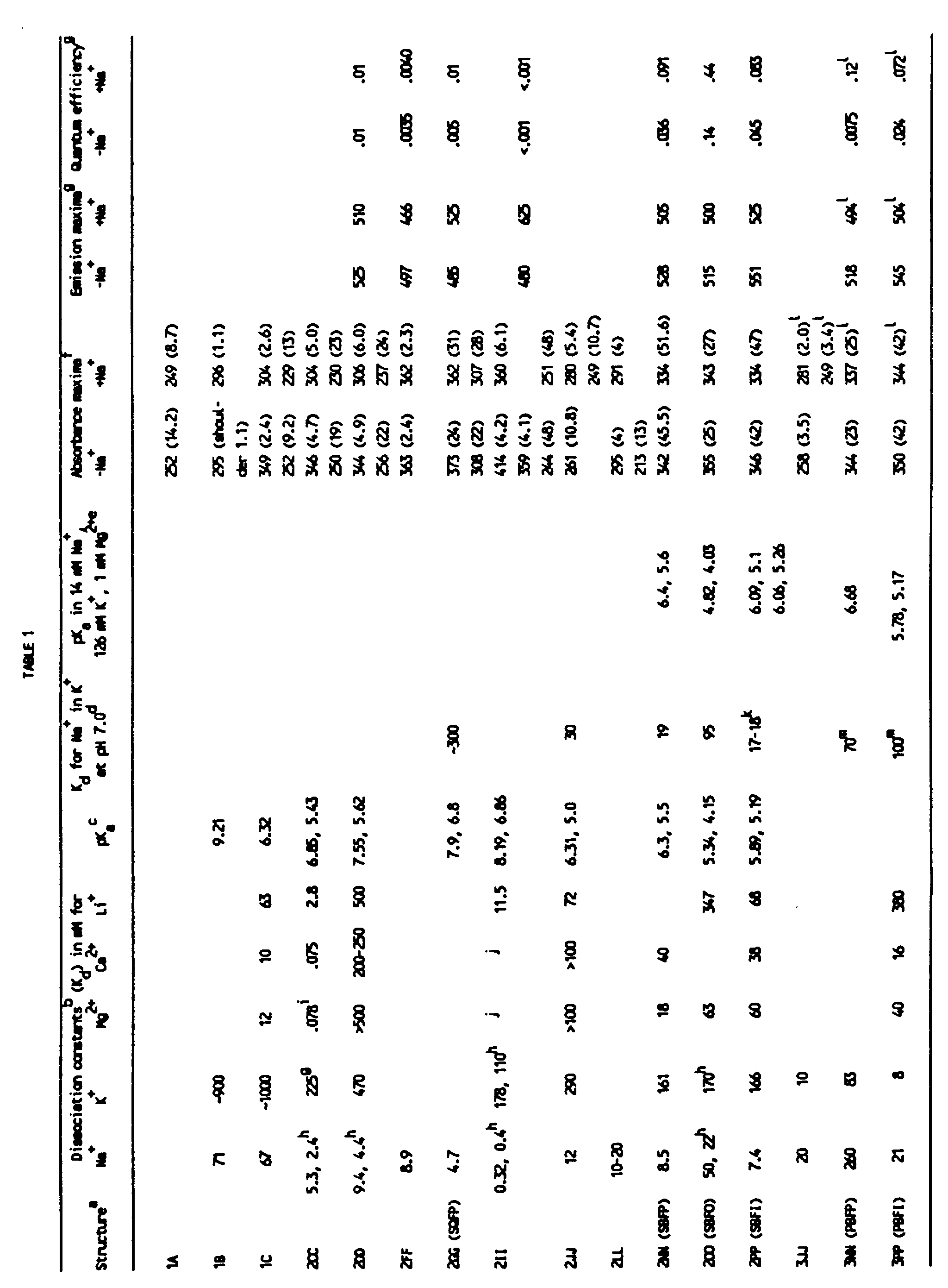

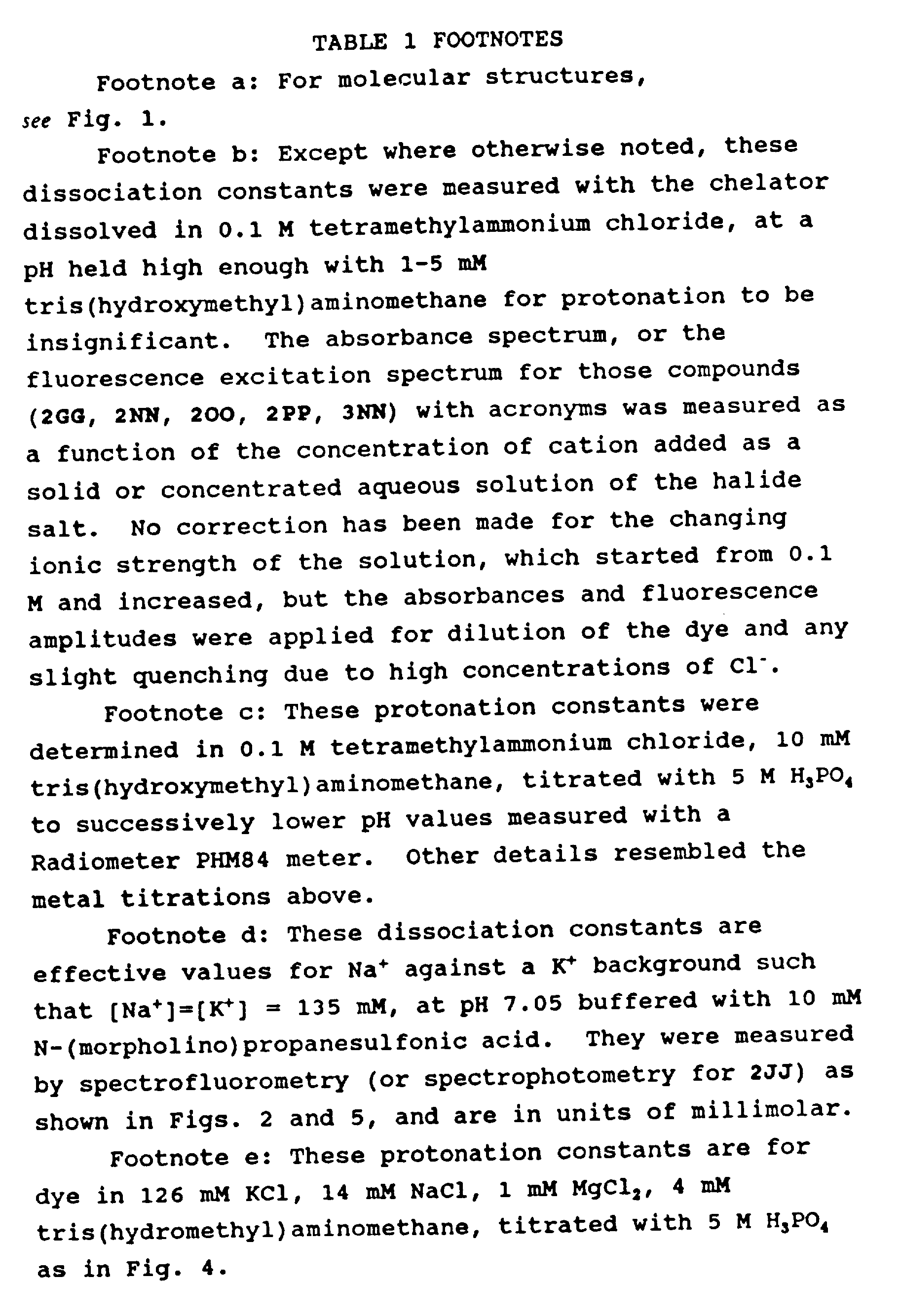

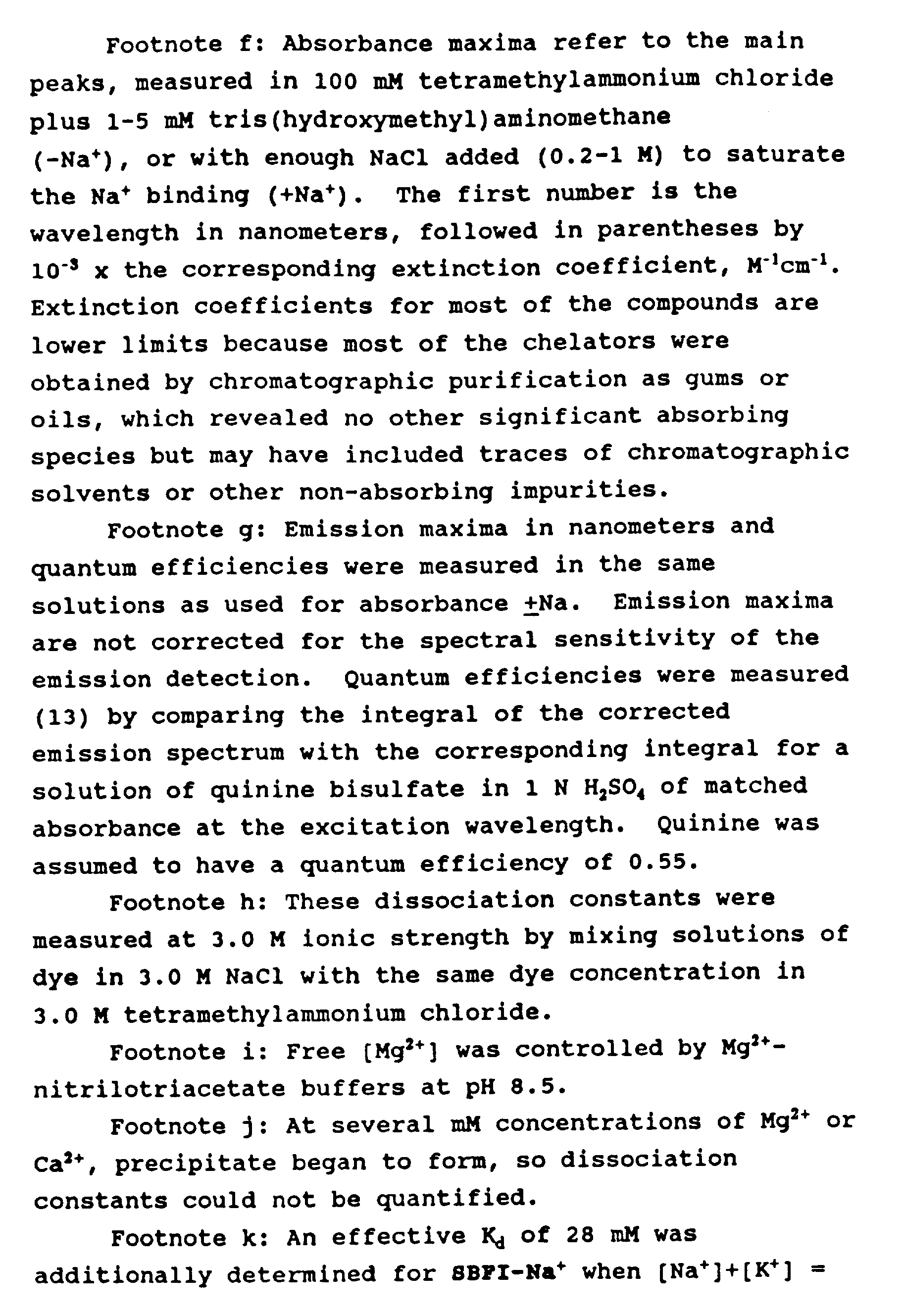

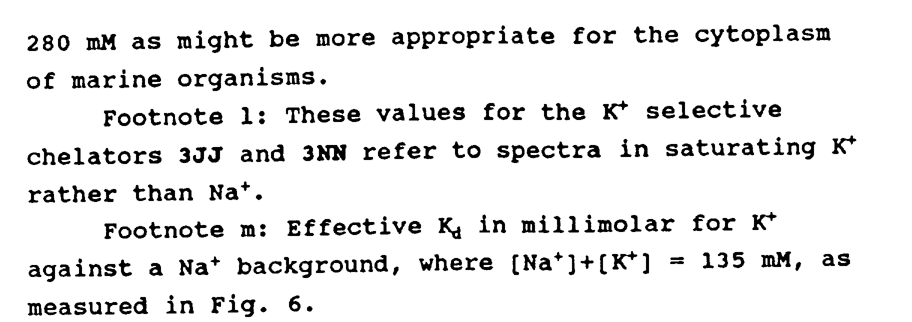

### EXPERIMENTAL PROCEDURES

UV absorbance spectra were recorded initially on a Cary 210 and later on a Perkin-Elmer Lambda Array 3840 spectrophotometer. Fluorescence excitation and emission spectra and quantum efficiencies were measured on a Spex Fluorolog 111 as described previously (13).

Proton dissociation constants of the chelators were measured by spectrophotometry or spectrofluorometry of buffered solutions, containing either 100 mM tetramethylammonium chloride as inert supporting electrolyte, or 121.5 mM K⁺, 13.5 mM Na⁺, and 1 mM Mg⁺ to simulate the cation environment of vertebrate cytoplasm (14). Traces of UV-absorbing impurities in the tetramethylammonium chloride (Alfa Inorganics) were removed by filtration through acid-washed activated charcoal. The concentration of the tetramethylammonium chloride was then measured by chloride titration, and the absence of significant Na⁺ contamination was verified with a sodium-selective glass electrode (Microelectrodes Inc.). When the chelators contained two protonatable nitrogens, the curve of absorbance or fluorescence versus pH was analyzed by computerized least-squares fitting to the equations for two arbitrary proton equilibria (15), with the added assumption that the two protonations each caused the same change in extinction coefficient or fluorescence. This assumption was based on the presence of two identical chromophores in each chelator and produced good fits to the experimental data.

Affinities for other cations were usually measured by titrating aqueous solutions of the indicator with metal chlorides added either as solid or from concentrated stock solutions, taking care to minimize dilution and correct for it. A convenient way to mass-produce premeasured micromole to millimole aliquots of solid NaCl or KCl was to pipet saline solutions into polypropylene micro test tubes and evaporate the water in an oven. The standard ionic background was again 100 mM tetramethylammonium chloride, usually with a few mM of tris(hydroxymethyl)-aminomethane base to hold the pH high enough (pH 8-9) to insure that protonation was negligible. This convenient titration procedure had the slight disadvantage that the ionic strength was not constant, especially when large quantities of a salt had to be added due to weak affinities. However, even drastic alterations in ionic strengths were found to have relatively little effect on apparent dissociation constants (Table I), as expected for monovalent cations binding to uncharged chelator sites. When constant ionic strength was desired, the chelator was made up at identical concentrations in matched solutions of NaCl, KCl, or tetramethylammonium chloride, then these stocks were mixed in the desired proportions. All measurements were made at room temperature (22°, ±2° C).

### COMPOUND SYNTHESIS

Proton NMR spectra were recorded on a Varian Instruments EM-390 at 90 MHz and on a 200 MHz Fourier-transform instrument (UCB-200) constructed in the Department of Chemistry, University of California, Berkeley. Peaks are reported below in the following format: NMR (solvent, operating frequency): chemical shift δ in ppm downfield from tetramethylsilane, multiplicity (s = singlet, d = doublet, dd = doublet of doublets, t = triplet, q = quartet, m = multiplet, br = broad), spin-spin coupling constant if appropriate, integrated number of protons; sometimes several adjacent peaks are too close for their integrals to be separated, in which case only the total integral for a cluster is stated. Column chromatography, reverse-phase thin layer chromatography, and preparative layer chromatography were done on EM Sciences (Cherry Hill, NJ) types 9385, 15687, and 5717 media respectively; centrifugal chromatography was performed on 1 mm layers cast from EM Sciences 7749 silica mounted in a model 7924T Chromatotron (Harrison Research, Palo Alto, CA). Unless otherwise stated, temperatures are in degrees Centigrade.

### N-(4-amino-2-carboxyphenyl)-aza[15]-crown-5 (1B)

2-Chloro-5-nitrobenzoic acid (Aldrich) (50.5 mg, 0.25 mmole) and 1-aza-4,7,10,13-tetraoxacyclopentadecane (46) (220 mg, 1 mmole) were heated together under reflux in pyridine (1 ml) overnight. The reaction mixture was evaporated to dryness *in vacuo* and purified by preparative thin layer chromatography (silica gel) to give N-(4-nitro-5-carboxyphenyl)-aza[15]-crown-5 as a yellowish brown gum (100 mg, 26% yield). NMR (CD₃OD, 90 MHz) δ 8.25, d, 3 Hz, 1H; 8.10, dd, 7 Hz, 3Hz, 1H; 7.55, d, 7 Hz, 1H; 3.65, s + m, 16H; 3.20, t, 4H. The nitro-compound (60 mg) was dissolved in ethanol (2 ml) and hydrogenated at room temperature and atmospheric pressure with 15 mg palladium (5% on charcoal) catalyst. After full hydrogen uptake the mixture was filtered and the solvent evaporated *in vacuo* to give an off-white solid (50 mg, 94% yield) of **1B**. NMR (CD₃OD, 90 MHz) δ 6.93, d, 7 Hz, 3 Hz, 1H; 3.50, s + m, 16H; 3.10, t, 4H.

### N-(5-acetamido-8-quinolinyl)-aza[15]-crown-5 (1C)

8-Tosyloxy-5-nitroquinoline (80 mg, 0.25 mmole) [prepared by tosylation of commercial 8-hydroxy-5-nitroquinoline in pyridine] and 1-aza-4,7,10,13-tetraoxacyclopentadecane (220 mg, 1 mmole) were heated together under reflux in pyridine (2 ml) overnight. The reaction mixture was evaporated to dryness *in vacuo* and purified by preparative thin layer chromatography (silica gel) to give N-(5-nitro-8-quinolinyl)-aza[15]-crown-5 as a brown gum (105 mg, 25% yield). NMR (CDCl₃, 90 MHz) δ 8.90, dd, 9 Hz, 2 Hz, 1H; 8.45, d, 3 Hz, 1H; 8.09, d, 9 Hz, 1H; 7.25, dd, 9 Hz, 3 Hz, 1H; 6.72, d, 9 Hz, 1H; 3.72, m, 8H; 3.30, s + m, 12H. The nitro compound (50 mg) was dissolved in acetic anhydride and hydrogenated at room temperature and atmospheric pressure with 10 mg palladium (5% in charcoal) catalyst. After full hydrogen uptake the mixture was filtered and the solvent evaporated to give **1C** as a brown solid (40 mg, 80%). NMR (CD₃OD, 90 MHz) δ 2.30, s, 3H; 3.40, m, 16H; 3.60, m, 4H; 7.20, m, 2H; 7.60, d, 9 Hz, 1H; 8.50, dd, 9 Hz, 2 Hz, 1H; 8.40, m, 1H.

### N,N-bis-(5-acetamido-8-quinolinyl)-diaza[15]-crown-5 (2CC)

8-Tosyloxy-5-nitroquinoline (0.85 g, 2.5 mmole) and commercial 1,7-diaza-4,10,13-trioxacyclopentadecane (Kryptofix 21, EM Sciences) (0.19 g, 0.8 mmole) were heated together under reflux in pyridine (5 ml) overnight. The reaction mixture was evaporated to dryness *in vacuo* and purified by preparative thin layer chromatography (silica gel) to give N,N-bis-(5-nitro-8-quinolinyl)-diaza[15]-crown-5 as a brown gum (180 mg, 32%). NMR (CDCl₃, 90 MHz) δ 9.35, dd, 2 Hz, 8 Hz, 1H; 8.70, dd, 2 Hz, 3 Hz, 1H; 8.40, d, 9 Hz, 1H; 7.5, dd, 8 Hz, 3 Hz, 1H; 6.89, d, 9 Hz, 1H; 4.10, m, 16H; 3.70, s, 4H. The nitro compound (60 mg) was dissolved in acetic anhydride (2.5 ml) and hydrogenated at room temperature and atmospheric pressure with 20 mg palladium (5% on charcoal) catalyst. After full hydrogen uptake, the mixture was filtered and the solvent evaporated to give **2CC** as a light brown solid (50 mg, 83%). NMR (CD₃OD, 90 MHz) δ 8.50, dd, 2 Hz, 8 Hz, 2H; 8.20, m, 2H; 7.85, d, 5 Hz, 2H; 7.40, m, 4H; 3.78, s, 4H; 3.48, m, 16H; 3.30, s, 6H.

### N,N-bis-(5-acetamido-2-methyl-8-quinolinyl)-diaza[15]-crown-5 (2DD)

8-Tosyloxy-5-nitro-2-methylquinoline (0.8 g, 2 mmole), prepared by tosylation of 5-nitro-2-methyl-8-quinolinol (47), was heated under reflux with 1,7-diaza-4,10,13-trioxacyclopentadecane (0.110 g, 0.5 mmole) in pyridine (5 ml) overnight. The reaction mixture was evaporated to dryness *in vacuo* and purified by preparative thin layer chromatography (silica gel) to give N,N-bis-(5-nitro-2-methyl-8-quinolinyl)-diaza[15]-crown-5 as a reddish brown gum (85 mg, 29%). NMR (CDCl₃, 90 MHz) δ 9.25, d, 9 Hz, 2H; 8.45, d, 9 Hz, 2H; 7.35, dd, 9 Hz, 2 Hz, 2H; 6.85, dd, 9 Hz, 2 Hz, 2H; 4.00, m, 16H; 3.60, t, 4H; 2.62, s, 3H. The nitro compound (60 mg) was dissolved in acetic anhydride (5 ml) and hydrogenated at room temperature under atmospheric pressure with 0.02 g palladium (5% on charcoal) catalyst. After full hydrogen uptake the mixture was filtered and the solvent evaporated *in vacuo* to give **2DD** as a brown solid (0.04 g, 78%). Mass spec. (FAB) m/e = 615 (M⁺ + 1).

### 1,8-bis-6-methoxy-2-methylquinolinyl-8-amino) -3,6-dioxaoctane

6-Methoxy-2-methyl-8-aminoquinoline [prepared by the method of Wan et al (21)] (3 g, 16 mmole) was dissolved in chloroform (25 ml) and triethylamine (8ml) was added. Then 3,6-dioxaoctanedioyl chloride (22) (2.6 g, 12 mmole) in chloroform (5 ml) was added slowly with stirring under nitrogen. After 30 minutes the reaction mixture was diluted with more chloroform and washed with sodium bicarbonate solution and then brine. The chloroform solution was then passed through a plug of alumina and then evaporated *in vacuo* to obtain *N.N*-bis-(6-methoxy-2-methyl-8-quinolinyl)-3,6-dioxaoctane-1,8-diamide as a white solid (2.4 g, 60%). M.p. 191-193°. NMR (CDCl₃, 90 MHz) δ 8.2, d, 3 Hz, 2H; 7.58, d, 7 Hz, 2H; 6.95, d, 7 Hz, 2H; 6.45, d, 3 Hz, 2H; 4.20, s, 4H; 3.92, s, 4H; 3.72, s, 6H; 2.50, s, 6H.

The amide (3 g, 6 mmole) was dissolved in dry tetrahydrofuran (50 ml) and aluminum hydride (23) solution (0.6M) in tetrahydrofuran (70 ml) was added slowly, and stirred over a period of two hours. Tetrahydrofuran-H₂O (1:1, 100 ml) was added followed by ether (200 ml). Stirring was continued for 30 more minutes and sodium hydroxide solution (20%) was added. The total reaction mixture was extracted with ether and the combined organic extracts were evaporated to dryness. The residue obtained was purified by column chromatography (silica gel, ethyl acetate:hexane: triethylamine, 75:25:1 v/v) to give the amine as a white solid (1.6 g, 57%). M.p. 89-91°. NMR (CDCl₃, 90 MHz) δ 7.85, d, 7 Hz, 2H; 7.20, d, 7 Hz, 2H; 6.35, s, 2H; 3.90, s, 6H; 3.80, t, 4H; 3.50, t, 4H; 2.70, s, 6H.

### N,N-bis-6-methoxy-2-methyl-8-quinolinyl)-diaza[15]-crown-5 (2EE)

The above amine (2.25 g, 4.5 mmole) was dissolved in chloroform (200 ml) containing dimethylaniline (2.5 ml) and acylated under high dilution conditions (5 drops per second) with diglycolic acid chloride (855 mg, 5 mmole) in chloroform (200 ml). The reaction mixture was evaporated *in vacuo* and the residue purified by column chromatography (silica gel) to give the amide as a white solid (1.8 g, 70%). M.p. 231-233°. NMR (CDCl₃, 90 MHz) δ 8.15, d, 9 Hz, 2H; 8.00, m, 2H; 7.40, d, 9 Hz, 2H; 7.30, d, 3 Hz, 2H; 4.25, s, 6H; 4.00, s + m, 20H; 2.85, s, 6H.

The macrocyclic amide (1.2 g, 2 mmole) was dissolved in dry THF (120 ml) and aluminum hydride (0.6M) in THF (25 ml) was added slowly and stirred over a period of two hours. THF-H₂O (1:1, 100 ml) was added, followed by ether (200 ml). Stirring was continued for 30 more minutes and sodium hydroxide solution 20% was added. The total reaction mixture was extracted with ether and the combined ether extracts were evaporated to dryness. The residue obtained was purified by column chromatography (silica gel) to give **2EE** as a whitish foam (340 mg, 30%). NMR (CDCl₃, 90 MHz) δ 7.75, d, 9 Hz, 2H; 7.05, d, 9 Hz, 2H; 6.70, d, 3 Hz, 2H; 6.48, d, 3 Hz, 2H; 3.70, s + m, 26H; 2.50, s, 6H.

### N,N-bis-(6-hydroxy-2-methyl-8-quinolinyl)-diaza[15]-crown-5 (2FF)

N,N-bis-(6-methoxy-2-methyl-8-quinolinyl)-diaza[15]-crown-5 (50 mg, 0.9 mmole) was dissolved in dry tetrahydrofuran (1 ml) and added to a solution (2 ml) of diphenylphosphine 0.5 ml in 1.5 ml of dry THF and 0.35 ml of 9.5M n-butyllithium in hexane. The mixture was stirred for three hours and water was added. It was then extracted three times with chloroform-methanol 9:1 and two times with ethyl acetate. The combined organic extracts were evaporated to dryness and triturated with hexane. The residue of **2FF** was purified by centrifugal chromatography with chloroform-methanol (4:1 v/v).

### N,N-bis(2-(3,4-dicarboxyphenyl)-7-methylfuro [3,2-f]quinolin-5-yl)-diaza[15]-crown-5 (2GG methyl ester) (= SQFP/Me)

The above hydroxyquinoline was used directly for formylation by dissolving it in dimethylformamide (300 µl) and adding 0.5 ml of a 1:4 (v/v) mixture of POCl₃ and dimethylformamide. After stirring for two hours, water was added to quench the reaction mixture followed by saturated potassium carbonate to basify the solution. The reaction mixture was then extracted three times with chloroform and the combined chloroform extracts were back-washed with water and evaporated to give the salicylaldehyde as a yellow gum. The gum was purified by centrifugal chromatography with ethyl acetate to give the salicylaldehyde derivate (20 mg, 38%). NMR (CDCl₃, 90 MHz) δ 10.30, s, 1H (aldehyde); 8.30, d, 9 Hz, 2H; 7.15, d, 9 Hz, 2H; 6.45, s, 2H; 3.90, m, 16H; 3.60, s + m, 4H; 2.50, s, 6H.

The salicylaldehyde (10 mg, 0.017 mmole), potassium carbonate (40 mg), dimethyl 4-bromomethylphthalate (24) (14 mg, 0.05 mmole) and dimethylformamide (500 µl) were heated together at 140° (bath temperature) for four hours. The mixture was allowed to cool and chloroform with 10% methanol was added. The entire mixture was washed with water and evaporated *in vacuo*. The residue was purified by centrifugal chromatography with chloroform-methanol (9:1 v/v) to give **SQPF (2GG)** methyl ester as a light brown gum (5.5 g, 33%). NMR (CDCl₃, 200 MHz) δ 2.75, s, 6H; 3.80, s + m, 32H; 8.10, d, 2H; 7.50, d, 2H; 7.60-7.80, s + m, 8H.

### N,N-bis-(1-acetamidoacridin-4-yl)-diaza[15]-crown-5 (2HH) and N,N-bis-(1-succinamidoacridin-4-yl)-diaza[15]-crown-5 (2II)

4-Methoxyacridine (1.4 g, 6.67 mmole) was dissolved in acetic anhydride (5 ml) and cooled to 0°. Concentrated nitric acid (1 eq, 0.7 ml) was added, followed by concentrated sulphuric acid (1 eq, 0.7 ml). After stirring for 1 hr, the product was filtered and the residue was extracted into methylene chloride and washed with sodium bicarbonate solution. It was then dried and evaporated *in vacuo* to give 1.2 g (70%) of 4-methoxy-1-nitroacridine. NMR (CDCl₃, 90 MHz) δ = 4.20, s, 3H; 6.95, d, 9 Hz, 1H; 6.70-8.00, m, 3H; 8.34, d, 9 Hz, 1H; 8.55, d, 9 Hz, 1H; 9.82, s, 1H. The methoxy compound (500 mg) was converted to the 4-hydroxy derivative by heating with KOH (2 ml of an 11 M aqueous solution) in DMSO (20 ml). This was tosylated with pyridine and toluenesulfonyl chloride to give the 4-tosyloxy derivative (260 mg, 34%). NMR (CDCl₃, 90 MHz) δ 2.2, s, 3H; 7.00, m, 2H, 6.30-6.80, m, 7H; 8.20, d, 9 Hz, 1H; 9.45, s, 1H. The tosyloxy compound (600 mg, 1.5 mmole) was dissolved in acetonitrile (4 ml) and 1,7-diaza-4,10,13-trioxacyclopentadecane (110 mg, 0.5 mmole) were added, followed by 1,8-bis(dimethylamino)naphthalene (290 mg, 2 mmole) and the reaction mixture heated under reflux overnight. The reaction mixture was evaporated *in vacuo* and the residue taken into chloroform and purified by column chromatography (silica gel, ethyl acetate, hexane 1:1) to give the N,N-bis-(1-nitro-4-acridinyl)-diaza[15]-crown-5 as a yellow gum (180 mg, 27%). NMR (CDCl₃, 90 MHz) δ 3.40, s, 4H; 3.82, m, 16H; 6.50, d, 9 Hz, 2H; 6.95, d, 9 Hz, 2H; 7.30, t, 9 Hz, 4H; 7.65, d, 9 Hz, 2H; 8.20, d, 9 Hz, 2H; 9.68, s, 2H.

The nitro-crown compound (70 mg, 0.1 mmole) was added to stannous chloride (200 mg in 2 ml ethanol), concentrated hydrochloric acid (3 ml), and ethanol (3 ml) and stirred overnight at room temperature. The reaction mixture was neutralized with sodium bicarbonate and extracted into chloroform. The chloroform layer was treated with acetic anhydride to give **2HH**, which was purified by column chromatography (silica gel, chloroform: methanol 85:15) to a soft yellow solid (30 mg, 41.6%). NMR (CDCl₃, 90 MHz) δ 2.45, s, 6H; 3.55, m, 20H; 6.30, t, 4H; 6.80, t, 2H; 7.30, d, 9 Hz, 2H; 7.50, d, 9 Hz, 2H; 7.82, d, 9 Hz, 2H; 9.15, s, 2H.

The succinamide derivative (**2II**) was obtained by reduction of the nitro compound (45 mg, 0.064 mmole) with stannous chloride (150 mg) and conc. HCl (3 ml) in ethanol. The resulting amine hydrochloride was washed repeatedly with ethanol, dissolved in pyridine (1.5 ml), and treated with succinic anhydride with stirring. The reaction was complete in three hours and the product (**2II**) was purified by column chromatography (silica gel, 10% methanol in chloroform) (15 mg, reddish yellow soft solid, 28%). Mass spec. (FAB) m/e = 802 (M⁺).

### N,N-bis-(4-acetamido-2-methoxyphenyl)-diaza[15]-crown-5 (2JJ)

2-Fluoro-5-nitroanisole (26) (250 mg, 1.5 mmole) was heated under reflux with 1,7-diaza-4,10,13-trioxacyclopentadecane (108 mg, 0.5 mmole) in pyridine (5 ml) overnight. The reaction mixture was evaporated *in vacuo* and the residue purified by preparative thin layer chromatography (silica gel) to obtain N,N-bis-(4-nitro-2-methoxyphenyl)-diaza[15]-crown-5 as a light yellow oil (120 mg, 23%). NMR (CDCl₃, 90 MHz) δ 7.80, dd, 8 Hz, 3 Hz, 2H; 7.70, d, 3 Hz, 2H; 6.89, d, 8 Hz, 2H; 3.87, s, 6H; 3.68, s + m, 20H. The nitro compound (0.05 g) was dissolved in acetic anhydride and hydrogenated at room temperature and atmospheric pressure with 0.02 g palladium (5% on charcoal) catalyst. After full hydrogen uptake the mixture was filtered and the solvent evaporated *in vacuo* to give **2JJ** as a whitish semi-solid (43 mg, 82%). Mass spec.: m/e = 544 (M⁺); m/e = 513 (M⁺ - OMe).

### N,N-bis-(3,6-dioxocyclohexa-1,4-dienyl)-diaza[15]-crown-5 (2KK)

p-Benzoquinone (2.0 g, 18.5 mmole) and 1,7-diaza-4,10,13-trioxacyclopentadecane (400 mg, 1.8 mmole) were dissolved in a 1:1 mixture of chloroform and methanol (13 ml) and heated under reflux overnight. The reaction mixture was evaporated off *in vacuo* and the residue dissolved in a large volume of chloroform and filtered through a column of silica gel packed in ethyl acetate. The excess benzoquinone was removed with more ethyl acetate and the product (**2KK**) was obtained by eluting with 4% methanol in ethyl acetate. Evaporation of the solvent gave **2KK** as a deep red foam (600 mg, 76%). NMR (CDCl₃, 90 MHz) δ 3.75, s, 4H; 3.88, s, 16H; 5.68, d, 1.5 Hz, 2H; 6.48, dd, 1.5 Hz, 4H.

### N,N-bis-(2,5-dimethoxyphenyl)-diaza[15]-crown-5 (2LL)

The bis-quinone (**2KK**) (210 mg, 0.49 mmole) was dissolved in 2 ml methanol and hydrogenated with 33 mg of palladium (5% on charcoal) catalyst at atmospheric pressure and room temperature. When H₂ uptake ceased after 1.5 hrs, the solution had changed to a dull yellowish brown color. The reaction flask was stirred under a slight positive pressure of H₂ while approximately 1 mmole of tetramethylammonium hydroxide pentahydrate was injected as a 4 M solution in methanol through a gas-tight rubber septum into the mixture. Then 1 mmole of neat dimethyl sulfate was similarly injected using a separate syringe. The alternate injection of tetramethylammonium hydroxide then dimethyl sulfate was repeated nine more times over the next 2.5 hrs, for a total of 10 mmoles each of base and alkylating reagent. This cyclical procedure of partial deprotonation and methylation was found to give better results than adding all the base and all the alkylating reagent simultaneously, in which case the latter two mainly destroy each other. Also by conducting the alkylation under H₂, the strong tendency of the phenoxide anion to re-oxidize was suppressed. Once the alkylation was complete, the product was reasonably stable to air and could be worked up by evaporating the methanol *in vacuo*, dissolving the residue in water, neutralizing the alkalinity with acetic acid, and extracting with chloroform. Evaporation of the chloroform gave 252 mg crude **2LL** (105% of the stoichiometrically expected weight), which could be purified by centrifugal chromatography. NMR (CF₃COOH, 90 MHz) δ 3.95, m + s, 32H; 7.15, m, 6H.

### N,N-bis-(2-methoxy-5-hydroxy-4-formylphenyl)-diaza[15]-crown-5 (2MM)

The dimethoxy compound (**2LL**) (30 mg, 0.06 mmole) was dissolved in dimethylformamide (200 µl) and kept at 0°. 0.5 ml of a 1:4 (v/v) mixture of POCl₃ and dimethylformamide was added and the reaction mixture stirred for 1 hour. Water (2 ml) was added to quench the reaction mixture followed by addition of saturated potassium carbonate to basify the solution. The reaction mixture was then extracted 3 times with chloroform. The combined chloroform extracts were back-washed with water and evaporated *in vacuo* to give the dimethoxyaldehyde as a yellow gum (25 mg, 75%). NMR (CDCl₃, 90 MHz) δ 3.80, m, 20H; 3.98, s, 6H; 4.20, s, 6H; 6.70, s, 2H; 7.48, s, 2H; 10.68, s, 2H.

The dimethoxyaldehyde (15 mg, 0.027 mmole) was dissolved in nitromethane (2 ml). Saturated zinc chloride in nitromethane solution (1 ml) was added, followed by 2 ml of 1.0 M BCl₃ solution in dichloromethane. The reaction mixture was stirred for 1.5 hours and a 1:1 mixture of water and methanol (2 ml) was added. Stirring was continued for 30 min and potassium carbonate-EDTA solution was added. Stirring was continued for 30 more minutes and the mixture was extracted with chloroform (3 times), followed by ethyl acetate (1 time). The combined organic extracts were washed with water and purified by centrifugal chromatography on ethyl acetate to give **2MM** as a yellow soft solid (11 mg, 78%). NMR (CDCl₃, 90 MHz) δ 3.75, m, 20H; 3.85, s, 6H; 6.50, s, 2H; 6.88, s, 2H; 9.84, s, 2H (aldehyde); 11.50, s, 1H, OH hydrogen bonded.

### N,N-bis-(2-(3,4-dicarboxyphenyl)-5-methoxybenzofuran-6-yl)-diaza[15]-crown-5 (2NN Me ester) (= SBFP/Me)

The salicylaldehyde (**2MM**) (9 mg, 0.017 mmole), potassium carbonate (40 mg), dimethyl 4-bromomethylphthalate (11 mg, 0.04 mmole), and dimethylformamide (0.5 ml) were heated together at 140° (bath temp.) for 4 hours. The mixture was allowed to cool. Chloroform with 10% methanol (3 ml) was added. The entire mixture was washed with water and evaporated *in vacuo*. The residue was dissolved in 5% MeOH in chloroform and purified by centrifugal chromatography with chloroform-methanol (24:1 v/v) to give **SBFP/Me** as a light brown gum (5 mg, 33%). NMR (CD₃OD, CDCl₃ 1:9, 200 MHz) δ 3.70, m, 20H; 3.97, 2s, 6H; 3.99, 2s, 6H; 4.10, 2s, 6H; 7.04, m, 2H; 7.10, s, 2H; 7.85, d, 2H; 7.95, d, 2H; 8.13, s, 2H.

### N,N-bis-(2-(5-carboxyoxazol-2-yl)-5-methoxybenzofuran-6-yl)-diaza[15]-crown-5 (2OO ethyl ester) (= SBFO/Et)

The salicylaldehyde (**2MM**) (6 mg, 0.012 mmole), potassium carbonate (40 mg), ethyl 2-chloromethyloxazole-5-carboxylate (13) (12 mg, 0.068 mmole), and dimethylformamide (300 µl) were heated together at 100° for 1 hour. The reaction mixture was allowed to cool and chloroform (3 ml) was added and the entire mixture washed with water to get rid of the solid potassium carbonate. The organic layer was evaporated *in vacuo* and taken into 5% methanol-chloroform for purification by centrifugal chromatography using chloroform-methanol (24:1 v/v). The product, **SBFO/Et**, was obtained as a yellow gum (5 mg, 57%). NMR (CD₃OD, CDCl₃ 1:9, 200 MHz) δ1.21, t, 6H; 3.60-3.80, m, 16H; 3.98, s, 4H; 4.45, q, 4H; 4.80, d, 2H; 7.18, s, 2H; 7.55, s, 2H; 7.75, s, 2H; 7.92, s, 2H.

### N,N-bis-(2-(2,4-dicarboxyphenyl)-5-methoxybenzofuran-6-yl)-diaza[15]-crown-5 (2PP methyl ester) (= SBFI/Me)

Dimethyl 4-bromomethylisophthalate was obtained by the method of Anzalone & Hirsch (24), using methanol instead of ethanol in the esterification procedure. M.p. 80-82°. NMR (CDCl₃, 90 MHz) δ 3.90, s, 3H; 3.92, s, 3H; 4.90, s, 2H; 7.45, d, 7 Hz, 1H; 8.05, dd, 2 Hz, 7 Hz, 1H; 8.50, d, 2 Hz, 1H.

The above isophthalate ester (150 mg, 0.52 mmole), the salicylaldehyde 2MM (40 mg, 77 mmole), K₂CO₃ (250 mg, 1.8 mmole), and dimethylformamide (2 ml) were heated together at 150° for 2.5 hrs. The mixture was diluted with chloroform and filtered. The filtrate was washed with water and evaporated *in vacuo*. The gummy residue was purified by centrifugal chromatography to give **SBFI/Me** (28 mg, 41%). NMR (CDCl₃, 200 MHz) δ 3.60-3.80, m, 20H; 3.90, s, 12H; 3.95, s, 6H; 7.05, s, 2H; 7.20, s, 2H; 7.30, s, 2H; 7.80, d, 2 Hz, 2H; 8.20, dd, 7 Hz, 2 Hz, 2H; 8.35, d, 2 Hz, 2H.

### N,N-bis(2-(3,4-dicarboxyphenyl)-5-methoxybenzofuran-6-yl)-diaza[18]-crown-5 (3NN Me ester) (=PBFP/Me)

The preparation of **PBFP/Me** was similar to that of **SBFP/Me** (**2NN**) except that 1,10-diaza-4,7,13,16-tetraoxacyclooctadecane (Kryptofix 22, EM Sciences) was used as the crown instead of 1,7-diaza-4,10,13-trioxacyclopentadecane. The properties of the various intermediates (including **3K**, **3L**, and **3M**) and **PBFP** are as follows:

N,N-bis-(3,6-dioxocyclohexa-1,4-dienyl)-diaza[18]-crown-6 (**3K**) was obtained in 70% yield as reddish-brown needles. M.p. 153-155°. NMR (CDCl₃, 90 MHz) δ 3.60-3.80, s + m, 24H; 5.60, d, 1.5 Hz, 2H; 6.48, dd, 1.5 Hz, 4H.

N,N-bis-(2,5-dimethoxyphenyl)-diaza[18]-crown-6 (**3L**) was obtained in 55% yield as a gum. NMR (CF₃COOH, 90 MHz) δ 3.40-3.80, s + m, 36H; 6.80, m, 6H.

N,N-bis-(1,4-dimethoxy-5-formyl-2-phenyl)-diaza[18]-crown-6 was obtained as an off-white soft solid. M.p. 131-133°. NMR (CDCl₃, 90 MHz) δ 3.50-3.70, s + m, 24H; 3.75, s, 6H; 3.85, s, 6H; 6.50, s, 2H; 7.20, s, 2H; 10.25, s, 2H (formyl).

N,N-bis-(1-methoxy-4-hydroxy-5-formyl-2-phenyl)-diaza[18]-crown-6 (**3M**) was obtained as an orange solid. M.p. 134-136°. NMR (CDCl₃, 90 MHz) δ 3.50-3.70, s + m, 24H; 3.75, s, 6H; 6.40, s, 2H; 6.90, s, 2H; 9.65, s, 2H (formyl); 11.40, s, 2H (hydrogen-bonded OH).

**PBFP/Me** (**3NN methyl ester**) was obtained as a light yellow gum. NMR (CDCl₃, 200 MHz) δ 3.60, m, 8H; 3.67, m, 16H; 3.90, s, 12H; 3.93, s, 6H; 6.90, s, 2H; 7.10, s, 2H; 7.48, s, 2H; 7.68, d, 2 Hz, 2H; 7.80, dd, 2 Hz, 1 Hz, 2H; 7.98, d, 1 Hz, 2H.

### Saponification of methyl or ethyl esters; preparation of acetoxymethyl ester

The esters of polycarboxylic acids **2NN**, **2OO**, **2PP**, and **3NN** were hydrolyzed by dissolving them in methanol or dioxane or a mixture of the two solvents, then adding excess base, usually tetramethylammonium hydroxide (TMA⁺OH⁻) or cesium hydroxide so that the cation would show negligible tendency to bind to the chelator. Acetoxymethyl (AM) esters were prepared by the standard procedure of realkylation of the polycarboxylate anions using acetoxymethyl bromide (13). A typical procedure is given below for **2PP** (=**SBFI**) and its AM ester:

**SBFI/Me** (6 mg, 6.7 µmole) was dissolved in 200 µl methanol and 200 µl dioxane. 1M TMA⁺OH⁻ (200 µl) was added and the reaction left overnight. When hydrolysis was complete as judged by reverse-phase thin layer chromatography, the mixture was evaporated to dryness. The residue was dissolved in dimethyl-formamide (2 ml) and ethyldiisopropylamine (200 µl) and acetoxymethyl bromide (300 µl) were added. The suspension was stirred overnight. Chloroform was added and the alkylammonium bromide salts filtered off. The filtrate was evaporated *in vacuo* and the residue purified by centrifugal chromatography (silica) to give the product as a hard gum (4 mg, 53%). NMR (CDCl₃, 200 MHz) δ 2.10, s, 6H; 2.18, s, 6H; 3.50-3.80, m, 20H; 3.88, s, 6H; 5.95, s, 4H; 6.05, s, 4H; 6.98, s, 2H; 7.05, s, 2H; 7.20, s, 2H; 7.87, d, 7 Hz, 2H; 8.20, dd, 7 Hz, 2 Hz, 2H; 8.35, d, 2 Hz, 2H.

### REFERENCES

The present specification refers to the following publications, each of which is expressly incorporated by reference herein.
1. Skou, J.C., Norby, J.G., Maunsback, A.B., and Esmann, M., eds., (1988) *The Na⁺, K⁺-Pump, Part B: Cellular Aspects* in Progr. Clin. Biol. Res., Vol. 268, A.R. Liss, New York.
2. Guernsey, D.L., and Edelman, I.S., (1983) in Molecular Basis of Thyroid Hormone Action (J.H. Oppenheimer and H.H. Samuels, eds.), pp. 293-324, Academic Press, New York.
3. Dix, J.-P. and Vögtle, F., (1980) *Chem. Berichte* **113**457-470.
4. Löhr, H.G., and Vögtle, F., (1985) *Acc. Chem. Res.* **18**, 65-72.
5. Lehn, J.M., and Sauvage, J.P., (1975) *J. Am. Chem. Soc.* **97**, 6700-6707.
6. Smith, G.A., Morris, P.G., Hesketh, T.R., and Metcalfe, J.C., (1986) *Biochem. Biophys. Acta* **889**, 72-83.
7. Smith, G.Z., Hesketh, T.R., and Metcalfe, J.C., (1988) *Biochem. J.* **250**, 227-232.
8. Cram, D.J., (1983) *Science* **219**, 1177-1183.
9. Cram, D.J., Carmack, R.A., and Helgeson, R.C., (1988) *J. Am. Chem. Soc.* **110**, 571-577.
10. Harootunian, A.T., Kawanishi, T., Kao, J.P.Y., Eckert, B.K., and Tsien, R.Y., manuscript in preparation.
11. Moore, E.D.W., Tsien, R.Y., Minta, A., and Fay, F.S., (1988) *FASEB J.* **2**, A754.
12. Negulescu, P.A., Harootunian, A.T., Minta, A., Tsien, R.Y., and Machen, T.E., (1988) *J. Gen. Physiol.* **92**, 26a.
13. Grynkiewicz, G., Poenie, M., and Tsien, R.Y., (1985) *J. Biol. Chem.* **260**, 3440-3450.
14. Tsien, R.Y., Pozzan, T., and Rink, T.J., (1982) *J. Cell Biol.* **94**, 325-334.
15. Rossotti, H., (1989) The Study of Ionic Equilibria. Longman, London.
16. Tsien, R.Y., (1980) *Biochemistry* **19**, 2396-2404.
17. Vögtle, F., and Weber, E., (1980) in The Chemistry of Ethers, Crown Ethers, Hydroxyl Groups and Their Sulplhur Analogues, Supplement E, part 1, (S. Patai, ed.), pp. 59-156, John Wiley, New York.
18. Tsien, R.Y., (1983) *Annu. Rev. Biophys. Bioeng.* **12**, 91-116.
19. Wun, T.-C., Bittman, R., and Borowitz, I.J., (1977) *Biochemistry* **16**, 2074-2079.
20. Tramer, A., (1969) *J. Mol. Struct.* **4**, 313-325.
21. Wan, Y.P., Porter, T.H., and Folkers, K., (1974) *J. Heterocyclic Chem.* **11**, 519-524.
22. Dietrich, B., Lehn, J.M., Sauvage, J.P., and Blanzat, J., (1973) *Tetrahedron* **29**, 1629-1645.
23. Yoon, N.M., and Brown, H.C., (1968) *J. Am. Chem. Soc.* **90**, 2927-2938.
24. Anzalone, L., and Hirsch, J.A., (1985) *J. Org. Chem.* **50**, 2128-2133.
25. Irving, H., Butler, E.J., and Ring, M.F., (1949) *J. Chem. Soc.* 1949, 1489-1498.
26. Ruyle, W.V., Sarett, L.H., and Matzuk, A.R., (1977) U.S. Patent No. 4,044,049.
27. Hikosaka, A., (1970) *Bull. Chem. Soc. Japan* **43**, 3928-3929.
28. Ulrich, H., and Richter, R., (1977) in Methoden der Organischen Chemie (Houben-Weyl), Vol. VII/3a, pp. 404-412, Georg Thieme, Stuttgart.
29. Dean, F.M., Goodchild, J., Houghton, L.E., Martin, J.A., Morton, R.B., Parton, B., Price, A.W., and Somvichien, N., (1966) *Tetrahedron Lett.* 4153-4159.
30. Somlyo, A.P., ed. (1986) Recent Advances in Electron and Light Optical Imaging in Biology and Medicine (*Ann. N.Y. Acad. Sci.,* Vol. 483), New York Academy of Sciences, N.Y.
31. Horowitz, S.B., and Paine, P.L., (1979) *Biophys. J.* **25**, 45-62.
32. Slack, C., Warner, A.E., and Warren, R.L., (1973) *J. Physiol. (London)* **232**, 297-312.
33. Springer, C.S., Jr., (1987) *Ann. N.Y. Acad. Sci.* **508**, 130-148.
34. Liebling, M.S., and Gupta, R.K., (1987) *Ann. N.Y. Acad. Sci.* **508**, 149-163.
35. Tsien, R.Y., (1986) in *Optical Methods in Cell Physiology* (P. de Weer and B.M. Salzberg, eds.) pp. 327-345, Wiley Interscience, N.Y.
36. Gramain, P., and Frère, Y., (1979) *Nouv. J. Chimie* **3**, 53-58.
37. Nakatsuji, Y., Nakamura, T., Yonetani, M., Yuya, H., and Okahara, M., (1988) *J. Am. Chem. Soc.* **110**, 531-538.
38. Takagi, M., and Ueno, K., (1984) *Topics Curr. Chem.* **121**, 39-65.
39. Shiga, M., Nishida, H., Nakamura, H., Takagi, M., and Ueno, K., (1983) *Bunseki Kagaku* **32**, E293-E300.
40. Tazaki, M., Nita, K., Takagi, M., and Ueno, K., (1982) *Chem. Lett.* 571-574.
41. Chang, C.A., and Ochaya, V.O. (1986) *Inorg. Chem.* **25**, 355-358.
42. Gandour, R.D., Fronczek, F.R., Gatto, V.J., Minganti, C., Schultz, R.A., White, B.D., Arnold, K.A., Mazzochi, D., Miller, S.R., and Gokel, G.W., (1986) *J. Am. Chem. Soc.* **108**, 4078-4088.
43. Gatto, V.J., Arnold, K.A., Viscariello, A.M., Miller, S.R., Morgan, C.R., and Gokel, G.W., (1986) *J. Org. Chem.* **51**, 5373-5384.
44. Thomas, R.C., and Cohen, C.J., (1981) *Pfügers Arch.* **390**, 96-98.
45. Lein, G.M., and Cram, D.J., (1982) *J. Chem. Soc., Chem. Commun.* 301-304.
46. Maeda, H., Furuyoshi, S., Nakatsuji, Y., and Okahara, M., (1983) *Bull. Chem. Soc. Jpn.* **56**, 212-218.
47. Perez-Cistue, J.I.C., (1956) *Rev. acad. cienc. exact. fis.-quim. y nat. Zaragosa* **11**, 33-87. *[Chem. Abst.* (1958) **52**, 13544-13545.]
48. Weber, E. and Vögtle, F., (1976) *Chem. Ber.* **109**, 1803-1831.
49. Laidler, D.Z., and Stoddart, J.F., (1980) in The Chemistry of Ethers, Crown Ethers, Hydroxyl Groups and Their Sulphur Analogues, Supplement E, Part 1, (S. Patai, ed.), pp. 1-58, John Wiley, New York.
50. Nakatsuji, Y., Nakamura, T., Okahara, M., Dishong, D.M., and Gokel, G.W., (1983) *J. Org. Chem.* **48**, 1237-1242.
51. Tsien, R.Y. (1981) *Nature* **290**, 527-528.
52. Minta, A.M. and Tsien, R.Y., (1989) *J. Biol. Chem.* In Press.

### SPECIFICATION SUMMARY

From the foregoing description, one of ordinary skill in the art can understand that the present invention provides new macrocyclic fluorescent chelating compounds for alkali metal cations. The new chelating compounds are comprised of: (1) crown ethers (that may or may not have substituent groups attached to the core carbons, but will always contain at least one core nitrogen) that are linked via the core nitrogen(s) to at least one (2) fluorophore that contains heteroaromatic ligands. The new fluorescent indicator compounds are used to nondestructively observe intracellular alkali metal cations such as cytosolic concentrations of free Na⁺, K⁺, and Li⁺. Tests in lymphocytes, hepatocytes, fibroblasts, smooth muscle cells, and gastric glands demonstrate the biological utility of the macrocyclic compounds of the present invention for nondestructive observation of [Na⁺]ᵢ in individual cells viewed by fluorescence microscopy.

Without departing from the spirit and scope of this invention, one or ordinary skill can make various changes and modifications to the invention to adapt it to various usages and conditions. As such, these changes and modifications are properly, equitable, and intended to be, within the full range of equivalence of the following claims.

## Claims (Claims for the following Contracting State(s): AT, BE, FR, DE, IT, GR, LU, NL, SE, CH, GB)

1. An aza-crown ether of the formula: 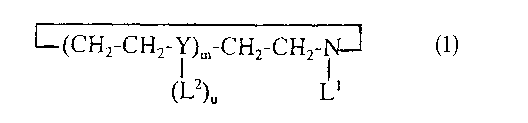 wherein:
m is an integer from 3 to 5,
each Y is independently N or O,
u is 1 if Y is N and 0 if Y is O
L¹ is a ligand of formula (i) or formula (ii) below 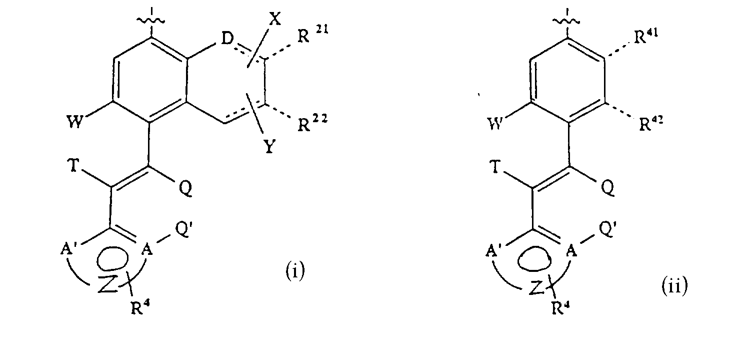 wherein:
A and A¹ are independently C, N, O or S;
D is N or O;
Q is H or NR¹R where R¹ and R are independently -H, C₁-C₄ alkyl, -CH₂COOH, -CH₂CH₂OH, or phenyl or R¹ and R together are -(CH₂)₄-, -(CH₂)₅-, or -CH₂CH₂OCH₂CH₂-;
Q¹ is H unless A is NR³, O or S; or
Q and Q¹ together are NR³, and A is C;
R³ is H, -CH₃, -C₂H₅ or CH₂COOH;
R⁴ is (E)ₙ where n = 0-3, and E is a polar electron-withdrawing function selected from -CO₂H, -CO₂R¹, -CONR¹R, -SO₃H, -SO₂NR¹R, -SO₂CF₃, -COCH₃, and -CN where R¹ and R are independently -H, C₁-C₄ alkyl, -CH₂COOH, -CH₂CH₂OH, or phenyl or R¹ and R together are -(CH₂)₄-, -(CH₂)₅-, or -CH₂CH₂OCH₂CH₂-;
R¹ is -H, -CH₃, -C₂H₅, other lower alkyl up to C₄, -COOH, C₁-C₄ alkoxy, or -OC(O)CH₃; or
R¹ and R together are -CH=CH-CH=CH-, both X and Y are double bonds and D is N so that D, R¹, and R form an acridine ring system;
R⁴¹ is -OH, -OCH₂COOH, -OCH₂CH₂OH, methoxy or C₂-C₄ alkoxy, -NR¹⁴R¹⁵, -COOH, -C(O)NR¹⁴R¹⁵, or -OC(O)CH₃ where R¹⁴ and R¹⁵ are independently -H, C₁-C₄ alkyl, -CH₂COOH, -CH₂CH₂OH, or phenyl;
R⁴ is -H, -CH₃ or -COOH;
W and T are each H or together are O or NR³;
X is a double bond when D is N, and a single bond when D is O;
Y may be either a double or a single bond;
and
Z is a moiety forming a (hetero) aromatic ring;
and L is H;
a ligand of formula (i) or formula (ii);
-CH₂C(O)NR¹R where R¹ and R are independently -H, C₁-C₄ alkyls -CH₂COOH, -CH₂CH₂OH, or phenyl, or R¹ and R together are -(CH₂)₄-, -(CH₂)₅-, or -CH₂CH₂OCH₂CH₂- (to form a pyrrolidine, piperidine, or morpholine ring, respectively);
-CH₂COOH;
-2-pyridylmethyl;
-2-tetrahydrofuranylmethyl;
-CH₂CH₂OR^{a} where R^{a} is -H, C₁-C₄ alkyl, -CH₂COOH, -CH₂CH₁OH, or phenyl; or
-2,5-dialkoxyphenyl where the alkoxy substituent is C₁-C₄ alkoxy.

2. A compound of claim 1, wherein 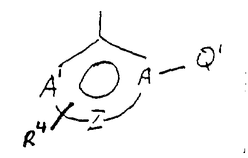 is 2,4-dicarboxyphenyl 3,4-(dicarboxyphenyl, or 5-carboxyoxazol-2-yl.

3. An aza-crown ether of the formula: 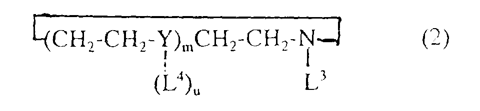 wherein:
m is an integer from 3 to 5;
each Y is independently N or O
u is I if Y is N and 0 if Y is O
L³ is quinone or a ligand of formula (iii) or formula (iv) below 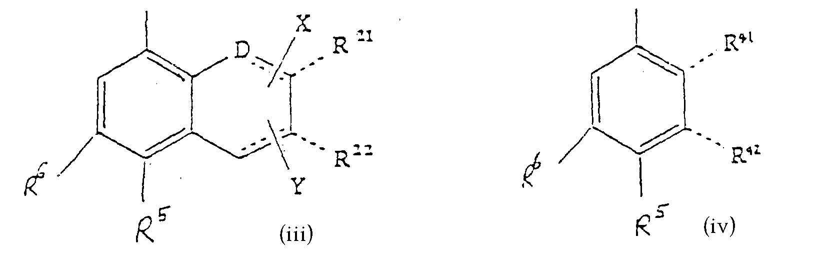
wherein:
D, R¹, R, R⁴¹, R⁴, X and Y are as defined in claim 1 ;
R⁵ is H,-NH₂, -CHO or -NHCOCH₃, or -NHCOCH₂CH₂COO⁻ or other acylated amino group; and
R⁶ is H,-OH, or -OCH₃ or other alkoxy, provided that one of R⁵ and R⁶ is not H;
and L⁴ is H;
quinone;
a ligand of formula (iii) or formula (iv);
-CH₂C(O)NR¹R where R¹ and R are independently -H, C₁-C₄ alkyl, -CH₂COOH, -CH₂CH₂OH, or phenyl, or R¹ and R together are -(CH₂)₄-, -(CH₂)₅-,or -CH₂CH₂OCH₂CH₂- (to form a pyrrolidine, piperidine, or morpholine ring, respectively);
-CH₂COOH;
-2-pyridylmethyl;
-2-tetrahydrofuranylmethyl;
-CH₂CH₂OR^{a}where R^{a} is -H, C₁-C₄ alkyl, -CH₂COOH, -CH₂CH₂OH, or phenyl; or
-2,5-dialkoxyphenyl where the alkoxy substituent is C₁-C₄ alkoxy.

4. A compound of claim 3, wherein R⁵ is H, -NH₂, -CHO, -NHCOCH₃, -NHCOCH₂CH₂COO⁻ or other amido group.

5. A compound of claim 4, wherein
R⁵ is H, -NH₂, -CH₃, -NHCOCH₃ or -NHCOCH₂CH₂COO⁻; and
R⁶ is H, -OH or -OCH₃.

6. A compound of any one of claims 3 to 5 wherein, in any L³ or L⁴ group of formula (iii), R⁵ is not -NH₂.

7. A compound of any one of claims 1 to 6, wherein at least one of the hydrogens on the aza-crown ether core carbons is substituted with a substituent selected from: -COOH, -CH₂OH, -C(O)N(CH₃)₂, or C₁-C₄ alkyl; or hydrogens on adjacent core carbons of the aza-crown ether are substituted with R³¹ and R³ wherein R³¹ and R³ together are -(CH₂)₃- or -(CH₂)₄- (wherein R³¹ and R³ are part of an aliphatic system), or R³¹ and R³ together are -(CH)₃- (wherein R³¹ and R³ are part of an aromatic system); or hydrogens on non-adjacent core carbons of the aza-crown ether are substituted with R³¹ and R³ wherein R³¹ and R³ together are -(CH₂)₂- or -(CH₂)₃- (wherein R³¹ and R³ are part of an aliphatic system), or R³¹ and R³ together are -(CH)₃- (wherein R³¹ and R³ are part of an aromatic system), and wherein optionally each L¹ and L group is of formula G, N, O or P of Figure 1 or, as the case may be, each L³ and L⁴ group is of formula C, D, E, F, H, I, J, K, L or M of Figure 1.

8. A compound of claim 7 wherein the aza-crown ether core is of formula (B) or (D) below: 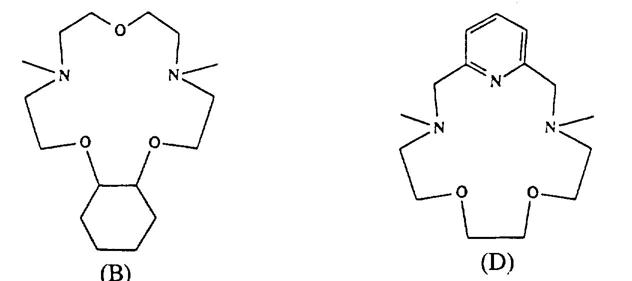 the sp³-hybridised N atom being substituted as defined in any one of claims 1 to 6.

9. A compound of any one of claims 1 to 7, wherein the aza-crown ether is a monoaza-, diaza-, triaza-, tretraaza-, pentaaza- or hexaaza- crown ether.

10. A compound of claim 9, wherein the aza-crown ether is monoaza-12-crown-4, diaza-12-crown-4, monoaza-15-crown-5, diaza-15-crown-5, monoaza-18-crown-6 or a diaza-18-crown-6 ether.

11. A compound of claim 10, wherein the aza-crown ether is 1,7-diaza-4,10,13-trioxacyclopentadecane or 1, 10-diaza-4, 7, 13, 16-tetraoxacyclo octadecane.

12. An aza-crown ether of claim 1 or claim 2 or of any of claims 7 to 11 when dependent on claim 1 or claim 2 wherein one or more of the L groups is replaced by an L⁴ group of any of claims 3 to 6.

13. A compound comprised of the combination of (1) an aza-crown ether and (2) at least one ligand(s), wherein the ligand(s) is/are attached to the aza-crown ether via the sp³-hybridized core nitrogen(s), and wherein (1) the crown ether is selected from the group consisting of monoaza-12-crown-4, diaza-12-crown-4, monoaza-15-crown-5, diaza-15-crown-5, mono aza-18-crown-6 and diaza-18-crown-6, and (2) the ligand(s) is/are selected from the groups whose formulas are shown in Figure 1 as group B, C, D, E, F, G, H, I, J, K, L, M, N, O, and P.

14. A compound of any one of claims 1 to 9, 11 or 12 wherein the aza-crown ether is a symmetrical diaza-crown ether or an asymmetrical diaza-crown ether, as hereinbefore defined.

15. A compound of any of claims 1 to 14 wherein either:
(a) the aza-crown ether has at least two core nitrogens having ligands attached, which ligands are identical ligands; or
(b) the aza crown ether has at least two core nitrogens having ligands attached, which ligands are not identical ligands.

16. A compound selected from:-
(a) compound SBFP, whose structure is shown in Figure 7(a) as 2NN;
(b) compound SBFO, whose structure is shown in Figure 7(a) as 2OO;
(c) compound SBFI, whose structure is shown in Figure 7(a) as 2PP;
(d) compound PBFP, whose structure is shown in Figure 7(a) as 3NN;
(e) compound PBFO, whose structure is shown in Figure 7(b) as 3OO; or
(f) compound PBFI, whose structure is shown in Figure 7(b) as 3PP.

17. A compound of any one of claims 1 to 16 wherein the compound is in the form of an ester wherein any carboxylates are esterified to form physiologically hydrolyzable esters, preferably acetoxymethyl esters, or the compound is in the form of a pharmaceutically or veterinarily acceptable salt or ester thereof.

18. An aza-crown ether of the formula 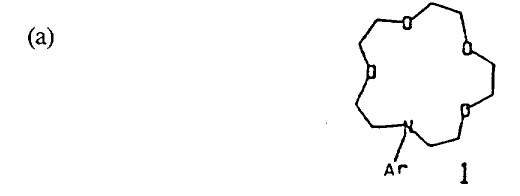 wherein Ar is a group whose formula is shown in Figure 1 as group C, D, E, F, G, H, I, J, K, L, M, N, O or P; 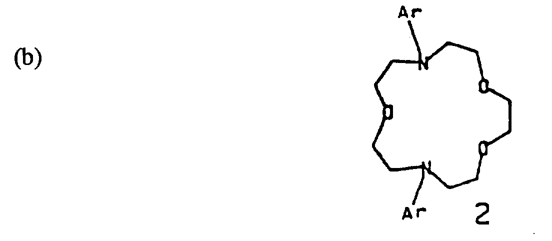 wherein the Ar groups are
(i) identical, or
(ii) not identical
and in either case (i) or (ii) are each a group whose formula is shown in Figure 1 as group C, D, E, F, G, H, I, J, K, L, M, N, O or P; or 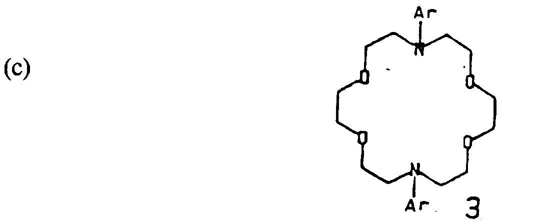 wherein the Ar groups are
(i) identical, or
(ii) not identical
and in either case (i) or (ii) are each a group whose formula is shown in Figure 1 as group C, D, E, F, G, H, I, J, K, L, M, N, O or P.

19. A compound of claim 18 wherein the aza-crown ether core is substituted as defined in claim 7 or claim 8 and/or the compound is in the form of an ester wherein any carboxylates are esterified to form a physiologically hydrolysable ester or is in the form of a pharmaceutically or veterinarily acceptable salt or ester thereof.

20. A process for preparing a macrocyclic fluorescent alkali metal chelating agent, comprising forming a combination of an aza-crown ether and one or more ligands attached to the crown ether via one or more of the sp³-hybridised core nitrogen(s), wherein the crown ether is of the formula 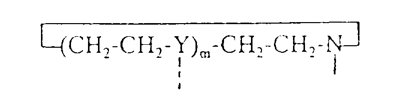 wherein m is an integer from 3 to 5 and each Y is independently O or N and at least one of the ligand(s) is of formula (i) or (ii) of claim 1 or of formula (iii) or (iv) of claim 3, the combination optionally being further defined by the specific feature(s) of any one or more of claims 2 or 3 to 11 or being as defined in any one of claims 12 to 18.

21. A process for preparing a compound as defined in claim 1, claim 3 or claim 12 comprising either:
(a) effecting a method chemically equivalent to a method comprising that described hereinbefore under any one of the headings "N,N-bis-(2-(3,4-dicarboxyphenyl)-5-methoxybenzofuran-6-yl)-diaza[15]-crown-5 (2NN Me ester) (=SBFP/Me)", "N,N-bis-(2-(5-carboxyoxazol-2-yl)-5-methoxybenzofuran-6-yl)-diaza[15]-crown-5 (2OO ethyl ester) (=SBFO/Et)" or "N,N-bis-(2-(2,4-dicarboxyphenyl)-5-methoxybenzofuran-6-yl)-diaza[15]-crown-5(2PPmethyl ester) (=SBFI/Me)", optionally preceded by one or more of the method steps in sequence leading up to compound 2MM as set out hereinbefore under "COMPOUND SYNTHESIS"; or
(b) effecting a process analogous to (a) but employing as the crown ether 1,7-diaza-4, 10-dioxacyclododecane or 1,10-diaza-4,7,13,16-tetraoxacyclooctadecane.

22. The use of a compound as defined in any one of claims 1 to 18 in the preparation of an alkali metal chelating formulation for non-destructive observation of an alkali metal ion concentrate in tissue.

23. A method of non-destructive observation of an alkali metal ion concentrating in tissue comprising employing in said tissue, as an alkali metal chelating agent, a compound as defined in any one of claims 1 to 18 and observing or measuring fluorescence.

## Claims (Claims for the following Contracting State(s): ES)

1. A method of non-destructive observation of an alkali metal ion concentration in tissue, comprising employing in said tissue an alkali metal chelating agent and observing or measuring fluorescence, wherein the chelating agent is an aza-crown ether of the formula: 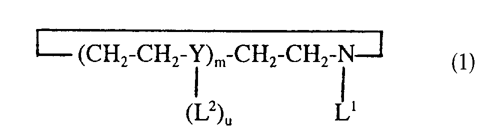 wherein:
m is an integer from 3 to 5,
each Y is independently N or O,
u is 1 if Y is N and 0 if Y is O
L¹ is a ligand of formula (i) or formula (ii) below 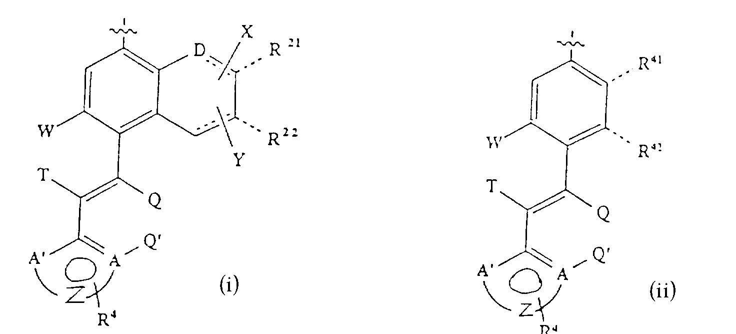 wherein:
A and A¹ are independently C, N, O or S;
D is N or O;
Q is H or NR¹R where R¹ and R are independently -H, C₁-C₄ alkyl, -CH₂COOH, -CH₂CH₂OH, or phenyl or R¹ and R together are -(CH₂)₄-, -(CH₂)₅-, or -CH₂CH₂OCH₂CH₂-;
Q¹ is H unless A is NR³, O or S; or
Q and Q¹ together are NR³, and A is C;
R³ is H, -CH₃, -C₂H₅ or CH₂COOH;
R⁴ is (E)ₙ where n = 0-3, and E is a polar electron-withdrawing function selected from -CO₂H, -CO₂R¹, -CONR¹R, -SO₃H,
-SO₂NR¹R, -SO₂CF₃, -COCH₃, and -CN where R¹ and R are independently -H, C₁-C₄ alkyl, -CH₂COOH, -CH₂CH₂OH, or phenyl or R¹ and R together are -(CH₂)₄-, -(CH₂)₅-, or -CH₂CH₂OCH₂CH₂-;
R¹ is -H, -CH₃, -C₂H₅, other lower alkyl up to C₄, -COOH, C₁-C₄ alkoxy, or -OC(O)CH₃; or
R¹ and R together are -CH = CH-CH =CH-, both X and Y are double bonds and D is N so that D, R¹, and R form an acridine ring system;
R⁴¹ is -OH, -OCH₂COOH, -OCH₂CH₂OH, methoxy or C₂-C₄ alkoxy, -NR¹⁴R¹⁵, -COOH, -C(O)NR¹⁴R¹⁵, or -OC(O)CH₃ where R¹⁴ and R¹⁵ are independently -H, C₁-C₄ alkyl, -CH₂COOH, -CH₂CH₂OH, or phenyl;
R⁴ is -H, -CH₃ or -COOH;
W and T are each H or together are O or NR³;
X is a double bond when D is N, and a single bond when D is O;
Y may be either a double or a single bond;
and
Z is a moiety forming a (hetero)aromatic ring;
and L is H;
a ligand of formula (i) or formula (ii);
-CH₂C(O)NR¹R where R¹ and R are independently -H, C₁-C₄ alkyl, -CH₂COOH, -CH₂CH₂BH, or phenyl, or R¹ and R together are -(CH₂)₄-, -(CH₂)₅-, or -CH₂CH₂OCH₂CH₂- (to form a pyrrolidine, piperidine, or morpholine ring, respectively);
-CH₂COOH;
-2-pyridylmethyl;
-2-tetrahydrofuranylmethyl;
-CH₂CH₂OR^{a} where R^{a} is -H, C₁-C₄ alkyl, -CH₂COOH, -CH₂CH₂OH, or phenyl; or
-2,5-dialkoxyphenyl where the alkoxy substituent is C₁-C₄ alkoxy.

2. A method of claim 1, wherein 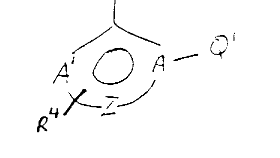 is 2,4-dicarboxyphenyl, 3,4-dicarboxyphenyl, or 5-carboxyoxazol-2-yl.

3. A method of non-destructive observation of an alkali metal ion concentration in tissue, comprising employing in said tissue an alkali metal chelating agent and observing or measuring fluorescence, wherein the chelating agent is an aza-crown ether of the formula: 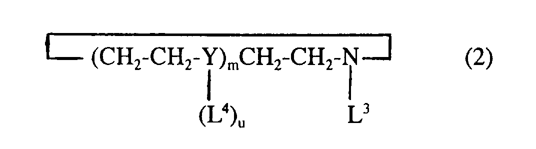 wherein:
m is an integer from 3 to 5;
each Y is independently N or O
u is 1 if Y is N and 0 if Y is O
L³ is quinone or a ligand of formula (iii) or formula (iv) below 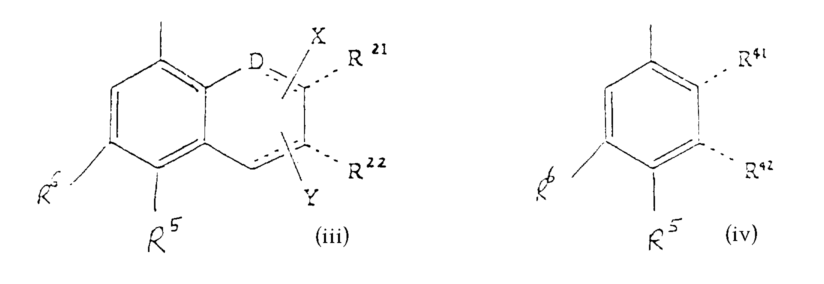 wherein:
D, R¹, R, R⁴¹, R⁴, X and Y are as defined in claim 1;
R⁵ is H,-NH₂, -CHO or -NHCOCH₃, or -NHCOCH₂CH₂COO⁻ or other acylated amino group; and
R⁶ is H,-OH, or -OCH₃ or other alkoxy, provided that one of R⁵ and R⁶ is not H;
and L⁴ is H;
quinone;
a ligand of formula (iii) or formula (iv);
-CH₂C(O)NR¹R where R¹ and R are independently -H, C₁-C₄ alkyl, -CH₂COOH, -CH₂CH₂OH, or phenyl, or R¹ and R together are -(CH₂)₄-, -(CH₂)₅-,or -CH₂CH₂OCH₂CH₂- (to form a pyrrolidine, piperidine, or morpholine ring, respectively);
-CH₂COOH; .
-2-pyridylmethyl;
-2-tetrahydrofuranylmethyl;
-CH₂CH₂OR^{a} where R^{a} is -H, C₁-C₄ alkyl, -CH₂COOH, -CH₂CH₂OH, or phenyl; or
-2,5-dialkoxyphenyl where the alkoxy substituent is C₁-C₄ alkoxy.

4. A method of claim 3, wherein R⁵ is H, -NH₂, -CHO, -NHCOCH₃, -NHCOCH₂CH₂COO⁻ or other amido group.

5. A method of claim 4, wherein
R⁵ is H, -NH₂, -CH₃, -NHCOCH₃ or -NHCOCH₂CH₂COO⁻; and
R⁶ is H, -OH or -OCH₃.

6. A method of any one of claims 3 to 5 wherein, in any L³ or L⁴ group of formula (iii), R⁵ is not -NH₂.

7. A method of any one of claims 1 to 6, wherein at least one of the hydrogens on the aza-crown ether core carbons is substituted with a substituent selected from: -COOH, -CH₂OH, -C(O)N(CH₃)₂, or C₁-C₄ alkyl; or hydrogens on adjacent core carbons of the aza-crown ether are substituted with R³¹ and R³ wherein R³¹ and R³ together are -(CH₂)₃- or -(CH₂)₄- (wherein R³¹ and R³ are part of an aliphatic system), or R³¹ and R³ together are -(CH)₃- (wherein R³¹ and R³ are part of an aromatic system); or hydrogens on non-adjacent core carbons of the aza-crown ether are substituted with R³¹ and R³ wherein R³¹ and R³ together are -(CH₂)₂- or -(CH₂)₃- (wherein R³¹ and R³ are part of an aliphatic system), or R³¹ and R³ together are -(CH)₃- (wherein R³¹ and R³ are part of an aromatic system), and wherein optionally each L¹ and L group is of formula G, N, O or P of Figure 1 or, as the case may be, each L³ and L⁴ group is of formula C, D, E, F, H, I, J, K, L or M of Figure 1.

8. A method of claim 7 wherein the aza-crown ether core is of formula (B) or (D) below: 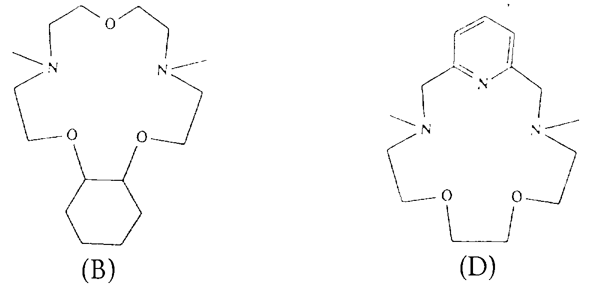 the sp³-hybridised N atom being substituted as defined in any one of claims 1 to 6.

9. A method of any one of claims 1 to 7, wherein the aza-crown ether is a monoaza-, diaza-, triaza-, tretraaza-, pentaaza- or hexaaza- crown ether.

10. A method of claim 9, wherein the aza-crown ether is monoaza-12-crown-4, diaza-12-crown-4, monoaza-15-crown-5, diaza-15-crown-5, monoaza-18-crown-6 or a diaza-18-crown-6 ether.

11. A method of claim 10, wherein the aza-crown ether is 1,7-diaza-4,10,13-trioxacyclopentadecane or 1, 10-diaza-4, 7, 13, 16-tetraoxacyclo octadecane.

12. A method of claim 1 or claim 2 or of any of claims 7 to 11 when dependent on claim 1 or claim 2 wherein one or more of the L groups is replaced by an L⁴ group of any of claims 3 to 6.

13. A method of non-destructive observation of an alkali metal ion concentration in tissue, comprising employing in said tissue an alkali metal chelating agent and observing or measuring fluorescence, wherein the chelating agent is a compound comprised of the combination of (1) an aza-crown ether and (2) at least one ligand(s), wherein the ligand(s) is/are attached to the aza-crown ether via the sp³-hybridized core nitrogen(s), and wherein (1) the crown ether is selected from the group consisting of monoaza-12-crown-4, diaza-12-crown-4, monoaza-15-crown-5, diaza-15-crown-5, monoaza -18-crown-6 and diaza-18-crown-6, and (2) the ligand(s) is/are selected from the groups whose formulas are shown in Figure 1 as group B, C, D, E, F, G, H, I, J, K, L, M, N, O, and P.

14. A method of any one of claims 1 to 9, 11 or 12 wherein the aza-crown ether is a symmetrical diaza-crown ether or an asymmetrical diaza-crown ether, as hereinbefore defined.

15. A method of any of claims 1 to 14 wherein either:
(a) the aza-crown ether has at least two core nitrogens having ligands attached, which ligands are identical ligands; or
(b) the aza crown ether has at least two core nitrogens having ligands attached, which ligands are not identical ligands.

16. A method of non-destructive observation of an alkali metal ion concentration in tissue, comprising employing in said tissue an alkali metal chelating agent and observing or measuring fluorescence, wherein the chelating agent is a compound selected from:-
(a) compound SBFP, whose structure is shown in Figure 7(a) as 2NN;
(b) compound SBFO, whose structure is shown in Figure 7(a) as 2OO;
(c) compound SBFI, whose structure is shown in Figure 7(a) as 2PP;
(d) compound PBFP, whose structure is shown in Figure 7(a) as 3NN;
(e) compound PBFO, whose structure is shown in Figure 7(b) as 3OO; or
(f) compound PBFI, whose structure is shown in Figure 7(b) as 3PP.

17. A method of any one of claims 1 to 16 wherein the compound is in the form of an ester wherein any carboxylates are esterified to form physiologically hydrolyzable esters, preferably acetoxymethyl esters, or the compound is in the form of a pharmaceutically or veterinarily acceptable salt or ester thereof.

18. A method of non-destructive observation of an alkali metal ion concentration in tissue, comprising employing in said tissue an alkali metal chelating agent and observing or measuring fluorescence, wherein the chelating agent is an aza-crown ether of the formula 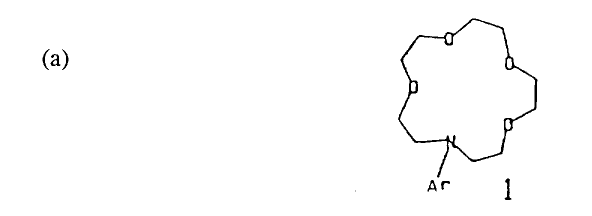 wherein Ar is a group whose formula is shown in Figure 1 as group C, D, E, F, G, H, I, J, K, L, M, N, O or P; 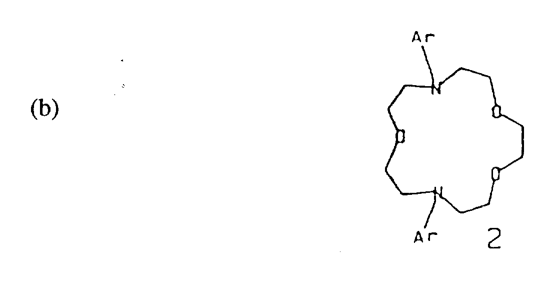 wherein the Ar groups are
(i) identical, or
(ii) not identical
and in either case (i) or (ii) are each a group whose formula is shown in Figure 1 as group C, D, E, F, G, H, I, J, K, L, M, N, O or P; or 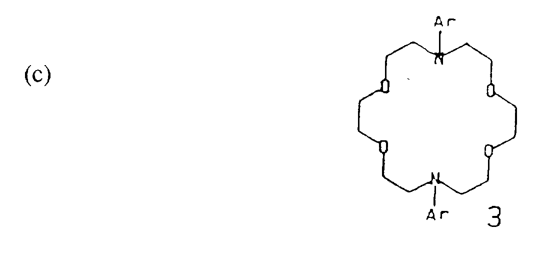 wherein the Ar groups are
(i) identical, or
(ii) not identical
and in either case (i) or (ii) are each a group whose formula is shown in Figure 1 as group C, D, E, F, G, H, I, J, K, L, M, N, O or P.

19. A method of claim 18 wherein the aza-crown ether core is substituted as defined in claim 7 or claim 8 and/or the compound is in the form of an ester wherein any carboxylates are esterified to form a physiologically hydrolysable ester or is in the form of a pharmaceutically or veterinarily acceptable salt or ester thereof.

20. A process for preparing a macrocyclic fluorescent alkali metal chelating agent, comprising forming a combination of an aza-crown ether and one or more ligands attached to the crown ether via one or more of the sp³-hybridised core nitrogen(s), wherein the crown ether is of the formula 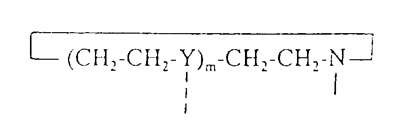 wherein m is an integer from 3 to 5 and each Y is independently O or N and at least one of the ligand(s) is of formula (i) or (ii) of claim 1 or of formula (iii) or (iv) of claim 3, the combination optionally being further defined by the specific feature(s) of any one or more of claims 2 or 4 to 18.

21. A process for preparing a compound as defined in any one of claims 1 to 19 comprising either:
(a) effecting a method chemically equivalent to a method comprising that described hereinbefore under any one of the headings "N,N-his-(2-(3,4-dicarboxyphenyl)-5-methoxybenzofuran-6-yl)-diaza[15]-crown-5 (2NN Me ester) (=SBFP/Me)", "N,N-bis-(2-(5-carboxyoxazol-2-yl)-5-methoxybenzofuran-6-yl)- diaza[15]-crown-5 (2OO ethyl ester) (=SBFO/Et)" or "N,N-bis-(2-(2,4-dicarboxyphenyl)-5-methoxybenzofuran-6-yl)-diaza[15]-crown-5(2PP methyl ester) (=SBFI/Me)", optionally preceded by one or more of the method steps in sequence leading up to compound 2MM as set out hereinbefore under "COMPOUND SYNTHESIS"; or
(b) effecting a process analogous to (a) but employing as the crown ether 1,7-diaza-4, 10-dioxacyclododecane or 1,10-diaza-4,7,13,16-tetraoxacyclooctadecane.

22. The use of a compound as defined in any one of claims 1 to 19 as a fluorescent indicator dye for alkali metal cations.

## Patentansprüche (Patentansprüche für folgende(n) Vertragsstaat(en): AT, BE, CH, DE, FR, GB, GR, IT, LI, LU, NL, SE)

1. Aza-Kronenether der Formel 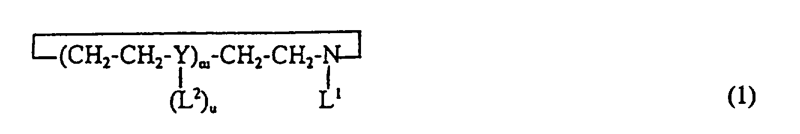 worin
m eine ganze Zahl von 3 bis 5 ist;
jedes Y unabhängig von einem anderen für N oder O steht;
u 1 ist, wenn Y für N steht, und 0 ist, wenn Y für O steht;
L¹ ein Ligand der unten angegebenen Formeln (i) oder (ii) ist 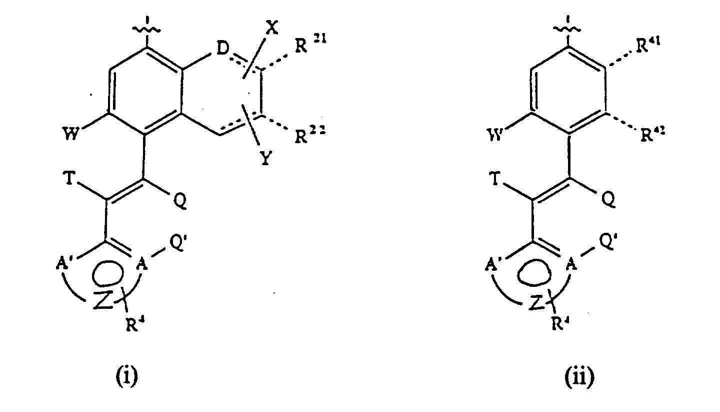 worin
A und A¹ unabhängig voneinander für C, N, O oder S stehen;
D für N oder O steht;
Q für H oder NR¹R steht, worin R¹ und R unabhängig voneinander -H, C₁- bis C₄-Alkyl, -CH₂COOH, -CH₂CH₂OH oder Phenyl sind, oder R¹ und R zusammengenommen -(CH₂)₄-, -(CH₂)₅- oder -CH₂CH₂OCH₂CH₂- sind;
Q¹ für H steht, wenn A nicht für NR³, O oder S steht; oder
Q und Q¹ zusammengenommen für NR³ stehen und A für C steht;
R³ für H, -CH₃, -C₂H₅ oder -CH₂CFSH steht;
R⁴ für (E)ₙ steht, worin n 0 bis 3 ist und E eine polare, Elektronen-abziehende funktionelle Gruppe ist, die gewählt ist aus -CO₂H, -CO₂R¹, -CONR¹R, -SO₃H, -SO₂NR¹R, -SO₂CF₃, -COCH₃ und -CN, worin R¹ und R unabhängig voneinander -H, C₁- bis C₄-Alkyl, -CH₂COOH, -CH₂CH₂OH oder Phenyl sind, oder R¹ und R zusammengenommen -(CH₂)₄-, -(CH₂)₅- oder -CH₂CH₂OCH₂CH, sind;
R¹ für -H, -CH₃, -C₂H₅, andere Niederalkyl-Gruppen bis zu C₄, -COOH, C₁- bis C₄-Alkoxy oder -OC(O)CH₃ steht; oder
R¹ und R zusammengenommen -CH=CH-CH=CH- sind, sowohl X als auch Y Doppelbindungen sind und D für N steht, so daß D, R¹ und R ein Acridin-Ringsystem bilden;
R⁴¹ für -OH, -OCH₂COOH, -OCH₂CH₂OH, Methoxy oder C₂- bis C₄-Alkoxy, -NR¹⁴R¹⁵, -COOH, -C(O)NR¹⁴R¹⁵ oder -OC(O)CH₃ steht, worin R¹⁴ und R¹⁵ unabhängig voneinander -H, C₁- bis C₄-Alkyl, -CH₂CFSH, -CH₂CH₂OH oder Phenyl sind;
R⁴ für -H, -CH₃ oder -COOH steht;
W und T jeweils für H stehen oder zusammengenommen für O oder NR³ stehen;
X eine Doppelbindung ist, wenn D für N steht, und eine Einfachbindung ist, wenn
D für O steht;
Y entweder eine Doppelbindung oder eine Einfachbindung sein kann; und
Z eine Einheit ist, die einen heterocyclischen aromatischen Ring bildet;
und L steht für H;
einen Liganden der Formeln (i) oder (ii);
-CH₂C(O)NR¹R, worin R¹ und R unabhängig voneinander -H, C₁- bis C₄-Alkyl,
-CH₂COOH, -CH₂CH₂OH oder Phenyl sind, oder R¹ und R zusammengenommen -(CH₂)₄-,
-(CH₂)₅- oder -CH₂CH₂OCH₂CHᵣ sind (um einen Pyrrolidin-, Piperidin- bzw. Morpholinring zu bilden);
-CH₂COOH;
-2-pyridylmethyl;
-2-tetrahydrofuranylmethyl;
-CH₂CH₂OR^{a}, worin R^{a} für -H, C₁- bis C₄-Alkyl, -CH₂COOH, -CH₂CH₂OH oder Phenyl steht; oder
-2,5-dialkoxyphenyl, worin der Alkoxy-Substituent eine C₁- bis C₄-Alkoxy-Gruppe ist.

2. Verbindung nach Anspruch 1, worin 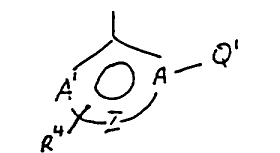 für eine 2,4-Dicarboxyphenyl-Gruppe, 3,4-Dicarboxyphenyl-Gruppe oder 5-Carboxyoxazol-2-yl-Gruppe steht.

3. Aza-Kronenether der Formel 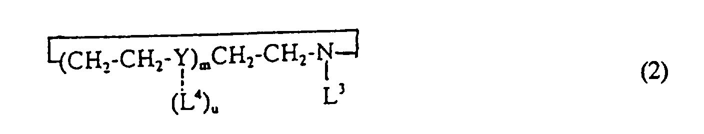 worin
m eine ganze Zahl von 3 bis 5 ist;
jedes Y unabhängig von einem anderen für N oder O steht;
u 1 ist, wenn Y für N steht, und 0 ist, wenn Y für O steht;
L³ Chinon oder ein Ligand der unten angegebenen Formeln (iii) oder (iv) ist 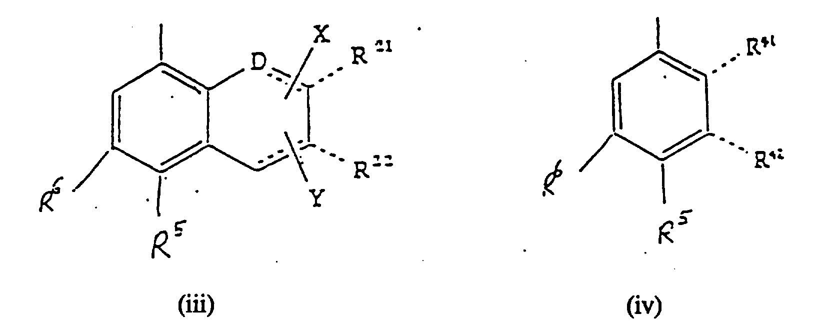
worin
D, R¹, R, R⁴¹, R⁴, X und Y dieselbe Bedeutung haben, wie in Anspruch 1;
R⁵ für -H, -NH₂, -CHO oder -NHCOCH₃ oder -NHCOCH₂CH₂COO⁻ oder eine andere acylierte Amino-Gruppe steht; und
R⁶ für -H, -OH oder -OCH₃ oder eine andere Alkoxy-Gruppe steht, mit der Maßgabe, daß einer der Reste R⁵ und R⁶ nicht H ist;
und L⁴ steht für H;
Chinon;
einen Liganden der Formeln (iii) oder (iv);
-CH₂C(O)NR¹(R, worin R¹ und R unabhängig voneinander -H, C₁- bis C₄-Alkyl,
-CH₂COOH, -CH₂CH₂OH oder Phenyl sind, oder R¹ und R zusammengenommen -(CH₂)₄-,
-(CH₂)₅- oder -CH₂CH₂OCH₂CH₂-, sind (um einen Pyrrolidin-, Piperidin- bzw. Morpholinring zu bilden);
-CH₂COOH;
-2-pyridylmethyl;
-2-tetrahydrofuranylmethyl;
-CH₂CH₂OR^{a}, worin R^{a} für -H, C₁- bis C₄-Alkyl, -CH₂COOH, -CH₂CH₂OH oder Phenyl steht; oder
-2,5-dialkoxyphenyl, worin der Alkoxy-Substituent eine C₁- bis C₄-Alkoxy-Gruppe ist.

4. Verbindung nach Anspruch 3, worin R⁵ für -H, -NH₂, -CHO, -NHCOCH₃, -NHCOCH₂CH₂COO⁻ oder eine andere Amido-Gruppe steht.

5. Verbindung nach Anspruch 4, worin R⁵ für -H, -NH₂, -CH₃, -NHCOCH₃ oder -NHCOCH₂CH₂COO⁻ steht und R⁶ für -H, -OH oder -OCH₃ steht.

6. Verbindung nach einem der Ansprüche 3 bis 5, worin in jeder Gruppe L³ oder L⁴ der Formel (iii) R⁵ nicht -NH₂ ist.

7. Verbindung nach einem der Ansprüche 1 bis 6, worin wenigstens eines der Wasserstoff-Atome an den Kern-Kohlenstoff-Atomen des Aza-Kronenethers ersetzt ist durch einen Substituenten, der gewählt ist aus -COOH, -CH₂OH, -C(O)N(CH₃)₂ oder C₁- bis C₄-Alkyl; oder Wasserstoff-Atome an benachbarten Kern-Kohlenstoff-Atomen des Aza-Kronenethers ersetzt sind durch R³¹ und R³, worin R³¹ und R³ zusammengenommen -(CH₂)₃- oder -(CH₂)₄- sind (worin R³¹ und R³ Teil eines aliphatischen Systems sind), oder R³¹ und R³ zusammengenommen -(CH)₃- sind (worin R³¹ und R³ Teil eines aromatischen Systems sind); oder Wasserstoff-Atome an nicht-benachbarten Kern-Kohlenstoff-Atomen des Aza-Kronenethers ersetzt sind durch R³¹ und R³, worin R³¹ und R³ zusammengenommen -(CH₂)₂- oder -(CH₂)₃- sind (worin R³¹ und R³ Teil eines aliphatischen Systems sind), oder R³¹ und R³ zusammengenommen -(CH)₃- sind (worin R³¹ und R³ Teil eines aromatischen Systems sind) und worin gegebenenfalls jede Gruppe L¹ und L die Formel G, N, O oder P von Figur 1 aufweist oder je nach dem jede Gruppe L³ und L⁴ die Formel C, D, E, F, H, I, J, K, L oder M von Figur 1 aufweist.

8. Verbindung nach Anspruch 7, worin der Aza-Kronenether-Kern die folgende Formel (B) oder (D) aufweist 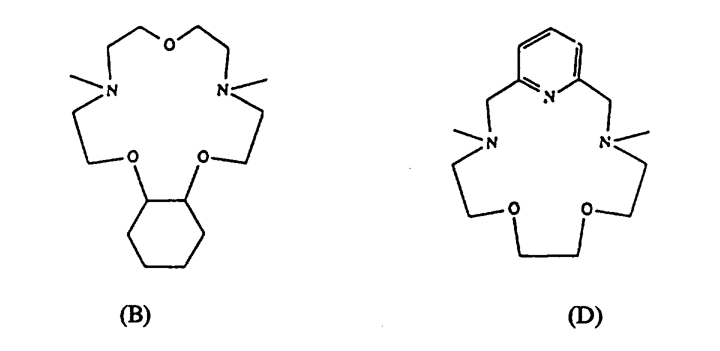 und das sp³-hybridisierte N-Atom substituiert ist, wie es in einem der Ansprüche 1 bis 6 definiert ist.

9. Verbindung nach einem der Ansprüche 1 bis 7, worin der Aza-Kronenether ein Monoaza-, Diaza-, Triaza-, Tetraaza-, Pentaaza- oder Hexaaza-Kronenether ist.

10. Verbindung nach Anspruch 9, worin der Aza-Kronenether ein Monoaza-12-Krone-4-, Diaza-12-Krone-4-, Monoaza-15-Krone-5- , Diaza-15-Krone-5-, Monoaza-18-Krone-6- oder Diam-18-Krone-6-Ether ist.

11. Verbindung nach Anspruch 10, worin der Aza-Kronenether 1,7-Diaza-4,10,13-tri-oxacyclopentadecan oder 1,10-Diaza-4,7,13,16-tetraoxacyclooctadecan ist.

12. Aza-Kronenether nach Anspruch 1 oder Anspruch 2 oder einem der Ansprüche 7 bis 11, wenn diese von Anspruch 1 oder Anspruch 2 abhängig sind, worin eine oder mehrere der Gruppen L ersetzt ist/sind durch eine Gruppe L⁴ nach einem der Ansprüche 3 bis 6.

13. Verbindung bestehend aus der Kombination von (1) einem Aza-Kronenether mit (2) wenigstens einem Liganden, worin der/die Ligand(en) an dem Aza-Kronenether über das/die sp³-hybridisierte(n) Kern-Stickstoff-Atom(e) gebunden ist/sind, und worin (1) der Kronenether gewählt ist aus der aus Monoaza- 12-Krone-4, Diaza-12-Krone-4, Monoaza-15-Krone-5, Diaza-15-Krone-5, Monoaza-18-Krone-6 und Diaza-18-Krone-6 bestehenden Gruppe, und (2) der/die Ligand(en) gewählt ist/sind aus den Gruppen, deren Formeln in Figur 1 als Gruppen B, C, D, E, F, G, H, I, J, K, L, M, N, O und P gezeigt sind.

14. Verbindung nach einem der Ansprüche 1 bis 9, 11 oder 12, worin der Aza-Kronenether ein symmetrischer Diaza-Kronenether oder ein unsymmetrischer Diaza-Kronenether gemäß vorstehender Definition ist.

15. Verbindung nach einem der Ansprüche 1 bis 14, worin entweder
(a) der Aza-Kronenether wenigstens zwei Kern-Stickstoff-Atome aufweist, welche gebundene Liganden aufweisen, wobei die Liganden identische Liganden sind; oder
(b) der Aza-Kronenether wenigstens zwei Kern-Stickstoff-Atome aufweist, welche gebundene Liganden aufweisen, wobei die Liganden nicht-identische Liganden sind.

16. Verbindung gewählt aus
(a) der Verbindung SBFP, deren Struktur in Figur 7 (a) als 2 NN angegeben ist;
(b) der Verbindung SBFO, deren Struktur in Figur 7 (a) als 2 OO angegeben ist;
(c) der Verbindung SBFI, deren Struktur in Figur 7 (a) als 2 PP angegeben ist;
(d) der Verbindung PBFP, deren Struktur in Figur 7 (a) als 3 NN angegeben ist;
(e) der Verbindung PBFO, deren Struktur in Figur 7 (b) als 3 OO angegeben ist; oder
(f) der Verbindung PBFI, deren Struktur in Figur 7 (b) als 3 PP angegeben ist.

17. Verbindung nach einem der Ansprüche 1 bis 16, worin die Verbindung in Form eines Esters vorliegt, worin alle Carboxylat-Reste unter Bildung von physiologisch hydrolysierbaren Estern, vorzugsweise Acetoxymethylestern, verestert sind oder die Verbindung in Form eines pharmazeutisch oder veterinärmedizinisch annehmbaren Salzes oder seines Esters vorliegt.

18. Aza-Kronenether der Formel 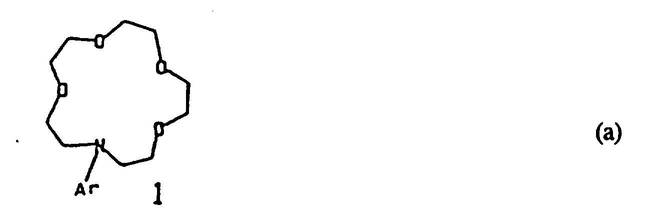 worin Ar für eine Gruppe steht, deren Formel in Figur 1 als Gruppe C, D, E, F, G, H, I, J, K, L, M, N, O oder P angegeben ist; 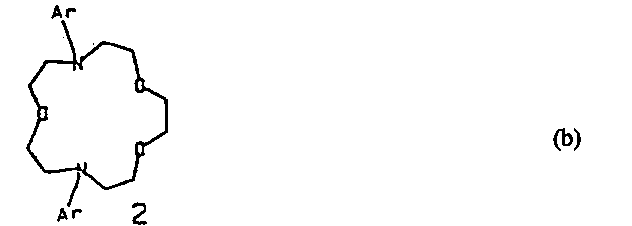 worin die Ar-Gruppen (i) identisch oder (ii) nicht-identisch sind und in jedem der Fälle (i) oder (ii) jeweils für eine Gruppe stehen, deren Formel in Figur 1 als Gruppe C, D, E, F, G, H, I, J, K, L, M, N, O oder P angegeben ist; oder 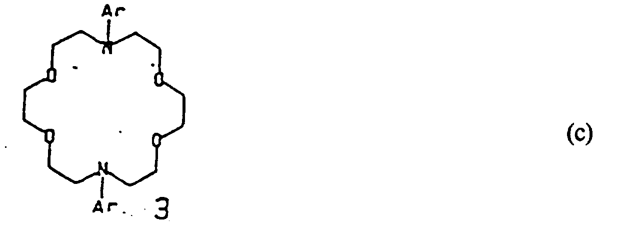 worin die Ar-Gruppen (i) identisch oder (ii) nicht-identisch sind und in jedem der Fälle (i) oder (ii) jeweils für eine Gruppe stehen, deren Formel in Figur 1 als Gruppe C, D, E, F, G, H, I, J, K, L, M, N, O oder P angegeben ist.

19. Verbindung nach Anspruch 18, worin der Aza-Kronenether-Kem substituiert ist gemäß Anspruch 7 oder Anspruch 8 und/oder die Verbindung in Form eines Esters vorliegt, worin alle Carboxylat-Reste unter Bildung eines physiologisch hydrolysierbaren Esters verestert sind oder die Verbindung in Form eines pharmazeutisch oder veterinärmedizinisch annehmbaren Salzes oder seines Esters vorliegt.

20. Verfahren zur Herstellung eines makrocyclischen fluoreszierenden Alkalimetall-Chelatisierungsmittels, das den Schritt umfaßt, daß man eine Kombination eines Aza-Kronenethers mit einem oder mehreren Ligand(en), der/die an dem Aza-Kronenether über ein oder mehrere sp³-hybridisierte(s) Kern-Stickstoff-Atom(e) gebunden ist/sind, bildet, worin der Kronenether die Formel 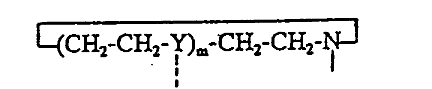 aufweist, worin m eine ganze Zahl von 3 bis 5 ist und jedes Y unabhängig von einem anderen für O oder N steht und wenigstens einer der Liganden die Formeln (i) oder (ii) gemäß Anspruch 1 oder die Formeln (iii) oder (iv) gemäß Anspruch 3 aufweist, wobei die Kombination gegebenenfalls weiter definiert wird durch das/die besondere(n) Merkmal(e) gemäß einem oder mehreren der Ansprüche 2 oder 3 bis 11 oder wie es/sie in einem der Ansprüche 12 bis 18 definiert ist/sind.

21. Verfahren zur Herstellung einer Verbindung nach Anspruch 1, Anspruch 3 oder Anspruch 12, welches die Schritte umfaßt, daß man entweder
(a) ein Verfahren durchführt, das chemisch equivalent einem Verfahren ist, das dasjenige umfaßt, das vorstehend unter einer der Überschriften "N,N-Bis-(2-(3,4-dicarboxy-phenyl-)5-methoxybenzofuran-6-yl-)diaza[15]-Krone-5-(2NN-Me-ester) (= SBFP/ Me)", "N,N-Bis-(2-(5-Carboxyoxazol-2-yl-)5-methoxybenzofuran-6-yl)-diaza[ 15]-Krone-5-(2OO-ethylester) ( = SBFO/Et)" oder"N,N-Bis-(2-(2,4-dicarboxyphenyl-)5-methoxybenzofuran-6-yl-)diaza[15]-Krone-5-(2PP-methylester) (= SBFI/Me)" beschrieben wurde, dem gegebenenfalls einer oder mehrere der Verfahrensschritte vorausgeht, der in Folge letztendlich zur Verbindung 2 MM führt, wie vorstehend im Kapitel "Synthese der Verbindungen" beschrieben wurde; oder
(b) ein zu (a) analoges Verfahren durchführt, jedoch als Kronenether 1,7-Diaza-4,10-dioxacyclododecan oder 1,10-Diaza-4,7,13,16-tetraoxacyclooctadecan verwendet.

22. Verwendung einer Verbindung, wie sie in einem der Ansprüche 1 bis 18 definiert ist, bei der Herstellung einer Alkalimetall chelatisierenden Formulierung zum nicht-zerstörenden Beobachten eines Alkalimetall-Ionen-Konzentrats in Gewebe.

23. Verfahren zum nicht-zerstörenden Beobachten einer Alkalimetall-Ionen-Konzentration in Gewebe, welches die Schritte umfaßt, daß man in dem Gewebe als Alkalimetall-Chelatisierungsmittel eine Verbindung gemäß einem der Ansprüche 1 bis 18 verwendet und die Fluoreszenz beobachtet oder mißt.

## Patentansprüche (Patentansprüche für folgende(n) Vertragsstaat(en): ES)

1. Verfahren zum nicht-zerstörenden Beobachten einer Alkalimetall-Ionen-Konzentration in Gewebe, welches die Schritte umfaßt, daß man in dem Gewebe ein Alkalimetall-Chelatisie-rungsmittel verwendet und die Fluoreszenz beobachtet oder mißt, worin das Chelatisierungsmittel ein Aza-Kronenether der Formel 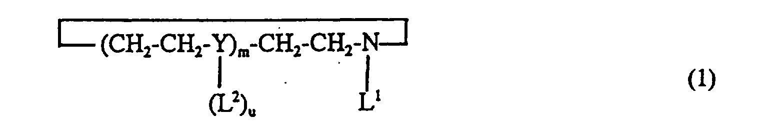 ist, worin
m eine ganze Zahl von 3 bis 5 ist;
jedes Y unabhängig von einem anderen für N oder O steht;
u 1 ist, wenn Y für N steht, und 0 ist, wenn Y für O steht;
L¹ ein Ligand der unten angegebenen Formeln (i) oder (ii) ist 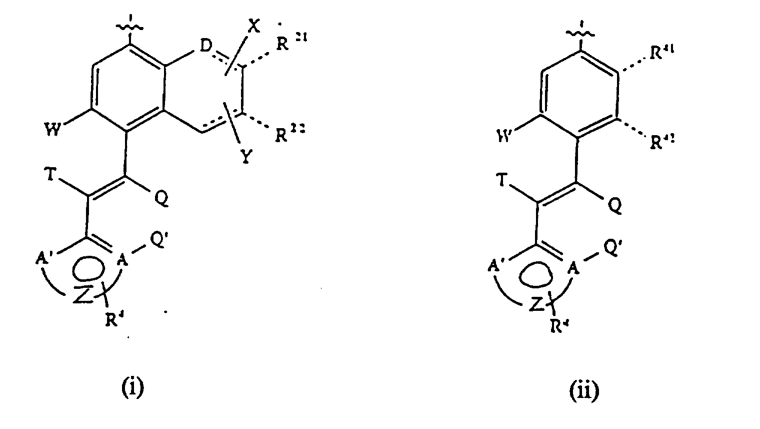
worin
A und A¹ unabhängig voneinander für C, N, O oder S stehen;
D für N oder O steht;
Q für H oder NR¹R steht, worin R¹ und R unabhängig voneinander -H, C₁- bis C₄-Alkyl, -CH₂COOH, -CH₂CH₂OH oder Phenyl sind, oder R¹ und R zusammengenommen -(CH₂)₄-, -(CH₂)₅- oder -CH₂CH₂OCH₂CH, sind;
Q¹ für H steht, wenn A nicht für NR³, O oder S steht; oder
Q und Q¹ zusammengenommen für NR³ stehen und A für C steht;
R³ für H, -CH₃, -C₂H₅ oder -CH₂COOH steht;
R⁴ für (E)ₙ steht, worin n 0 bis 3 ist und E eine polare, Elektronen-abziehende funktionelle Gruppe ist, die gewählt ist aus -CO₂H, -CO₂R¹, -CONR¹R, -SO₃H, -SO₂NR¹R,-SO₂CF₃, -COCH₃ und -CN, worin R¹ und R unabhängig voneinander -H, C₁- bis C₄-Alkyl, -CH₂COOH, -CH₂CH₂OH oder Phenyl sind, oder R¹ und R zusammengenommen -(CH₂)₄-, -(CH₂)₅- oder -CH₂CH₂OCH₂CH₂ sind;
R¹ für -H, -CH₃, -C₂H₅, andere Niederalkyl-Gruppen bis zu C₄, -COOH, C₁- bis C₄-Alkoxy oder -OC(O)CH₃ steht; oder
R¹ und R zusammengenommen -CH=CH-CH=CH- sind, sowohl X als auch Y Doppelbindungen sind und D für N steht, so daß D, R¹ und R ein Acridin-Ringsystem bilden;
R⁴¹ für -OH, -OCH₂COOH, -OCH₂CH₂OH, Methoxy oder C₂- bis C₄-Alkoxy, -NR¹⁴R¹⁵, -COOH, -C(O)NR¹⁴R¹⁵ oder -OC(O)CH₃ steht, worin R¹⁴ und R¹⁵ unabhängig voneinander -H, C₁- bis C₄-Alkyl, -CH₂COOH, -CH₂CH₂OH oder Phenyl sind;
R⁴ für -H, -CH₃ oder -COOH steht;
W und T jeweils für H stehen oder zusammengenommen für O oder NR³ stehen;
X eine Doppelbindung ist, wenn D für N steht, und eine Einfachbindung ist, wenn D für O steht;
Y entweder eine Doppelbindung oder eine Einfachbindung sein kann; und
Z eine Einheit ist, die einen heterocyclischen aromatischen Ring bildet;
und L steht für H;
einen Liganden der Formeln (i) oder (ii);
-CH₂C(O)NR¹R, worin R¹ und R unabhängig voneinander -H, C₁- bis C₄-Alkyl, -CH₂COOH, -CH₂CH₂OH oder Phenyl sind, oder R¹ und R zusammengenommen -(CH₂)₄-, -(CH₂)₅- oder -CH₂CH₂OCH₂CH₂- sind (um einen Pyrrolidin-, Piperidin- bzw. Morpho-linring zu bilden);
-CH₂COOH;
-2-pyridylmethyl;
-2-tetrahydrofuranylmethyl;
-CH₂CH₂OR^{a}, worin R^{a} für -H, C₁- bis C₄-Alkyl, -CH₂COOH, -CH₂CH₂OH oder Phenyl steht; oder
-2,5-dialkoxyphenyl, worin der Alkoxy-Substituent eine C₁- bis C₄-Alkoxy-Gruppe ist.

2. Verfahren nach Anspruch 1, worin 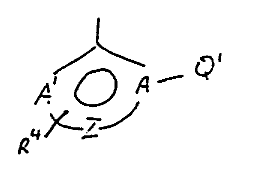 für eine 2,4-Dicarboxyphenyl-Gruppe, 3,4-Dicarboxyphenyl-Gruppe oder 5-Carboxyoxazol-2-yl-Gruppe steht.

3. Verfahren zum nicht-zerstörenden Beobachten einer Alkalimetall-Ionen-Konzentration in Gewebe, welches die Schritte umfaßt, daß man in dem Gewebe ein Alkalimetall-Chelatisierungsmittel verwendet und die Fluoreszenz beobachtet oder mißt, worin das Chelatisierungsmittel ein Aza-Kronenether der Formel 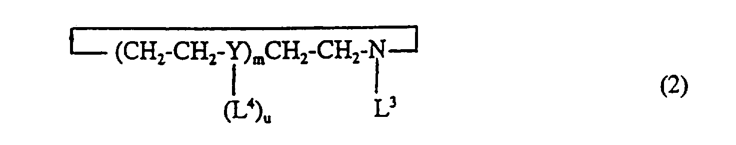 ist, worin
m eine ganze Zahl von 3 bis 5 ist;
jedes Y unabhängig von einem anderen für N oder O steht;
u 1 ist, wenn Y für N steht, und 0 ist, wenn Y für O steht;
L³ Chinon oder ein Ligand der unten angegebenen Formeln (iii) oder (iv) ist 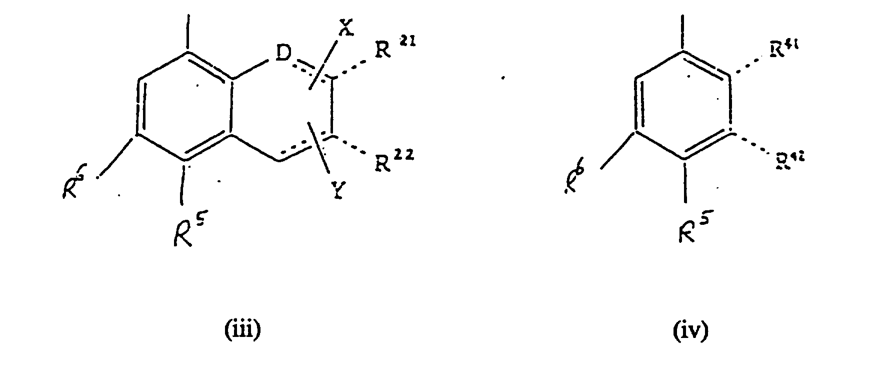
worin
D, R¹, R, R⁴¹, R⁴, X und Y dieselbe Bedeutung haben, wie in Anspruch 1;
R⁵ für -H, -N₂, -CHO oder -NHCOCH₃ oder -NHCOCH₂CH₂COO⁻ oder eine andere acylierte Amino-Gruppe steht; und
R⁶ für -H, -OH oder -OCH₃ oder eine andere Alkoxy-Gruppe steht, mit der Maßgabe, daß einer der Reste R⁵ und R⁶ nicht H ist;
und L⁴ steht für H;
Chinon;
einen Liganden der Formeln (iii) oder (iv);
-CH₂C(O)NR¹R, worin R¹ und R unabhängig voneinander -H, C₁- bis C₄-Alkyl,
-CH₂COOH, -CH₂CH₂OH oder Phenyl sind, oder R¹ und R zusammengenommen -(CH₂)₄-,
-(CH₂)₅- oder -CH₂CH₂OCH₂CH₂- sind (um einen Pyrrolidin-, Piperidin- bzw. Morpholinring zu bilden);
-CH₂COOH;
-2-pyridylmethyl;
-2-tetrahydrofuranylmethyl;
-CH₂CH₂OR^{a}, worin R^{a} für -H, C₁- bis C₄-Alkyl, -CH₂COOH, -CH₂CH₂OH oder Phenyl steht; oder
-2,5-dialkoxyphenyl, worin der Alkoxy-Substituent eine C₁- bis C₄-Alkoxy-Gruppe ist.

4. Verfahren nach Anspruch 3, worin R³ für -H, -NH₂, -CHO, -NHCOCH₃, -NHCOCH₂CH₂COO⁻ oder eine andere Amido-Gruppe steht.

5. Verfahren nach Anspruch 4, worin R⁵ für -H, -NH₂, -CH₃, -NHCOCH₃ oder -NHCOCH₂CH₂COO⁻ steht und R⁶ für -H, -OH oder -OCH₃ steht.

6. Verfahren nach einem der Ansprüche 3 bis 5, worin in jeder Gruppe L³ oder L⁴ der Formel (iii) R⁵ nicht -NH₂ ist.

7. Verfahren nach einem der Ansprüche 1 bis 6, worin wenigstens eines der Wasserstoff-Atome an den Kern-Kohlenstoff-Atomen des Aza-Kronenethers ersetzt ist durch einen Substituenten, der gewählt ist aus -COOH, -CH₂OH, -C(O)N(CH₃)₂ oder C₁- bis C₄-Alkyl; oder Wasserstoff-Atome an benachbarten Kern -Kohlenstoff-Atomen des Aza-Kronenethers ersetzt sind durch R³¹ und R³, worin R³¹ und R³ zusammengenommen -(CH₂)₃- oder -(CH₂)4- sind (worin R³¹ und R³ Teil eines aliphatischen Systems sind), oder R³¹ und R³ zusammengenommen -(CH)₃- sind (worin R³¹ und R³ Teil eines aromatischen Systems sind); oder Wasserstoff-Atome an nicht-benachbarten Kern-Kohlenstoff-Atomen des Aza-Kronenethers ersetzt sind durch R³¹ und R³, worin R³¹ und R³ zusammengenommen -(CH₂)₂ oder -(CH₂)₃- sind (worin R³¹ und R³ Teil eines aliphatischen Systems sind), oder R³¹ und R³ zusammengenommen -(CH)₃- sind (worin R³¹ und R³ Teil eines aromatischen Systems sind) und worin gegebenenfalls jede Gruppe L¹ und L die Formel G, N, O oder P von Figur 1 aufweist oder je nach dem jede Gruppe L³ und L⁴ die Formel C, D, E, F, H, I, J, K, L oder M von Figur 1 aufweist.

8. Verfahren nach Anspruch 7, worin der Aza-Kronenether-Kern die folgende Formel (B) oder (D) aufweist 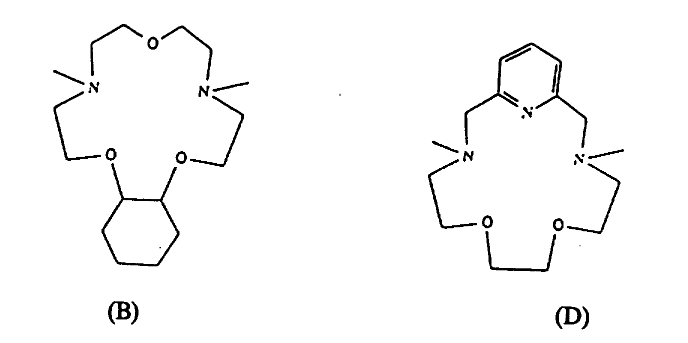 und das sp³-hybridisierte N-Atom substituiert ist, wie es in einem der Ansprüche 1 bis 6 definiert ist.

9. Verfahren nach einem der Ansprüche 1 bis 7, worin der Aza-Kronenether ein Mono-aza-, Diaza-, Triaza-, Tetraaza-, Pentaaza- oder Hexaaza-Kronenether ist.

10. Verfahren nach Anspruch 9, worin der Aza-Kronenether ein Monoaza-12-Krone-4-, Diaza-12-Krone-4-, Monoaza-15-Krone-5-, Diaza-15-Krone-5-, Monoaza-18-Krone-6- oder Diaza-18-Krone-6-Ether ist.

11. Verfahren nach Anspruch 10, worin der Aza-Kronenether 1,7-Diaza-4,10,13-tri-oxacyclopentadecan oder 1,10-Diaza-4,7,13,16-tetraoxacyclooctadecan ist.

12. Verfahren nach Anspruch 1 oder Anspruch 2 oder einem der Ansprüche 7 bis 11, wenn diese von Anspruch 1 oder Anspruch 2 abhängig sind, worin eine oder mehrere der Gruppen L ersetzt ist/sind durch eine Gruppe L⁴ nach einem der Ansprüche 3 bis 6.

13. Verfahren zum nicht-zerstörenden Beobachten einer Alkalimetall-Ionen-Konzentration in Gewebe, welches die Schritte umfaßt, daß man in dem Gewebe ein AlkalimetallChelatisierungsmittel verwendet und die Fluoreszenz beobachtet oder mißt, worin das Chelatisierungsmittel eine Verbindung ist, bestehend aus der Kombination von (1) einem Aza-Kronenether mit (2) wenigstens einem Liganden, worin der/die Ligand(en) an dem Aza-Kronenether über das/die sp³-hybridisierte(n) Kern-Stickstoff-Atom(e) gebunden ist/sind, und worin (1) der Kronenether gewählt ist aus der aus Monoaza-12-Krone-4, Diaza-12-Krone-4, Monoaza-15-Krone-5, Diaza-15-Krone-5, Monoaza-18-Krone-6 und Diaza-18-Krone-6 bestehenden Gruppe, und (2) der/die Ligand(en) gewählt ist/sind aus den Gruppen, deren Formeln in Figur 1 als Gruppen B, C, D, E, F, G, H, I, J, K, L, M, N, O und P gezeigt sind.

14. Verfahren nach einem der Ansprüche 1 bis 9, 11 oder 12, worin der Aza-Kronenether ein symmetrischer Diaza-Kronenether oder ein unsymmetrischer Diaza-Kronenether gemäß vorstehender Definition ist.

15. Verfahren nach einem der Ansprüche 1 bis 14, worin entweder
(a) der Aza-Kronenether wenigstens zwei Kern-Stickstoff-Atome aufweist, welche gebundene Liganden aufweisen, wobei die Liganden identische Liganden sind; oder
(b) der Aza-Kronenether wenigstens zwei Kern-Stickstoff-Atome aufweist, welche gebundene Liganden aufweisen, wobei die Liganden nicht-identische Liganden sind.

16. Verfahren zum nicht-zerstörenden Beobachten einer Alkalimetall-Ionen-Konzentration in Gewebe, welches die Schritte umfaßt, daß man in dem Gewebe ein Alkalimetall-Chelatisierungsmittel verwendet und die Fluoreszenz beobachtet oder mißt, worin das Chelatisierungsmittel eine Verbindung ist, gewählt aus
(a) der Verbindung SBFP, deren Struktur in Figur 7 (a) als 2 NN angegeben ist;
(b) der Verbindung SBFO, deren Struktur in Figur 7 (a) als 2 OO angegeben ist;
(c) der Verbindung SBFI, deren Struktur in Figur 7 (a) als 2 PP angegeben ist;
(d) der Verbindung PBFP, deren Struktur in Figur 7 (a) als 3 NN angegeben ist;
(e) der Verbindung PBFO, deren Struktur in Figur 7 (b) als 3 OO angegeben ist; oder
(f) der Verbindung PBFI, deren Struktur in Figur 7(b) als 3 PP angegeben ist.

17. Verfahren nach einem der Ansprüche 1 bis 16, worin die Verbindung in Form eines Esters vorliegt, worin alle Carboxylat-Reste unter Bildung von physiologisch hydrolysierbaren Estern, vorzugsweise Acetoxymethylestern, verestert sind oder die Verbindung in Form eines pharmazeutisch oder veterinärmedizinisch annehmbaren Salzes oder seines Esters vorliegt.

18. Verfahren zum nicht-zerstörenden Beobachten einer Alkalimetall-Ionen-Konzentration in Gewebe, welches die Schritte umfaßt, daß man in dem Gewebe ein Alkalimetall-Chelatisierungsmittel verwendet und die Fluoreszenz beobachtet oder mißt, worin das Chelatisierungsmittel ein Aza-Kronenether der Formel 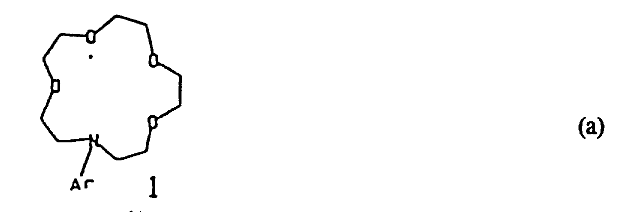 ist, worin Ar für eine Gruppe steht, deren Formel in Figur 1 als Gruppe C, D, E, F, G, H, I, J, K, L, M, N, O oder P angegeben ist; 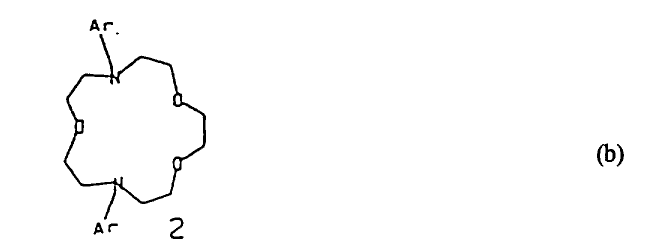 worin die Ar-Gruppen (i) identisch oder (ii) nicht-identisch sind und in jedem der Fälle (i) oder (ii) jeweils für eine Gruppe stehen, deren Formel in Figur 1 als Gruppe C, D, E, F, G, H, I, J, K, L, M, N, O oder P angegeben ist; oder 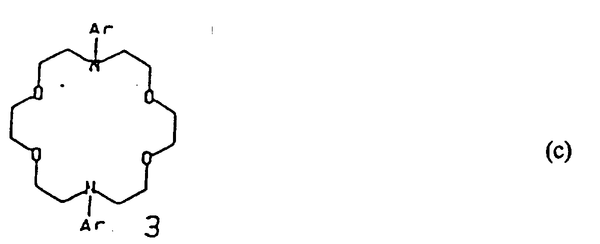 worin die Ar-Gruppen (i) identisch oder (ii) nicht-identisch sind und in jedem der Fälle (i) oder (ii) jeweils für eine Gruppe stehen, deren Formel in Figur 1 als Gruppe C, D, E, F, G, H, I, J, K, L, M, N, O oder P angegeben ist.

19. Verfahren nach Anspruch 18, worin der Aza-Kronenether-Kern substituiert ist gemäß Anspruch 7 oder Anspruch 8 und/oder die Verbindung in Form eines Esters vorliegt, worin alle Carboxylat-Reste unter Bildung eines physiologisch hydrolysierbaren Esters verestert sind oder die Verbindung in Form eines pharmazeutisch oder veterinärmedizinisch annehmbaren Salzes oder seines Esters vorliegt.

20. Verfahren zur Herstellung eines makrocyclischen fluoreszierenden Alkalimetall-Che-latisierungsmittels, das den Schritt umfaßt, daß man eine Kombination eines Aza-Kronenethers mit einem oder mehreren Ligand(en), der/die an dem Aza-Kronenether über ein oder mehrere sp³-hybridisierte(s) Kern-Stickstoff-Atom(e) gebunden ist/sind, bildet, worin der Kronenether die Formel 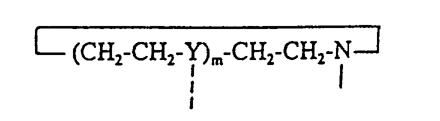 aufweist, worin m eine ganze Zahl von 3 bis 5 ist und jedes Y unabhängig von einem anderen für O oder N steht und wenigstens einer der Liganden die Formeln (i) oder (ii) gemäß Anspruch 1 oder die Formeln (iii) oder (iv) gemäß Anspruch 3 aufweist, wobei die Kombination gegebenenfalls weiter definiert wird durch das/die besondere(n) Merkmal(e) gemäß einem oder mehreren der Ansprüche 2 oder 4 bis 18.

21. Verfahren zur Herstellung einer Verbindung nach einem der Ansprüche 1 bis 19, welches die Schritte umfaßt, daß man entweder
(a) ein Verfahren durchführt, das chemisch equivalent einem Verfahren ist, das dasjenige umfaßt, das vorstehend unter einer der Überschriften "N,N-Bis-(2-(3,4-dicarboxy-phenyl-)5-methoxybenzofuran-6-yl-)diaza[15]-Krone-5-(2NN-Me-ester) (= SBFP/ Me)", "N,N-Bis-(2-(5-Carboxyoxazol-2-yl-)5-methoxybenzofuran-6-yl)-diaza[15]-Krone-5-(2OO-ethylester) (= SBFO/Et)" oder "N,N-Bis-(2-(2,4-dicarboxyphenyl-)5-methoxybenzofuran-6-yl-)diaza[15]-Krone-5-(2PP-methylester) (= SBFI/Me)" beschrieben wurde, dem gegebenenfalls einer oder mehrere der Verfahrensschritte vorausgeht, der in Folge letztendlich zur Verbindung 2 MM führt, wie vorstehend im Kapitel "Synthese der Verbindungen" beschrieben wurde; oder
(b) ein zu (a) analoges Verfahren durchführt, jedoch als Kronenether 1,7-Diam-4,10-dioxacyclododecan oder 1,10-Diaza-4,7,13,16-tetraoxacyclooctadecan verwendet.

22. Verwendung einer Verbindung, wie sie in einem der Ansprüche 1 bis 19 definiert ist, als Fluoreszenz-Indikatorfarbstoff für Alkalimetall-Kationen.

## Revendications (Revendications pour l'(les) Etat(s) contractant(s) suivant(s): AT, BE, CH, DE, FR, GB, GR, IT, LI, LU, NL, SE)

1. Ether aza-couronne de formule : 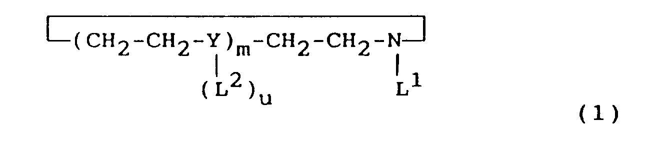 dans laquelle :
m est un entier compris entre 3 et 5 ;
chaque Y est indépendamment N ou O ;
u est 1 si Y est N et O si Y est O ;
L¹ est un ligand de formule (i) ou (ii) ci-dessous : 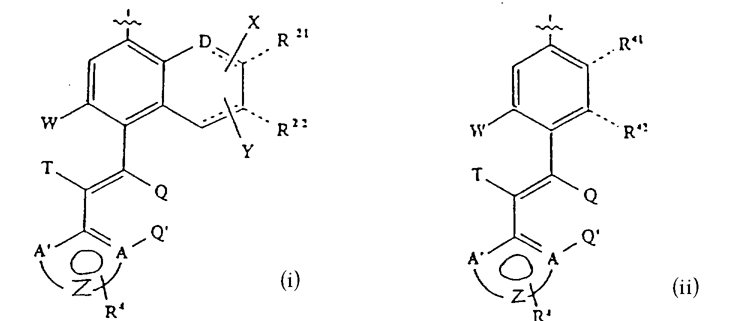 dans laquelle :
A et A¹ sont indépendamment C, N, O ou S ;
D est N ou O ;
Q est H ou NR¹R où R¹ et R sont indépendamment -H, un alkyle en C₁-C_{4,} -CH₂COOH, -CH₂CH₂OH ou un phényle, ou bien R¹ et R ensemble sont -(CH₂)₄-, -(CH₂)₅- ou -CH₂CH₂OCH₂CH₂- ;
Q¹ est H sauf si A est NR³, O ou S ; ou bien
Q et Q¹ ensemble sont NR³, et A est C ;
R³ est H, -CH₃, -C₂H₅ ou CH₂COOH ;
R⁴ est (E)ₙ où n = 0-3, et E est une fonction polaire preneuse d'électron choisie parmi -CO₂H, -CO₂R¹, -CONR¹R, -SO₃H, -SO₂NR¹R, -SO₂CF₃, -COCH₃ et -CN, où R¹ et R sont indépendamment -H, un alkyle en C₁-C₄, -CH₂COOH, -CH₂CH₂OH ou un phényle, ou bien R¹ et R ensemble sont -(CH₂)₄-, -(CH₂)₅-, ou -CH₂CH₂OCH₂CH₂- ;
R¹ est -H, -CH₃, -C₂H₅, un autre alkyle de degré faible jusqu'à C₄, -COOH, un alcoxy en C₁-C₄ ou -OC(O)CH₃ ; ou bien
R¹ et R ensemble sont -CH=CH-CH=CH-, X et Y sont tous deux des liaisons doubles et D est N de telle sorte que D, R¹ et R forment un cycle acridine ;
R⁴¹ est -OH, -OCH₂COOH, -OCH₂CH₂OH, un méthoxy ou un alcoxy en C₂-C₄, -NR¹⁴R¹⁵, -COOH, -C(O)NR¹⁴R¹⁵ ou -OC(O)CH₃, où R¹⁴ et R¹⁵ sont indépendamment -H, un alkyle en C₁-C₄, -CH₂COOH, -CH₂CH₂OH ou un phényle ;
R⁴ est -H, -CH₃, ou -COOH ;
W et T sont chacun H, ou bien sont ensemble O ou NR³ ;
X est une liaison double lorsque D est N, et une liaison simple lorsque D est O ;
Y peut être soit une liaison double, soit une liaison simple ;
et
Z est une partie formant un cycle (hétéro)aromatique ;
et L est H ;
un ligand de formule (i) ou de formule (ii) ;
-CH₂C(O)NR¹R où R¹ et R sont indépendamment -H, un alkyle en C₁-C₄, -CH₂COOH, -CH₂CH₂OH ou un phényle, ou bien R¹ et R ensemble sont -(CH₂)₄-, -(CH₂)₅- ou -CH₂CH₂OCH₂CH₂- (pour former un cycle respectivement pyrrolidine, piperidine, ou morpholine) ;
-CH₂COOH ;
un -2-pyridylméthyle ;
un -2-tétrahydrofurannylméthyle ;
-CH₂CH₂OR^{a}, où R^{a} est -H, un alkyle en C₁-C₄, -CH₂COOH, -CH₂CH₂OH, ou un phényle ; ou
un -2,5-dialcoxyphényle où l'alcoxy substituant est un alcoxy en C₁-C₄.

2. Composé selon la revendication 1, caractérisé en ce que : 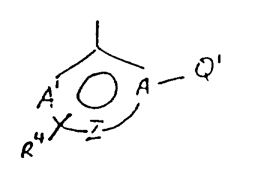 est un 2,4-dicarboxyphényle, un 3,4-dicarboxyphényle ou un 5-carboxyoxazole-2-yle.

3. Ether aza-couronne de formule : 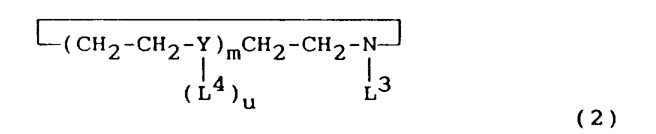 dans laquelle :
m est un entier compris entre 3 et 5 ;
chaque Y est indépendamment N ou O ;
u est 1 si Y est N et O si Y est O ;
L³ est une quinone ou un ligand de formule (iii) ou (iv) ci-dessous : 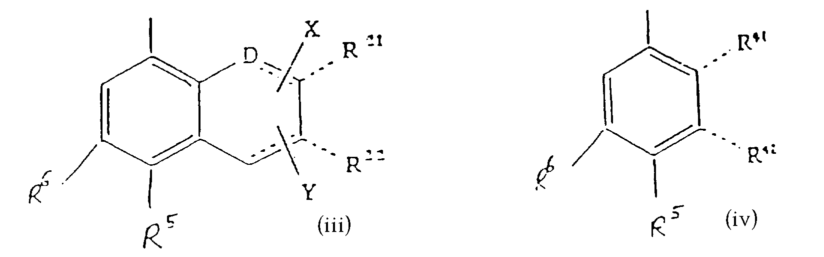 dans laquelle :
D, R¹, R, R⁴¹, R⁴, X et Y sont tels qu'ils sont définis dans la revendication 1 ;
R⁵ est H, -NH₂, -CHO ou -NHCOCH₃, ou -NHCOCH₂CH₂COO⁻ ou un autre groupe amino acylé ; et
R⁶ est H, -OH, ou -OCH₃ ou un autre alcoxy, avec comme condition que l'un de R⁵ et de R⁶
ne soit pas H ;
et L⁴ est H
une quinone ;
un ligand de formule (iii) ou (iv) ;
-CH₂C(O)NR¹R où R¹ et R sont indépendamment -H, un alkyle en C₁-C₄, -CH₂COOH, -CH₂CH₂OH ou un phényle, ou bien R¹ et R ensemble sont -(CH₂)₄-, -(CH₂)₅- ou -CH₂CH₂OCH₂CH₂- (pour former un cycle respectivement pyrrolidine, piperidine, ou morpholine) ;
-CH₂COOH ;
un -2-pyridylméthyle ;
un -2-tétrahydrofurannylméthyle;
-CH₂CH₂OR^{a}, ,où R^{a} est -H, un alkyle en C₁-C₄, -CH₂COOH, -CH₂CH₂OH ou un phényle ; ou
un -2,5-dialcoxyphényle où l'alcoxy substituant est un alcoxy en C₁-C₄.

4. Composé selon la revendication 3, caractérisé en ce que R⁵ est H, -NH₂, -CHO, -NHCOCH₃, -NHCOCH₂CH₂COO⁻ ou un autre groupe amido.

5. Composé selon la revendication 4, caractérisé en ce que :
R⁵ est H, -NH₂, -CH₃, -NHCOCH₃ ou -NHCOCH₂CH₂COO⁻ ; et
R⁶ est H, -OH ou -OCH₃.

6. Composé selon l'une quelconque des revendications 3 à 5, caractérisé en ce que, dans l'un quelconque des groupes L³ ou L⁴ de formule (iii), R⁵ n'est pas -NH₂.

7. Composé selon l'une quelconque des revendications 1 à 6, caractérisé en ce que l'un au moins des hydrogènes sur les carbones du noyau de l'éther aza-couronne est substitué par un substituant choisi parmi : -COOH, CH₂OH, -C(O)N(CH₃)₂, ou un alkyle en C₁-C₄ ; ou bien des hydrogènes sur des carbones de noyau adjacents de l'éther aza-couronne sont substitués par R³¹ et R³, où R³¹ et R³ ensemble sont -(CH₂)₃- ou -(CH₂)₄- (où R³¹ et R³ font partie d'un système aliphatique) ou bien R³¹ et R³ ensemble sont -(CH)₃-(où R³¹ et R³ font partie d'un système aromatique) ; ou bien des hydrogènes sur des carbones de noyau non adjacents de l'éther aza-couronne sont substitués par R³¹ et R³, où R³¹ et R³ ensemble sont -(CH₂)₂- ou -(CH₂)₃- (où R³¹ et R³ font partie d'un système aliphatique), ou bien où R³¹ et R³ ensemble sont -(CH)₃- (où R³¹ et R³ font partie d'un système aromatique), et en ce que, de manière optionnelle, chaque groupe L¹ et L est de formule G, N, O ou P de la figure 1, ou bien, selon le cas, chaque groupe L³ et L⁴ est de formule C, D, E, F, H, I, J, K, L ou M de la figure 1.

8. Composé selon la revendication 7, caractérisé en ce que le noyau de l'éther aza-couronne est de formule (B) ou (D) ci-dessous : 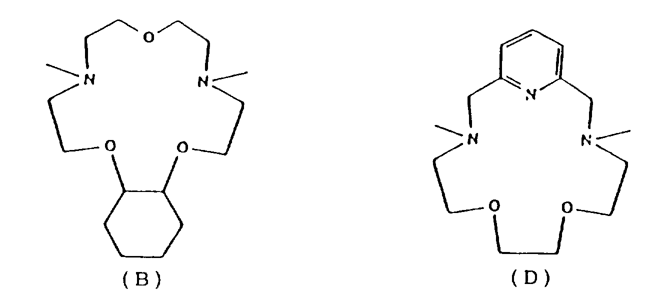 l'atome N hybridé en sp³ étant substitué comme défini dans l'une quelconque des revendications 1 à 6.

9. Composé selon l'une quelconque des revendications 1 à 7, caractérisé en ce que l'éther aza-couronne est un éther couronne monoaza, diaza, triaza, tétraaza, pentaaza ou hexaaza.

10. Composé selon la revendication 9, caractérisé en ce que l'éther aza-couronne est un éther monoaza-12-couronne-4, diaza-12-couronne-4, monoaza-15-couronne-5, diaza-15-couronne-5, monoaza-18-couronne-6 ou diaza-18-couronne-6.

11. Composé selon la revendication 10, caractérisé en ce que l'éther aza-couronne est un 1,7-diaza-4, 10, 13-trioxacyclopentadécane ou un l,10-diaza-4,7,13,16-tétraoxacyclooctadécane.

12. Ether aza-couronne selon la revendication 1 ou la revendication 2, ou l'une quelconque des revendications 7 à 11 lorsqu'elles dépendent de la revendication 1 ou de la revendication 2, caractérisé en ce que l'un ou plus des groupes L est remplacé par un groupe L⁴ de l'une quelconque des revendications 3 à 6.

13. Composé formé de la combinaison de (1) un éther aza-couronne et (2) au moins un ligand, caractérisé en ce que le(s) ligand()s est/sont attaché(s) à l'éther aza-couronne par l'intermédiaire du ou des azote(s) du noyau hybridé(s) en sp³, et en ce que (1) l'éther couronne est choisi dans le groupe formé du monoaza-12-couronne-4, du diaza-12-couronne-4, du monoaza-15-couronne-5, du diaza-15-couronne-5, du monoaza-18-couronne-6 et du diaza-18-couronne-6, et (2) le(s) ligand(s) est/sont choisi(s) dans les groupes dont les formules sont représentées sur la figure 1 comme les groupes B, C, D, E, F, G, H, I, J, K, L, M, N, O et P.

14. Composé selon l'une quelconque des revendications 1 à 9, 11 ou 12, caractérisé en ce que l'éther aza-couronne est un éther diaza-couronne symétrique ou un éther diaza-couronne asymétrique, de la façon définie précédemment.

15. Composé selon l'une quelconque des revendications 1 à 14, caractérisé en ce que :
. soit (a) l'éther aza-couronne possède au moins deux azotes du noyau ayant des ligands attachés, ces ligands étant identiques ;
. soit (b) l'éther aza-couronne possède au moins deux azotes du noyau ayant des ligands attachés, ces ligands n'étant pas identiques.

16. Composé choisi parmi :
(a) le composé SBFP, dont la structure est représentée sur la figure 7(a) comme 2NN ;
(b) le composé SBFO, dont la structure est représentée sur la figure 7(a) comme 2OO ;
(c) le composé SBFI, dont la structure est représentée sur la figure 7(a) comme 2PP ;
(d) le composé PBFP, dont la structure est représentée sur la figure 7(a) comme 3NN ;
(e) le composé PBFO, dont la structure est représentée sur la figure 7(b) comme 3OO ; ou
(f) le composé PBFI, dont la structure est représentée sur la figure 7(b) comme 3PP.

17. Composé selon l'une quelconque des revendications 1 à 16, caractérisé en ce que le composé est sous forme d'un ester dans lequel tous les carboxylates sont estérifiés pour former des esters hydrolysables physiologiquement, de préférence des esters d'acétoxyméthyle, ou bien le composé est sous forme d'un sel acceptable par la pharmacie humaine ou animale, ou un ester de celui-ci.

18. Ether aza-couronne de formule : 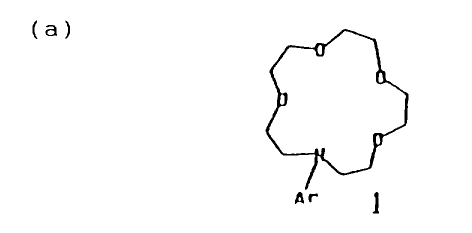 où Ar est un groupe dont la formule est représentée sur la figure 1 comme le groupe C, D, E, F, G, H, I, J, K, L, M, N, O ou P ; 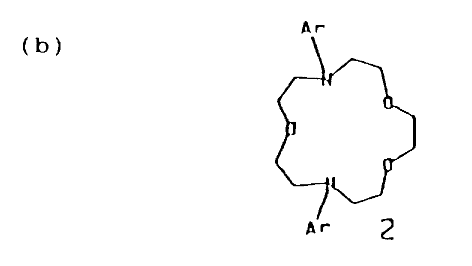 où les groupe Ar sont
(i) identiques, ou
(ii) non identiques,
et dans l'un (i) ou l'autre (ii) cas, sont chacun un groupe dont la formule est représentée sur la figure 1 comme le groupe C, D, E, F, G, H, I, J, K, L, M, N, O ou P ; ou 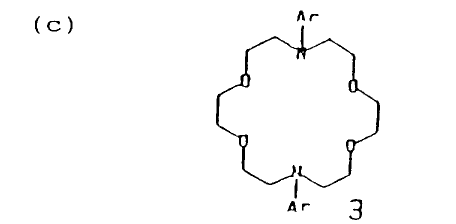 où les groupes Ar sont
(i) identiques, ou
(ii) non identiques,
et dans l'un (i) ou l'autre (ii) cas, sont chacun un groupe dont la formule est représentée sur la figure 1 comme le groupe C, D, E, F, G, H, I, J, K, L, M, N, O ou P.

19. Composé selon la revendication 18, caractérisé en ce que le noyau de l'éther aza-couronne est substitué comme cela est défini dans la revendication 7 ou la revendication 8, et/ou le composé est sous forme d'un ester dans lequel tous les carboxylates sont estérifiés pour former un ester hydrolysable physiologiquement, ou bien est sous forme d'un sel acceptable par la pharmacie humaine ou animale, ou d'un ester de celui-ci.

20. Procédé de préparation d'un agent chélatant de métal alcalin fluorescent macrocyclique, comprenant la formation d'une combinaison d'un éther aza-couronne et d'un ou plusieurs ligand(s) attaché(s) à l'éther couronne par l'intermédaiire d'un ou plusieurs azote(s) du noyau hybridé(s) en sp³, dans lequel l'éther couronne est de formule : 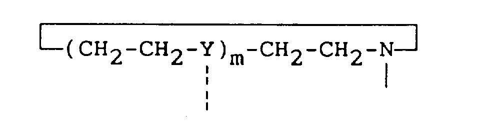 où m est un entier compris entre 3 et 5, chaque Y est indépendamment O ou N, et au moins un du ou des ligand(s) est de formule (i) ou (ii) de la revendication 1, ou bien de formule (iii) ou (iv) de la revendication 3, la combinaison étant définie en outre de manière optionnelle par la ou les caractéristique(s) spécifique(s) de l'une quelconque ou plus des revendications 2 ou 3 à 11, ou étant telle qu'elle est définie dans l'une quelconque des revendications 12 à 18.

21. Procédé de préparation d'un composé tel que défini dans la revendication 1, la revendication 3 ou la revendication 12, comprenant :
. soit (a) la mise en oeuvre d'une méthode chimiquement équivalente à une méthode comprenant celle décrite précédemment sous l'un quelconque des intitulés "N,N-bis-(2-(3,4-dicarboxyphényl)-5-méthoxybenzofurann-6-yl)-diaza[15]-couronne-5 (ester de Me de 2NN) (=SBFP/Me)", "N,N-bis-(2-(5-carboxyoxazol-2-yl)-5-méthoxybenzofurann-6-yl)-diaza[15]-couronne-5 (ester d'éthyle de 2OO) (=SBFO/Et)" ou "N,N-bis-(2-(2,4-dicarboxyphényl)-5-méthoxybenzofurann-6-yl)-diaza[15] -couronne-5 (ester de méthyle de 2PP) (=SBFI/Me)", précédé de manière optionnelle par une ou plusieurs des étapes successives des méthodes conduisant à un composé 2MM tel que décrit précédemment sous l'intitulé "SYNTHESE DU COMPOSE" ;
. soit (b) la mise en oeuvre d'un procédé analogue à celui du (a), mais en utilisant comme éther couronne du 1,7-diaza-4, 10-dioxacyclododécane ou du 1, 10-diaza-4, 7,13, 16-tétraoxacyclooctadécane.

22. Utilisation d'un composé tel que défini dans l'une quelconque des revendications 1 à 18 pour la préparation d'une formulation chélatant les métaux alcalins pour l'observation non destructive d'un concentré d'ion de métal alcalin dans les tissus.

23. Méthode d'observation non destructive d'un ion de métal alcalin se concentrant dans un tissu, comprenant l'utilisation dans ce tissu, comme agent chélatant du métal alcalin, d'un composé tel que défini dans l'une quelconque des revendications 1 à 18, et l'observation ou la mesure de la fluorescence.

## Revendications (Revendications pour l'(les) Etat(s) contractant(s) suivant(s): ES)

1. Méthode d'observation non destructive d'une concentration d'un ion de métal alcalin dans un tissu, comprenant l'utilisation dans ce tissu d'un agent chélatant les métaux alcalins et l'observation ou la mesure de la fluorescence, dans laquelle l'agent chélatant est un éther aza-couronne de formule : 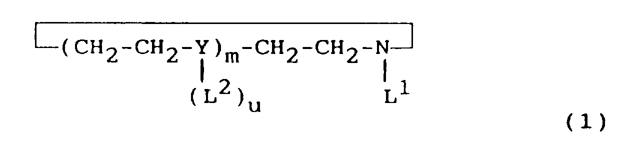 dans laquelle :
m est un entier compris entre 3 et 5 ;
chaque Y est indépendamment N ou O ;
u est 1 si Y est N et O si Y est O ;
L¹ est un ligand de formule (i) ou (ii) ci-dessous : 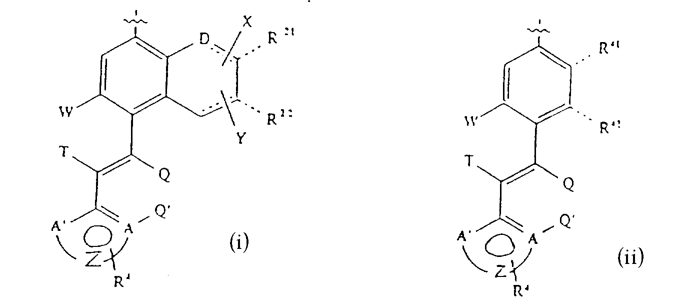 dans laquelle :
A et A¹ sont indépendamment C, N, O ou S ;
D est N ou O ;
Q est H ou NR¹R où R¹ et R sont indépendamment -H, un alkyle en C₁-C₄, -CH₂COOH, -CH₂CH₂OH ou un phényle, ou bien R¹ et R ensemble sont -(CH₂)₄-, -(CH₂)₅- ou -CH₂CH₂OCH₂CH₂- ;
Q¹ est H sauf si A est NR³, O ou S ; ou bien
Q et Q¹ ensemble sont NR³, et A est C ;
R³ est H, -CH₃, C₂H₅ ou CH₂COOH ;
R⁴ est (E)ₙ où n = 0-3, et E est une fonction polaire preneuse d'électron choisie parmi -CO₂H, -CO₂R¹, -CONR¹R, -SO₃H, -SO₂NR¹R, -SO₂CF₃, -COCH₃ et -CN, où R¹ et R sont indépendamment -H, un alkyle en C₁-C₄, -CH₂COOH, -CH₂CH₂OH ou un phényle, ou bien R¹ et R ensemble sont -(CH₂)₄-, -(CH₂)₅- ou -CH₂CH₂OCH₂CH₂- ;
R¹ est -H, -CH₃, -C₂H₅, un autre alkyle de degré faible jusqu'à C₄, -COOH, un alcoxy en C₁-C₄ ou -OC(O)CH₃ ; ou bien
R¹ et R ensemble sont -CH=CH-CH=CH-, X et Y sont tous deux des liaisons doubles et D est N de telle sorte que D, R¹ et R forment un cycle acridine ;
R⁴¹ est -OH, -OCH₂COOH, -OCH₂CH₂OH, un méthoxy ou un alcoxy en C₂-C₄, -NR¹⁴R¹⁵, -COOH, -C(O)NR¹⁴R¹⁵ ou -OC(O)CH₃, où R¹⁴ et R¹⁵ sont indépendamment -H, un alkyle en C₁-C₄, -CH₂COOH, -CH₂CH₂OH ou un phényle ;
R⁴ est -H, -CH₃, ou -COOH ;
W et T sont chacun H ou bien sont ensemble O ou NR³ ;
X est une liaison double lorsque D est N, et une liaison simple lorsque D est O ;
Y peut être soit une liaison double, soit une liaison simple ;
et
Z est une partie formant un cycle (hétéro)aromatique ;
et L est H ;
un ligand de formule (i) ou de formule (ii) ;
-CH₂C(O)NR¹R où R¹ et R sont indépendamment -H, un alkyle en C₁-C₄, -CH₂COOH, -CH₂CH₂OH ou un phényle, ou bien R¹ et R sont ensemble -(CH₂)₄-, -(CH₂)₅- ou -CH₂CH₂OCH₂CH₂- (pour former un cycle respectivement pyrrolidine, piperidine, ou morpholine) ;
-CH₂COOH ;
un -2-pyridylméthyle ;
un -2-tétrahydrofurannylméthyle ;
-CH2CH2OR^{a}, où R^{a} est -H, un alkyle en C₁-C₄, -CH₂COOH, -CH₂CH₂OH ou un phényle ; ou
un -2,5-dialcoxyphényle où l'alcoxy substituant est un alcoxy en C₁-C₄.

2. Méthode selon la revendication 1, caractérisée en ce que : 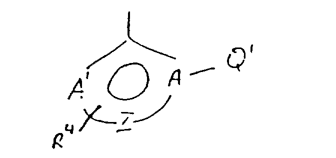 est un 2,4-dicarboxyphényle, un 3,4-dicarboxyphényle ou un 5-carboxyoxazol-2-yle.

3. Méthode d'observation non destructive d'une concentration d'un ion de métal alcalin dans un tissu, comprenant l'utilisation dans ce tissu d'un agent chélatant les métaux alcalins et l'observation ou la mesure de la fluorescence, dans laquelle l'agent chélatant est un éther aza-couronne de formule : 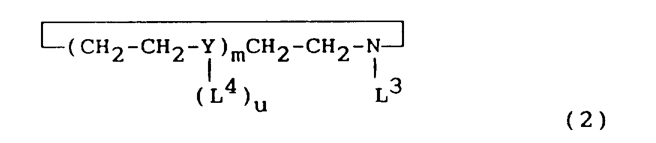 dans laquelle :
m est un entier compris entre 3 et 5 ;
chaque Y est indépendamment N ou O ;
u est 1 si Y est N et O si Y est O ;
L³ est une quinone ou un ligand de formule (iii) ou (iv) ci-dessous : 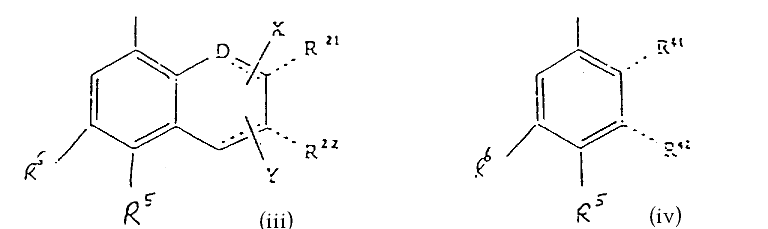 dans laquelle :
D, R¹, R, R⁴¹, R⁴, X et Y sont tels qu'ils sont définis dans la revendication 1 ;
R⁵ est H, -NH₂, -CHO ou -NHCOCH₃, ou -NHCOCH₂CH₂COO⁻ ou un autre groupe amino acyle ; et
R⁶ est H, -OH, ou -OCH₃ ou un autre alcoxy, à condition que l'un de R⁵ et de R⁶ ne soit pas H ;
et L⁴ est H
une quinone ;
un ligand de formule (iii) ou (iv) ;
-CH₂C(O)NR¹R où R¹ et R sont indépendamment -H, un alkyle en C₁-C₄, -CH₂COOH, -CH₂CH₂OH ou un phényle, ou bien R¹ et R ensemble sont -(CH₂)₄-, -(CH₂)₅- ou -CH₂CH₂OCH₂CH₂- (pour former un cycle respectivement pyrrolidine, piperidine, ou morpholine) ;
-CH₂COOH ;
un -2-pyridylméthyle ;
un -2-tétrahydrofurannylméthyle ;
-CH₂CH₂OR^{a}, où R^{a} est -H, un alkyle en C₁-C₄, -CH₂COOH, -CH₂CH₂OH ou un phényle ; ou
un -2,5-dialcoxyphényle où l'alcoxy substituant est un alcoxy en C₁-C₄.

4. Méthode selon la revendication 3, caractérisé en ce que R⁵ est H, -NH₂, -CHO, -NHCOCH₃, -NHCOCH₂CH₂COO⁻ ou un autre groupe amido.

5. Méthode selon la revendication 4, caractérisée en ce que :
R⁵ est H, -NH₂, -CH₃, -NHCOCH₃ ou -NHCOCH₂CH₂COO⁻ ; et
R⁶ est H, -OH ou -OCH₃.

6. Méthode selon l'une quelconque des revendications 3 à 5, caractérisée en ce que, dans l'un quelconque des groupes L³ ou L⁴ de formule (iii), R⁵ n'est pas -NH₂.

7. Méthode selon l'une quelconque des revendications 1 à 6, caractérisée en ce que l'un au moins des hydrogènes sur les carbones du noyau de l'éther aza-couronne est substitué par un substituant choisi parmi : -COOH, CH₂OH, -C(O)N(CH₃)₂ ou un alkyle en C₁-C₄ ; ou bien des hydrogènes sur des carbones du noyau de l'éther aza-couronne sont substitués par R³¹ et R³, où R³¹ et R³ ensemble sont -(CH₂)₃- ou - (CH₂)₄- ( où R³¹ et R³ font partie d'un système aliphatique) ou bien où R³¹ et R³ ensemble sont -(CH)₃- (où R³¹ et R³ font partie d'un système aromatique) ; ou bien des hydrogènes sur des carbones du noyau non adjacents de l'éther aza-couronne sont substitués par R³¹ et R³, où R³¹ et R³ ensemble sont -(CH₂)₂- ou -(CH₂)₃- (où R³¹ et R³ font partie d'un système aliphatique), ou bien où R³¹ et R³ ensemble sont -(CH)₃- (où R³¹ et R³ font partie d'un système aromatique), et en ce que, de manière optionnelle, chaque groupe L¹ et L est de formule G, N, O ou P de la figure 1, ou bien, selon le cas, chaque groupe L³ et L⁴ est de formule C, D, E, F, H, I, J, K, L ou M de la figure 1.

8. Méthode selon la revendication 7, caractérisée en ce que le noyau de l'éther aza-couronne est de formule (B) ou (D) ci-dessous : 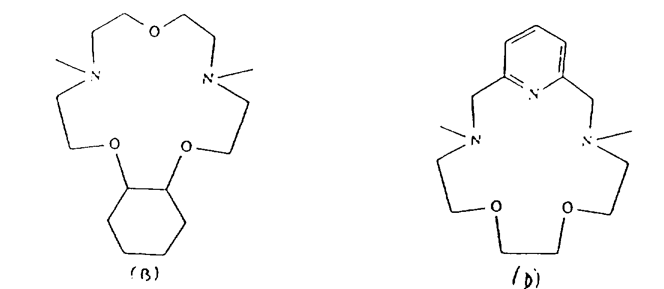 l'atome N hybridé en sp³ étant substitué comme défini dans l'une quelconque des revendications 1 à 6.

9. Méthode selon l'une quelconque des revendications 1 à 7, caractérisée en ce que l'éther aza-couronne est un éther couronne monoaza-, diaza, triaza, tétraaza, pentaaza ou hexaaza.

10. Méthode selon la revendication 9, caractérisée en ce que l'éther aza-couronne est un éther monoaza-12-couronne-4, diaza-12-couronne-4, monoaza-15-couronne-5, diaza-15-couronne-5, monoaza-18-couronne-6 ou diaza-18-couronne-6.

11. Méthode selon la revendication 10, caractérisé en ce que l'éther aza-couronne est un 1,7-diaza-4, 10,13-trioxacyclopentadécane ou un 1,10-diaza-4,7,13,16-tétraoxacyclooctadécane.

12. Méthode selon la revendication 1 ou la revendication 2, ou l'une quelconque des revendications 7 à 11 lorsqu'elles dépendent de la revendication 1 ou de la revendication 2, caractérisé en ce que l'un ou plus des groupes L est remplacé par un groupe L⁴ de l'une quelconque des revendications 3 à 6.

13. Méthode d'observation non destructive d'une concentration d'un ion de métal alcalin dans un tissu, comprenant l'utilisation dans ce tissu d'un agent chélatant les métaux alcalins et l'observation ou la mesure de la fluorescence, dans laquelle l'agent chélatant est un composé formé de la combinaison de (1) un éther aza-couronne et (2) au moins un ligand, caractérisée en ce que le ou les ligand(s) est/sont attaché(s) à l'éther aza-couronne par l'intermédiaire du ou des azote(s) du noyau hybridé(s) en sp³, et en ce que (1) l'éther couronne est choisi dans le groupe formé du monoaza-12-couronne-4, diaza-12-couronne-4, monoaza-15-couronne-5, diaza-15-couronne-5, monoaza-18-couronne-6 ou diaza-18-couronne-6, et (2) le ou les ligand(s) est/sont choisi(s) dans les groupes dont les formules sont représentées sur la figure 1 comme les groupes B, C, D, E, F, G, H, I, J, K, L, M, N, O et P.

14. Méthode selon l'une quelconque des revendications 1 à 9, 11 ou 12, caractérisée en ce que l'éther aza-couronne est un éther diaza-couronne symétrique ou un éther diaza-couronne assymétrique, de la façon définie précédemment.

15. Méthode selon l'une quelconque des revendications 1 à 14, caractérisée en ce que :
. soit (a) l'éther aza-couronne possède au moins deux azotes du noyau ayant des ligands attachés, ces ligands étant identiques ;
. soit (b) l'éther aza-couronne possède au moins deux azotes du noyau ayant des ligands attachés, ces ligands n'étant pas identiques.

16. Méthode d'observation non destructive d'une concentration d'un ion de métal alcalin dans un tissu, comprenant l'utilisation dans ce tissu d'un agent chélatant les métaux alcalins et l'observation ou la mesure de la fluorescence, dans laquelle l'agent chélatant est un composé choisi parmi :
(a) le composé SBFP, dont la structure est représentée sur la figure 7(a) comme 2NN ;
(b) le composé SBFO, dont la structure est représentée sur la figure 7(a) comme 2OO ;
(c) le composé SBFI, dont la structure est représentée sur la figure 7(a) comme 2PP ;
(d) le composé PBFP, dont la structure est représentée sur la figure 7(a) comme 3NN ;
(e) le composé PBFO, dont la structure est représentée sur la figure 7(b) comme 3OO ;
ou
(f) le composé PBFI, dont la structure est représentée sur la figure 7(b) comme 3PP ;

17. Méthode selon l'une quelconque des revendications 1 à 16, caractérisée en ce que le composé est sous la forme d'un ester dans lequel tous les carboxylates sont estérifiés pour former des esters hydrolysables physiologiquement, de préférence des esters d'acétoxyméthyle, ou bien le composé est sous forme d'un sel acceptable par la pharmacie humaine ou animale, ou un ester de celui-ci.

18. Méthode d'observation non destructive d'une concentration d'un ion de métal alcalin dans un tissu, comprenant l'utilisation dans ce tissu d'un agent chélatant les métaux alcalins et l'observation ou la mesure de la fluorescence, dans laquelle l'agent chélatant est un éther aza-couronne de formule : 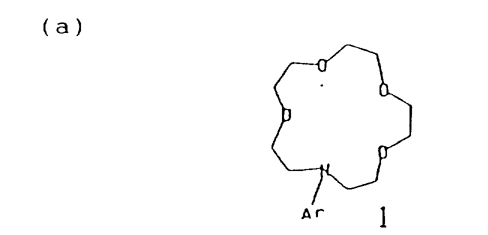 où Ar est un groupedont la formule est représentée sur la figure 1 comme le groupe C, D, E, F, G, H, I, J, K, L, M, N, O ou P ; 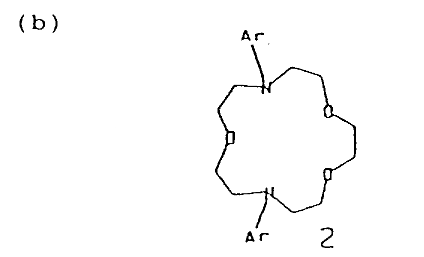 où les groupe Ar sont
(i) identiques, ou
(ii) non identiques,
et dans l'un (i) ou l'autre (ii) cas, sont chacun un groupe dont la formule est représentée sur la figure 1. comme le groupe C, D, E, F, G, H, I, J, K, L, M, N, O ou P ; ou 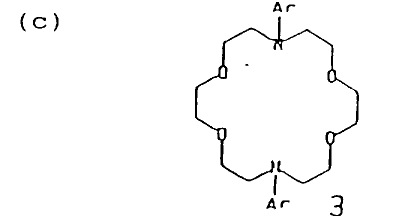 où les groupes Ar sont
(i) identiques, ou
(ii) non identiques,
et dans l'un(i) ou l'autre (ii) cas, sont chacun un groupe dont la formule est représentée sur la figure 1 comme le groupe C, D, E, F, G, H, I, J, K, L, M, N, O ou P.

19. Méthode selon la revendication 18, caractérisée en ce que le noyau de l'éther aza-couronne est substitué comme cela est défini dans la revendication 7 ou la revendication 8, et/ou le composé est sous forme d'un ester dans lequel tous les carboxylates sont estérifiés pour former un ester hydrolysable physiologiquement, ou bien est sous forme d'un sel acceptable par la pharmacie humaine ou animale, ou d'un ester de celui-ci.

20. Procédé de préparation d'un agent fluorescent macrocyclique chélatant les métaux alcalins, comprenant la formation d'une combinaison d'un éther aza-couronne et d'un ou plusieurs ligands attachés à l'éther couronne par l'intermédiaire d'un ou plusieurs azote(s) du noyau hybridé(s) en sp³, dans lequel l'éther couronne est de formule : 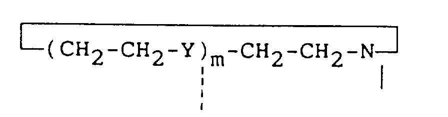 où m est un entier entre 3 et 5, chaque Y est indépendamment O ou N, et au moins un du ou des ligand(s) est de formule (i) ou (ii) de la revendication 1, ou bien de formule (iii) ou (iv) de la revendication 3, la combinaison étant définie en outre de manière optionnelle par la ou les caractéristique(s) spécifique(s) de l'une quelconque des revendications 2 ou 4 à 18.

21. Procédé de préparation d'un composé tel que défini dans l'une quelconque des revendications 1 à 19, comprenant :
. soit (a) la mise en oeuvre d'une méthode chimiquement équivalente à une méthode comprenant celle décrite précédemment sous l'un quelconque des intitulés "N,N-bis-(2-(3,4-dicarboxyphényl)-5-méthoxybenzofurann-6-yl)-diaza[15]-couronne-5 (ester de Me de 2NN) (=SBFP/Me)", "N,N-bis-(2-(5-carboxyoxazol-2-yl)-5-méthoxybenzofurann-6-yl)-diaza[15]-couronne-5 (ester d'éthyle de 2OO) (=SBFO/Et)" ou "N,N-bis-(2-(2,4-dicarboxyphényl)-5-méthoxybenzofurann-6-yl)-diaza[15] -couronne-5 (ester de méthyle de 2PP) (=SBFI/Me )", précédé de manière optionnelle par un ou plusieurs des étapes successives des méthodes conduisant à un composé 2MM tel que décrit précédemment sous l'intitulé "SYNTHESE DU COMPOSE" ;
. soit (b) la mise en oeuvre d'un procédé analogue à celui du (a), mais en utilisant comme éther couronne du 1,7-diaza-4,10-dioxacyclododécane ou du 1,10-diaza-4,7,13 16-tétraoxacyclooctadécane.

22. Utilisation d'un composé tel que défini dans l'une quelconque des revendications 1 à 19 comme colorant fluorescent d'indication pour des cations de métaux alcalins.
